# EUROPEAN PATENT APPLICATION

(11) **EP 2 256 120 A1**
(43) Date of publication of application: **01.12.2010**
(21) Application number: 10004213.4
(22) Date of filing: 07.08.2006
(51) Int. Cl.: C07D 498/14, C07F 7/00, C07D 401/12, C07D 401/04, A61K 31/4745, A61P 31/04

(54) **Oxazolidinone-quinolone hybrids as antibacterial compounds**

(30) Priority: 08.08.2005 WO PCT/EP2005/008573
(62) Divisional of application: 06795600.3
(71) Applicant: Actelion Pharmaceuticals Ltd., 4123 Allschwil (CH)
(72) Inventor: Hubschwerlen, Christian, 68480 Durmenach (FR); Panchaud, Philippe, 4123 Allschwil (CH); Sigwalt, Christine, 68320 Kunheim (FR); Specklin, Jean-Luc, 68680 Kembs (FR)
(74) Representative: Ruhlmann, Eric

(57) **Abstract**

The invention relates to novel chimeric antibiotics of formula I 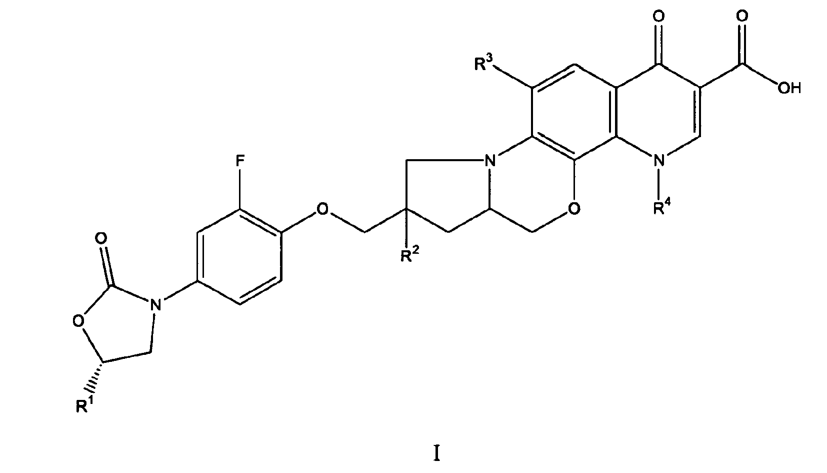 wherein
R¹ is heteroarylmethyl, heteroaryloxymethyl or heteroarylaminomethyl;
R² is H, OH, OSO₃H, OPO₃H₂, OCH₂OPO₃H₂, OCOCH₂CH₂COOH, OCOR⁶;
R³ is H or halogen;
R⁴ is (C₁-C₃)alkyl, (C₁-C₃)haloalkyl or cycloalkyl;
R⁶ is the residue of a naturally occurring amino acid or of dimethylaminoglycine.

These chimeric compounds are useful in the manufacture of medicaments for the treatment of infections (e.g. bacterial infections).

## Description

The present invention concerns novel chimeric antibiotics that are obtained from oxazolidinone derivatives linked to a quinolone via a spacer, pharmaceutical antibacterial composition containing them and use thereof in the manufacture of a medicament for the treatment of infections (e.g. bacterial infections). These chimeric compounds are useful antimicrobial agents effective against a variety of human and veterinary pathogens including among others Gram-positive aerobic bacteria, Gram-negative bacteria, anaerobic organisms and acid-fast organisms.

The intensive use of antibiotics has exerted a selective evolutionary pressure on microorganisms to produce genetically based resistance mechanisms. Modem medicine and socio-economic behaviour exacerbate the problem of resistance development by creating slow growth situations for pathogenic microbes, e.g. in artificial joints, and by supporting long-term host reservoirs, e.g. in immuno-compromised patients.

In hospital settings, an increasing number of strains of *Staphylococcus aureus, Streptococcus pneumonia, Enterococcus spp.,* and *Pseudomonas aeruginosa,* major sources of infections, are becoming multi-drug resistant and therefore difficult if not impossible to treat:
- *S. aureus* is β-lactam, quinolone and now even vancomycin resistant;
- *S. pneumoniae* is becoming resistant to penicillin, quinolone and even to new macrolides;
- *Enteroccocci* are quinolone and vancomycin resistant and β-lactams were never efficacious against these strains.

Further new emerging organisms like *Acinetobacter spp.* or *C. difficile,* which have been selected during therapy with the currently used antibiotics, are becoming a real problem in hospital settings.

In addition, microorganisms that are causing persistent infections are increasingly being recognized as causative agents or cofactors of severe chronic diseases like peptic ulcers or heart diseases.

In a chimeric molecule two or more molecules that exist separately in their native state are joined together to form a single entity (i.e. molecule) having the desired functionality of all of its constituent molecules.

Molecules wherein two antibiotics that have two different modes of action have been linked have been reported in the literature (*e.g.* Journal of Antimicrobial Chemotherapy (1994), 33, 197-200). Many of them are however such that the two antibiotic parts are released after biological activation (*e.g*. central ester cleavage, beta-lactam cleavage). Chemically and biochemically stable chimeric molecules that bind, as such, in two different targets have been more seldom reported. For example, oxazolidinone-quinolone hybrids have been reported as useful antimicrobial agents effective against a variety of multi-drug resistant pathogens (WO 03/032962, WO 03/031443 and WO 2004/096221). Further, synthesis and biological evaluation of these hybrids (Bioorg. & Med. Chem. (2003), 11, 2313-2319) and the influence of the central spacer on the antibacterial activity in the structure-activity relationship in the oxazolidinone-quinolone series have also been reported (Bioorg. Med. Chem. Lett. (2003), 13, 4229-4233). All these derivatives contain a 4-aminomethyl-oxazolidinone rest as part of the oxazolidinone pharmacophore.

It has now been surprisingly found that the chimeric derivatives of formula I as defined hereafter are useful antimicrobial agents that show effective against a variety of multi-drug resistant bacteria.

Thus, the present invention relates to compounds of the formula I 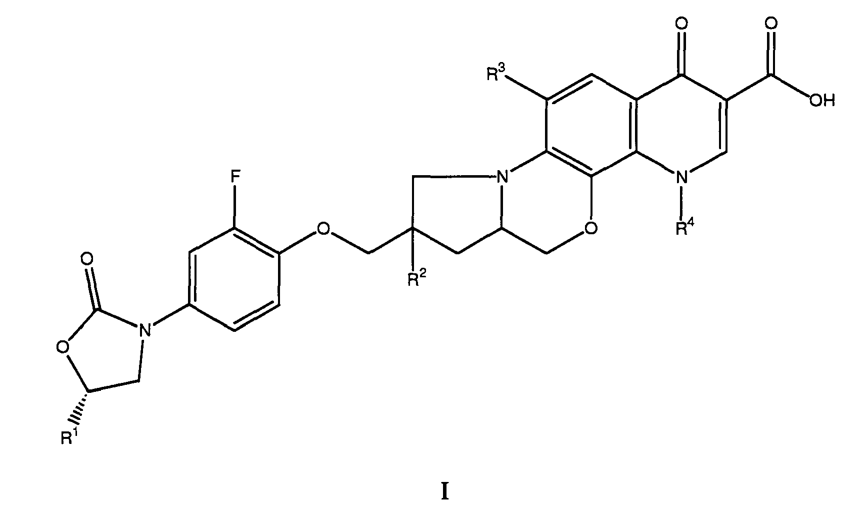 wherein
R¹ is CH₂NHCOR⁵, heteroarylmethyl, heteroaryloxymethyl or heteroarylaminomethyl;
R² is H, OH, OSO₃H, OPO₃H₂, OCH₂OPO₃H₂, OCOCH₂CH₂COOH or OCOR⁶;
R³ is H or halogen;
R⁴ is (C₁-C₃) alkyl, (C₁-C₃) haloalkyl or cycloalkyl;
R⁵ is alkyl or haloalkyl; and
R⁶ is the residue of a naturally occurring amino acid or of dimethylaminoglycine.

Any reference to a compound of general formula I is to be understood as referring also to configurational isomers, mixtures of enantiomers such as racemates, diastereomers, mixtures of diastereomers, diastereomeric racemates, and mixtures of diastereomeric racemates, as well as salts, solvent complexes, and morphological forms, as appropriate and expedient.

Salt-forming groups are groups or radicals having basic or acidic properties. Compounds having at least one basic group or at least one basic radical, for example amino, a secondary amino group not forming a peptide bond or a pyridyl radical, may form acid addition salts, for example with inorganic acids (e.g. HCl) or organic acids (e.g. tartaric acid). When several basic groups are present mono- or poly-acid addition salts may be formed.

Compounds having acidic groups, such as a carboxy group or a phenolic hydroxy group, may form metal or ammonium salts, such as alkali metal or alkaline earth metal salts, for example sodium, potassium, magnesium or calcium salts, or ammonium salts with ammonia or suitable organic amines, such as tertiary monoamines, for example triethylamine or tri-(2-hydroxyethyl)-amine, or heterocyclic bases, for example *N*-ethylpiperidine or *N*,*N'*-dimethylpiperazine, or also amino acids. Mixtures of salts are possible.

Compounds having both acidic and basic groups can form internal salts.

For the purposes of isolation or purification, as well as in the case of compounds that are used further as intermediates, it is also possible to use pharmaceutically unacceptable salts, e.g. the picrates. Only pharmaceutically acceptable, non-toxic salts may be used for therapeutic purposes, however, and those salts are therefore preferred.

A further embodiment of the compounds of the above formula I relates therefore to their prodrugs, their optically pure enantiomers, mixtures of enantiomers, racemates, optically pure diastereoisomers, mixtures of diastereoisomers, diastereoisomeric racemates, mixture of diastereoisomeric racemates, meso forms, pharmaceutically acceptable salts, solvent complexes and morphological forms thereof. Particularly preferred are the prodrugs, the optically pure enantiomers, optically pure diastereoisomers, meso forms, pharmaceutically acceptable salts, solvent complexes and morphological forms (and notably the pharmaceutically acceptable salts).

The following paragraphs provide definitions of the various chemical moieties for the compounds according to the invention and are intended to apply uniformly throughout the specification and claims unless an otherwise expressly set out definition provides a broader or narrower definition.

Unless specified otherwise, the term "alkyl" (whether used alone or in combination) refers to a saturated straight or branched chain alkyl group containing 1 to 6 carbon atoms, and preferably 1 to 3 carbon atoms. Representative examples of alkyl groups include, but are not limited to, methyl, ethyl, propyl, *iso*-propyl, butyl, *iso*-butyl, *sec*-butyl, *tert*-butyl, *n*-pentyl, neopentyl, *iso*-pentyl, *n*-hexyl and *iso*-hexyl. The term "(C₁-Cₓ)alkyl" (x being an integer) refers to a saturated straight or branched chain alkyl group containing 1 to x carbon atoms.

The term "alkenyl" refers to a straight or branched hydrocarbon chain containing 2 to 6 carbon atoms (and preferably 2 to 4 carbon atoms) with at least one carbon-carbon double bond. Representative examples of alkenyl include, but are not limited to, ethenyl, 2-propenyl, 3-butenyl, 4-pentenyl or 5-hexenyl.

The term "alkoxy" refers to a saturated straight or branched chain alkoxy group, containing 1 to 6 carbon atoms, and preferably 1 to 3 carbon atoms. Representative examples of alkoxy groups include, but are not limited to, methoxy, ethoxy, propoxy, *iso*-propoxy, *n*-butoxy, *iso*-butoxy, *sec*-butoxy, *tert*-butoxy or *n*-hexyloxy. The term "(C₁-Cₓ)akoxy" (x being an integer) refers to a straight or branched chain alkoxy group containing 1 to x carbon atoms.

The term "haloalkyl" refers to a saturated straight or branched chain alkyl group, containing from 1 to 6 and preferably 1 to 3 carbon atoms, in which at least one hydrogen atom (and possibly all) has been replaced by a halogen atom. The term "(C₁-Cₓ)haloalkyl" (x being an integer) refers to a straight of branched chain haloalkoxy group containing 1 to x carbon atoms. Representative examples of haloalkyl (and of (C₁-C₃)haloalkyl) groups include, but are not limited to, trifluoromethyl, chloromethyl, 2-chloroethyl and 3-fluoro-*n*-propyl (and notably trifluoromethyl, 2-chloroethyl and 3-fluoro-*n*-propyl).

The term "trialkylsilyl" refers to a trialkylsilyl group wherein each of the alkyl groups is independently a saturated straight or branched chain alkyl group containing 1 to 6 carbon atoms, and preferably 1 to 3 carbon atoms. Representative examples of trialkylsilyl groups include, but are not limited to, trimethylsilyl, triisopropylsilyl and *tert*-butyldimethylsilyl.

The term "halogen" refers to fluorine, chlorine, bromine or iodine, preferably to fluorine or chlorine.

The term "cycloalkyl", used alone or in combination, refers to a saturated cyclic hydrocarbon moiety containing 3 to 5 carbon atoms. Representative examples of cycloalkyl groups include, but are not limited to, cyclopropyl and cyclopentyl.

The term "heteroaryl", used alone or in combination, refers to an aromatic monocyclic 5-membered group containing one to four heteroatoms selected from the group consisting of oxygen, nitrogen and sulphur. Representative examples of heteroaryl groups include, but are not limited to, thiophenyl, furyl, imidazolyl, pyrazolyl, pyrrolyl, oxazolyl, isoxazolyl, thiazolyl, isothiazolyl, 1,2,3-triazolyl, 1,2,4-triazolyl, oxadiazolyl, thiadiazolyl and tetrazolyl groups. Any heteroaryl group as defined herein may be substituted with one or two (and preferably by one) substituents each independently selected from the group consisting of halogen, alkyl, alkoxy and hydroxymethyl. Specific examples of heteroaryl are 1,2,3-triazol-1-yl, 1,2,4-triazol-1-yl, thiophen-2-yl, thiazol-2-yl, 4-methyl-thiazol-2-yl, pyrazol-1-yl, imidazol-1-yl, 4-methyl-1,2,3-triazol-1-yl, tetrazol-1-yl and isoxazol-3-yl (and notably 1,2,3-triazolyl, thiophen-2-yl, thiazol-2-yl and 4-methyl-thiazol-2-yl).

The term "arylalkyl" refers to a saturated straight or branched chain alkyl group containing 1 to 6 carbon atoms, and preferably 1 to 3 carbon atoms, in which one hydrogen atom has been replaced by a phenyl group, which phenyl group may be substituted one to three times by substituents each independently selected from the group consisting of halogen, alkyl, alkoxy and nitro.

When it is written that R⁶ is the residue of an amino acid, it is meant thereby that R⁶-COOH is the corresponding amino acid.

The expression "pharmaceutically acceptable salts" encompasses either salts with inorganic acids or organic acids like hydrochloric acid, hydrobromic acid, hydroiodic acid, sulfuric acid, sulfamic acid, phosphoric acid, nitric acid, phosphorous acid, nitrous acid, citric acid, formic acid, acetic acid, oxalic acid, maleic acid, lactic acid, tartaric acid, fumaric acid, benzoic acid, mandelic acid, cinnamic acid, pamoic acid, stearic acid, glutamic acid, aspartic acid, methanesulfonic acid, ethanesulfonic acid, ethanedisulfonic acid, p-toluenesulfonic acid, salicylic acid, succinic acid, trifluoroacetic acid, and the like that are non toxic to living organisms or, in case the compound of formula I is acidic in nature, with an inorganic base like an alkali or earth alkali base, e.g. sodium hydroxide, potassium hydroxide, calcium hydroxide and the like. For other examples of pharmaceutically acceptable salts, reference can be made notably to "Salt selection for basic drugs", Int. J. Pharm. (1986), 33, 201-217.

Unless used regarding temperatures, the term "about" placed before a numerical value "X" refers in the current application to an interval extending from X minus 10% of X to X plus 10% of X, and preferably to an interval extending from X minus 5% of X to X plus 5% of X. In the particular case of temperatures, the term "about" placed before a temperature "Y" refers in the current application to an interval extending from the temperature Y minus 10°C to Y plus 10°C, and preferably to an interval extending from Y minus 5°C to Y plus 5°C; besides, room temperature shall mean in the current patent application 25°C.

Moreover, the sign "*" placed near an atom will be used to designate the point of attachment of a radical to the rest of a molecule. For example: 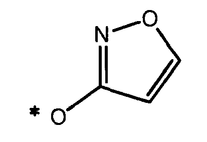 designates the isoxazol-3-yloxy radical.

In particular, the invention relates to compounds of formula I that are also compounds of formula I_{CE} 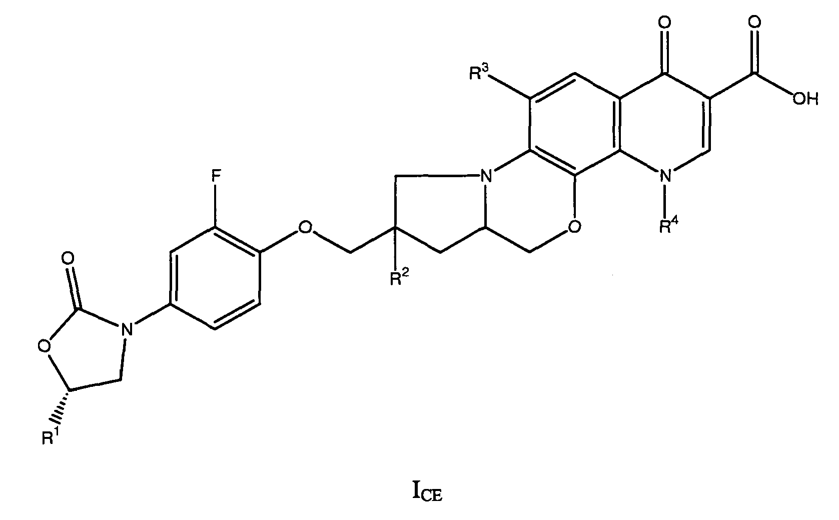 wherein
R¹ is CH₂NHCOR⁵, heteroarylmethyl or heteroaryloxymethyl;
R² is H, OH or OPO₃H₂;
R³ is H or halogen;
R⁴ is (C₁-C₃) alkyl or cycloalkyl; and
R⁵ is alkyl or haloalkyl;
and to salts of compounds of formula I_{CE}.

Preferably, the compounds of formula I_{CE} and their salts will be such that they have at least one of the following characteristics: 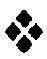
R¹ is CH₂NHCOR⁵, 1,2,3-triazol-1-ylmethyl, 1,2,4-triazol-1-ylmethyl, pyrazol-1-ylmethyl, imidazol-1-ylmethyl, tetrazol-1-ylmethyl or isoxazol-3-yloxymethyl; 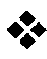
R³ is H or fluorine (and notably fluorine);
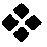 R⁴ is (C₁-C₃)alkyl or cyclopropyl (notably ethyl or cyclopropyl and especially cyclopropyl);
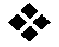 R⁵ is methyl or trifluoromethyl.

According to one variant of the invention, the compounds of formula I will be such that they have the following stereochemistry:

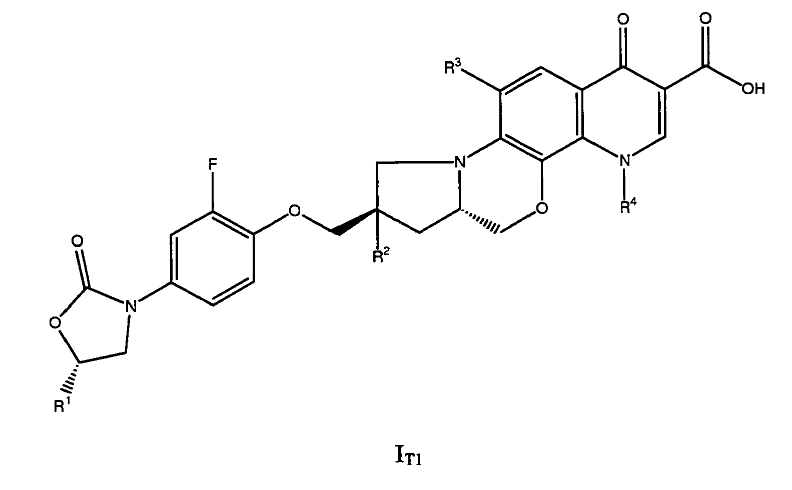

According to another variant of the invention, the compounds of formula I will be such that they have the following stereochemistry:

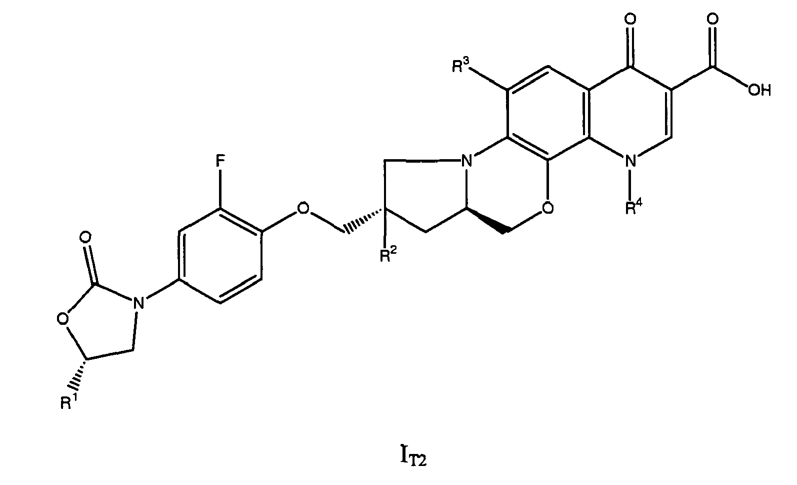

According to yet another variant of the invention, the compounds of formula I will be such that they have the following stereochemistry:

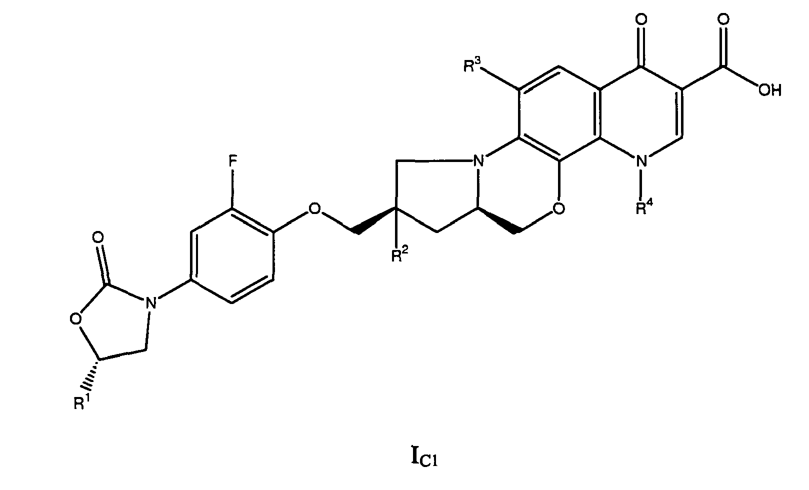

According to yet another variant of the invention, the compounds of formula I will be such that they have the following stereochemistry:

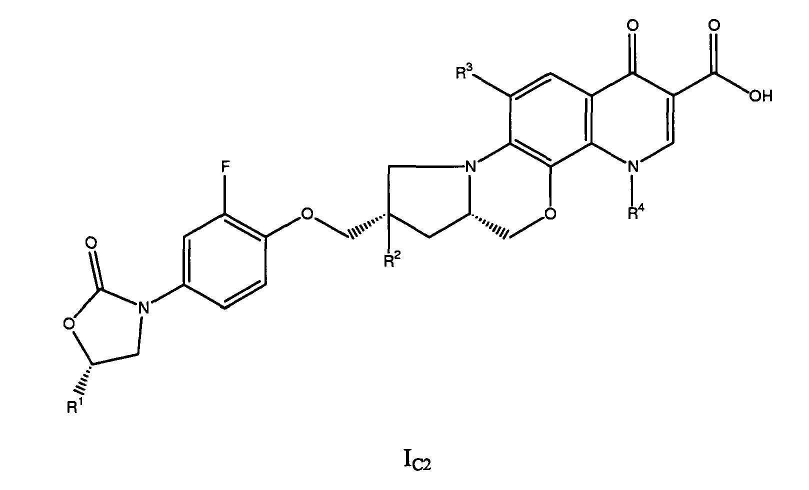

According to a particular embodiment of this invention, the compounds of formula I will be such that R¹ is CH₂NHCOR⁵. In this particular embodiment, R⁵ will preferably be methyl or trifluoromethyl.

According to another particular embodiment of this invention, the compounds of formula I will be such that R¹ is heteroarylmethyl (notably 1,2,3-triazol-1-ylmethyl, 1,2,4-triazol-1-ylmethyl, pyrazol-1-ylmethyl, imidazol-1-ylmethyl or tetrazol-1-ylmethyl and in particular 1,2,3-triazol-1-ylmethyl).

According to still a further particular embodiment of this invention, the compounds of formula I will be such that R¹ is heteroaryloxymethyl (notably isoxazol-3-yloxymethyl).

According to still a further particular embodiment of this invention, the compounds of formula I will be such that R¹ is heteroarylaminomethyl.

According to an important variant of this invention, the compounds of formula I will be such that R² is H.

According to another important variant of this invention, the compounds of formula I will be such that R² is OH.

According to yet another important variant of this invention, the compounds of formula I will be such that R² is OSO₃H, OPO₃H₂, OCH₂OPO₃H₂, OCOCH₂CH₂COOH or OCOR⁶ (and notably OPO₃H₂).

R¹ in its various embodiments can mean e.g.:
- CH₂NHCOR⁵, e.g. CH₂NHCOCH₃, CH₂NHCOCF₃ or CH₂NHCOCH₂Cl (and notably CH₂NHCOCH₃ or CH₂NHCOCF₃);
- heteroaryl-methyl, e.g. imidazol-1-ylmethyl, pyrazol-1-ylmethyl, thiazol-2-ylmethyl, thiazol-4-ylmethyl, thiazol-5-ylmethyl, isoxazol-3-ylmethyl, furan-2-ylmethyl, thiophen-2-ylmethyl, 1,2,3-triazol-1-ylmethyl, 4-methyl-1,2,3-triazol-1-yl, 1,2,4-triazol-1-ylmethyl, 1,2,3,4-tetrazol-2-ylmethyl, or 1,2,3,4-tetrazol-1-ylmethyl (and notably imidazol-1-ylmethyl, pyrazol-1-ylmethyl, thiazol-2-ylmethyl, thiazol-4-ylmethyl, thiazol-5-ylmethyl, isoxazol-3-ylmethyl, furan-2-ylmethyl, thiophen-2-ylmethyl, 1,2,3-triazol-1-ylmethyl, 1,2,3,4-tetrazol-2-ylmethyl or 1,2,3,4-tetrazol-1-ylmethyl);
- heteroaryloxy-methyl, e.g. imidazol-1-yloxymethyl, pyrazol-1-yloxymethyl, thiazol-2-yloxymethyl, thiazol-4-yloxymethyl, thiazol-5-yloxymethyl, isoxazol-3-yloxymethyl, furan-2-yloxymethyl, thiophen-2-yloxymethyl, 1,2,3-triazol-1-yloxymethyl, 1,2,3,4-tetrazol-2-yloxymethyl or 1,2,3,4-tetrazol-1-yloxymethyl;
- heteroarylamino-methyl, e.g. imidazol-1-ylaminomethyl, pyrazol-1-ylaminomethyl, thiazol-2-ylaminomethyl, thiazol-4-ylaminomethyl, thiazol-5-ylaminomethyl, isoxazol-3-ylaminomethyl, furan-2-ylaminomethyl, thiophen-2-ylaminomethyl, 1,2,3-triazol-1-ylaminomethyl, 1,2,3,4-tetrazol-2-ylaminomethyl or 1,2,3,4-tetrazol-1-ylaminomethyl.

According to the instant invention, R² is preferably hydrogen, OH, OPO₃H₂ or OCOR⁶.

Preferably, the amino acid residue R⁶ is such that R⁶-COOH represents Arg or Lys.

Preferred compounds of formula I are also those wherein at least one of the following characteristics is present:
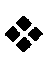 R¹ is CH₂NHCOR⁵ or heteroaryl-methyl, R⁵ being (C₁-C₃)alkyl (and especially methyl);
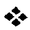 R² is OH, OPO₃H₂ or OCOR⁶;
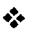 R³ is halogen;
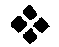 R⁴ is (C₁-C₃)alkyl or cycloalkyl.

Even more preferred compounds of formula I are also those wherein at least one of the following characteristics is present:
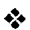 R¹ is CH₂NHCOR⁵ or heteroaryl-methyl, wherein R⁵ is C₁-C₃ alkyl (and especially methyl) and wherein the heteroaryl of the heteroarylmethyl group is a heteroaryl group containing at least one, and preferably two, nitrogen atom(s) (especially 1,2,3-triazol-1-yl);
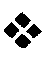 R² is OH;
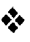 R³ is bromine, chlorine or fluorine (and in particular fluorine);
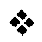 R⁴ is cycloalkyl (in particular cyclopropyl).

The following compounds of formula I are particularly preferred:
- (*16S,13S*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid;
- (*16S*,*13S*)-16-{4-[(*5S*)-(acetylamino-methyl)-2-oxo-oxazolidin-3-yl]-2-fluoro-phenoxymethyl}-1-cyclopropyl-7-fluoro-16-hydroxy-4-oxo-1,4,13,15,16,17-hexahydro-12*H*-11-oxa-1,14-diaza-cyclopenta[*a*]phenanthrene-3-carboxylic acid;
- (*16S*,*13R*)-16-{4-[(*5S*)-5-(acetylamino-methyl)-2-oxo-oxazolidin-3-yl]-2-fluoro-phenoxymethyl}-1-cyclopropyl-7-fluoro-16-hydroxy-4-oxo-1,4,13,15,16,17-hexahydro-12*H*-11-oxa-1,14-diaza-cyclopenta[a]phenanthrene-3-carboxylic acid;
- (*16S*,*13R*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid;
- (*13S*,*16R*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-11-oxa-1,14-diazacyclopenta[*a*]phenanthrene-3-carboxylic acid;
- (*13R*,*16R*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-11-oxa-1,14-diazacyclopenta[*a*]phenanthrene-3-carboxylic acid;
- (*13R*,*16R*)-1-cyclopropyl-7-fluoro-16-[2-fluoro-4-((*5S*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid;
- (*13S*,*16S*)-1-cyclopropyl-7-fluoro-16-[2-fluoro-4-((*5S*)-2-oxo-5-[1,2,3]triazol-1-yhnethyl-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid;
- (*13S*,*16R*)-1-cyclopropyl-7-fluoro-16-[2-fluoro-4-((*5S*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-11-oxa-1,14-diazacyclopenta[*a*]phenanthrene-3-carboxylic acid;
- (*13R*,*16S*)-1-cyclopropyl-7-fluoro-16-[2-fluoro-4-((*5S*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-11-oxa-1,14-diazacyclopenta[*a*]phenanthrene-3-carboxylic acid;
- (*13S*,*16S*)-1-cyclopropyl-7-fluoro-16-[2-fluoro-4-((*5R*)-2-oxo-5-pyrazol-1-ylmethyl-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid;
- (*13S*,*16S*)-1-cyclopropyl-7-fluoro-16-[2-fluoro-4-((*5S*)-5-imidazol-1-ylmethyl-2-oxo-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid;
- (*13S*,*16S*)-1-cyclopropyl-7-fluoro-16-{2-fluoro-4-[(*5R*)-5-(4-methyl-[1,2,3]triazol-1-ylmethyl)-2-oxo-oxazolidm-3-yl]-phenoxymethyl}-16-hydroxy-4 oxo-1,4,13,15,16,17-hexahydro-12*H*-11-oxa-1,14-diaza-cyclopenta[*a*]phenanthrene-3-carboxylic acid;
- (*13S*,*16S*)-1-cyclopropyl-7-fluoro-16-[2-fluoro-4-((*5R*)-2-oxo-5-tetrazol-1-ylmethyl-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid;
- (*13S*,*16S*)-1-cyclopropyl-7-fluoro-16-[2-fluoro-4-((*5R*)-2-oxo-5-[1,2,4]triazol-1-ylmethyl-oxazolidin-3-yl)-phenoxymethyl]-16-hydroxy-4-oxo-1,4,13,15,16,17-hexahydro-12*H*-11-oxa-1,14-diazacyclopenta[*a*]phenanthrene-3-carboxylic acid;
- (*13S*,*16S*)-1-cyclopropyl-7-fluoro-16-{2-fluoro-4-[(*5R*)-5-(isoxazol-3 yloxymethyl)-2-oxo-oxazolidin-3-yl]-phenoxymethyl}-16-hydroxy-4-oxo-1,4,13,15,16,17-hexahydro-12*H*-11-oxa-1,14-diaza-cyclopenta[*a*]phenanthrene-3-carboxylic acid;
- (*13S*,*16S*)-1-cyclopropyl-7-fluoro-16-[2-fluoro-4-((*5S*)-2-oxo-5-[(2,2,2-trifluoro-acetylamino)methyl]-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid;
- (*13S*,*16R*)-1-cyclopropyl-7-fluoro-16-[2-fluoro-4-((*5S*)-2-oxo-5-[(2,2,2-trifluoro-acetylamino)methyl]-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid;
- (*13S*,*16S*)-1-ethyl-7-fluoro-16-[2-fluoro-4-((*5R*)-2-oxo-5[1,2,3]triazol-1-ylmethyl-oxazolidin-3-yl)-phenoxymethyl]-16-hydroxy-4-oxo-1,4,13,15,16,17 hexahydro-12*H*-11-oxa-1,14-diaza-cyclopenta[*a*]phenanthrene-3-carboxylic acid;
- (*13S*,*16S*)-1-cyclopropyl-16-[2-fluoro-4-((*5R*)-2-oxo-5-[1,2,3]triazol-1-ylmethyl-oxazolidin-3-yl)-phenoxymethyl]-16-hydroxy-4-oxo-1,4,13,15,16,17 hexahydro-12*H*-11-oxa-1,14-diazacyclopenta[*a*]phenanthrene-3-carboxylic acid;
- (*13S*,*16S*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-4-oxo-1,4,13,15,16,17-hexahydro-12*H*-11-oxa-1,14-diazacyclopenta[a]phenanthrene-3-carboxylic acid;
- (*13S*,*16S*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-4-oxo-16-phosphonooxy-1,4,13,15,16,17-hexahydro-12*H*-11-oxa-1,14-diaza-cyclopenta[*a*]phenanthrene-3-carboxylic acid;
- (*13R*,*16S*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-4-oxo-16-phosphonooxy-1,4,13,15,16,17-hexahydro-12*H*-11-oxa-1,14-diaza-cyclopenta[*a*]phenanthrene-3-carboxylic acid;
- (*13S*,*16R*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-4-oxo-16-phosphonooxy-1,4,13,15,16,17-hexahydro-12*H*-11-oxa-1,14-diaza-cyclopenta[*a*]phenanthrene-3-carboxylic acid;
- (*13R*,*16S*)-1-cyclopropyl-7-fluoro-16-[2-fluoro-4-((*5S*)-2-oxo-5-[1,2,3]triazol-1-ylmethyl-oxazolidin-3-yl)-phenoxymethyl]-4-oxo-16-phosphonooxy-1,4,13,15,16,17-hexahydro-12*H*-11-oxa-1,14-diaza-cyclopenta[*a*]phenanthrene-3-carboxylic acid;
- (*13S*,*16S*)-1-cyclopropyl-7-fluoro-16-[2-fluoro-4-((*5S*)-2-oxo-5-[1,2,3]triazol-1-ylmethyl-oxazolidin-3-yl)-phenoxymethyl]-4-oxo-16-phosphonooxy-1,4,13,15,16,17-hexahydro-12*H*-11-oxa-1,14-diaza-cyclopenta[*a*]phenanthrene-3-carboxylic acid;
- (*13S*,*16R*)-1-cyclopropyl-7-fluoro-16-[2-fluoro-4-((*5S*)-2-oxo-5-[1,2,3]triazol-1-ylmethyl-oxazolidin-3-yl)-phenoxymethyl]-4-oxo-16-phosphonooxy-1,4,13,15,16,17-hexahydro-12*H*-11-oxa-1,14-diaza-cyclopenta[*a*]phenanthrene-3-carboxylic acid;
as well as pharmaceutically acceptable salts thereof.

The following compounds of formula I are especially preferred:
- (*16S*,*13S*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid;
- (*16S*,*13S*)-16-{4-[(*5S*)-(acetylamino-methyl)-2-oxo-oxazolidin-3-yl]-2-fluoro-phenoxymethyl}-1-cyclopropyl-7-fluoro-16-hydroxy-4-oxo-1,4,13,15,16,17-hexahydro-12*H*-11-oxa-1,14-diaza-cyclopenta[*a*]phenanthrene-3-carboxylic acid;
- (*16S*,*13R*)-16-{4-[(*5S*)-5-(acetylamino-methyl)-2-oxo-oxazolidin-3-yl]-2-fluoro-phenoxymethyl}-1-cyclopropyl-7-fluoro-16-hydroxy-4-oxo-1,4,13,15,16,17-hexahydro-12*H*-11-oxa-1,14-diaza-cyclopenta[*a*]phenanthrene-3-carboxylic acid;
- (*16S*,*13R*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid;
as well as pharmaceutically acceptable salts thereof.

Chimeric derivatives of formula I are suitable for the use as medicaments, particularly as antimicrobial agents, in human medicine but also in veterinary medicine in the treatment of species like pigs, ruminants, horses, dogs, cats and poultry.

Chimeric derivatives of formula I according to the present invention are also useful for the manufacture of a medicament for the treatment of infections (notably bacterial infections or protozoal infections) and disorders related to infections (notably disorders related to bacterial infections or to protozoal infections).

The compounds according to this invention are particularly active against bacteria and bacteria-like organisms. They are therefore particularly suitable in human, as well as in animals, for the prophylaxis and chemotherapy of local and systemic infections caused by these pathogens as well as disorders related to bacterial infections comprising pneumonia, otitis media, sinusitis, bronchitis, tonsillitis, and mastoiditis related to infection by *Streptococcus pneumoniae*, *Haemophilus influenzae*, *Moraxella catarrhalis*, *Staphylococcus aureus, Enterococcus faecalis, E. faecium, E. casseliflavus*, *S. epidermidis*, *S. haemolyticus,* or *Peptostreptococcus spp*.; pharyngitis, rheumatic fever, and glomerulonephritis related to infection by *Streptococcus pyogenes*, Groups C and G streptococci, *Corynebacterium diphtheriae*, or *Actinobacillus haemolyticum*; respiratory tract infections related to infection by *Mycoplasma pneumoniae, Legionella pneumophila, Streptococcus pneumoniae, Haemophilus influenzae*, or *Chlaniydia pneumoniae*; blood and tissue infections, including endocarditis and osteomyelitis, caused by *S. aureus, S. haemolyticus, E. faecalis, E. faecium, E. durans*, including strains resistant to known antibacterials such as, but not limited to, beta-lactams, vancomycin, aminoglycosides, quinolones, chloramphenicol, tetracyclines and macrolides; uncomplicated skin and soft tissue infections and abscesses, and puerperal fever related to infection by *Staphylococcus aureus,* coagulase-negative staphylococci (*i.e., S. epidermidis, S. haemolyticus,* etc.), *Streptococcus pyogenes, Streptococcus agalactiae,* Streptococcal groups C-F (minute colony streptococci), viridans streptococci, *Corynebacterium minutissimum, Clostridium spp*., or *Bartonella henselae*; uncomplicated acute urinary tract infections related to infection by *Staphylococcus aureus,* coagulase-negative staphylococcal species, or *Enterococcus spp.*; urethritis and cervicitis; sexually transmitted diseases related to infection by *Chlamydia trachomatis, Haemophilus ducreyi, Treponema pallidum, Ureaplasma urealyticum,* or *Neiserria gonorrhoeae*; toxin diseases related to infection by *S. aureus* (food poisoning and toxic shock syndrome), or Groups A, B, and C streptococci; ulcers related to infection by *Helicobacter pylori*; systemic febrile syndromes related to infection by *Borrelia recurrentis*; Lyme disease related to infection by *Borrelia burgdorferi*; conjunctivitis, keratitis, and dacrocystitis related to infection by *Chlamydia trachomatis, Neisseria gonorrhoeae*, *S. aureus, S. pneumoniae, S. pyogenes*, *H. influenzae*, or *Listeria* spp.; disseminated *Mycobacterium avium* complex (MAC) disease related to infection by *Mycobacterium avium,* or *Mycobacterium intracellulare*; infections caused by *Mycobacterium tuberculosis, M. leprae, M. paratuberculosis*, *M. kansasii*, or *M. chelonei*; gastroenteritis related to infection by *Campylobacter jejuni*; intestinal protozoa related to infection by *Cryptosporidium* spp.; odontogenic infection related to infection by viridans streptococci; persistent cough related to infection by *Bordetella pertussis*; gas gangrene related to infection by *Clostridium perfringens* or *Bacteroides* spp.; and atherosclerosis or cardiovascular disease related to infection by *Helicobacter pylori* or *Chlamydia pneumoniae.*

Compounds of formula I according to the present invention are further useful for the preparation of a medicament for the treatment of infections that are mediated by bacteria such as *E. coli, Klebsiella pneumoniae* and other Enterobacteriaceae, *Acinetobacter spp., Stenothrophomonas maltophilia, Neisseria meningitidis*, *Bacillus cereus*, *Bacillus anthracis*, *Corynebacterium spp*., *Propionibacterium acnes* and bacteroide *spp.* In addition, compounds of formula I according to the present invention are further useful for the preparation of a medicament for the treatment of infections that are mediated by *C. difficile.*

Compounds of formula I according to the present invention are further useful to treat protozoal infections caused by *Plasmodium malaria, Plasmodium falciparum, Toxoplasma gondii*, *Pneumocystis carinii*, *Trypanosoma brucei* and *Leishmania spp*.

The preceding lists of pathogens are to be interpreted merely as examples and in no way as limiting.

As well as in humans, bacterial infections can also be treated in other species like pigs, ruminants, horses, dogs, cats and poultry.

As mentioned above, therapeutically useful agents that contain compounds of formula I, their solvates, salts or formulations are also comprised in the scope of the present invention. In general, compounds of formula I can be administered, for example, perorally, e.g. as tablets, coated tablets, dragees, soft and hard gelatine capsules, pills, aqueous or oily solutions, emulsions, suspensions or syrups, rectally, e.g. in the form of suppositories, parenterally e.g. in the form of injection or infusion solutions, or topically, e.g. in the form of ointments, creams or oils.

The production of the pharmaceutical compositions can be effected in a manner which will be familiar to any person skilled in the art (see for example Mark Gibson, Editor, Pharmaceutical Preformulation and Formulation, IHS Health Group, Englewood, CO, USA, 2001; Remington, The Science and Practice of Pharmacy, 20th Edition, Philadelphia College of Pharmacy and Science) by bringing the described compounds of formula I and their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

Another aspect of the invention concerns a method for the treatment of an infection comprising the administration to the patient of a pharmaceutically active amount of a compound according to formula I or of a pharmaceutically acceptable salt thereof.

Moreover, the compounds of formula I may also be used for cleaning purposes, e.g. to remove pathogenic microbes and bacteria from surgical instruments or to make a room or an area aseptic. For such purposes, the compounds of formula I could be contained in a solution or in a spray formulation.

The invention also relates to intermediates useful in the preparation of the compounds of formula I, namely the compounds of formula III_{G} 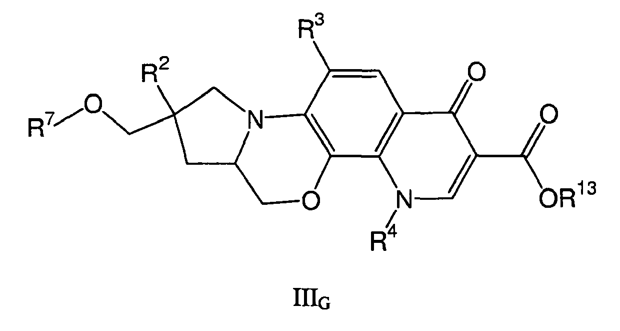 wherein
R² is H or OH,
R³ and R⁴ are as defined in formula I,
R⁷ is H, 2-tetrahydropyranyl, methoxymethyl, 2-methoxyethoxymethyl, allyl, alkylcarbonyl, trialkylsilyl or SO₂R¹⁵,
or also R² and R⁷ are such that R² and OR⁷ form, together with the carbon atoms that carry them, an acetonide ring,
or also R² and R⁷ are such that R² and OR⁷ form, together with the carbon atoms that carry them, an epoxide ring (in other words, R² and R⁷ taken together represent a bond),
R¹³ is H, alkyl or arylalkyl, and
R¹⁵ is alkyl, trifluoromethyl, phenyl or tolyl;
and to salts of compounds of formula III_{G}.

According to a first variant, the compounds of formula III_{G} will also correspond to the formula: 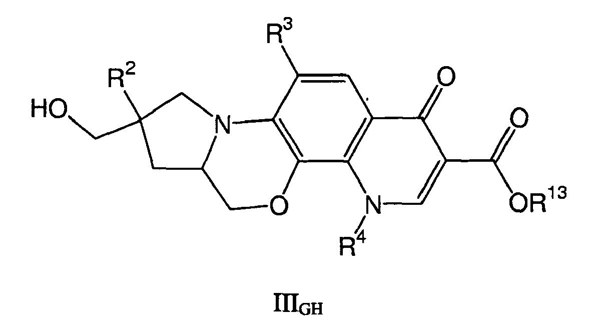 wherein R², R³, R⁴ and R¹³ have the same meaning as in formula III_{G}.

According to a second variant, the compounds of formula III_{G} will also correspond to the formula: 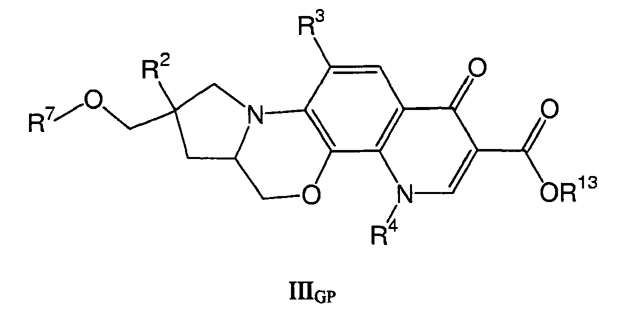 wherein
R⁷ is 2-tetrahydropyranyl, methoxymethyl, 2-methoxyethoxymethyl, allyl, alkylcarbonyl or trialkylsilyl, and
R², R³, R⁴ and R¹³ have the same meaning as in formula III_{G}.

According to a third variant, the compounds of formula III_{G} will also correspond to the formula: 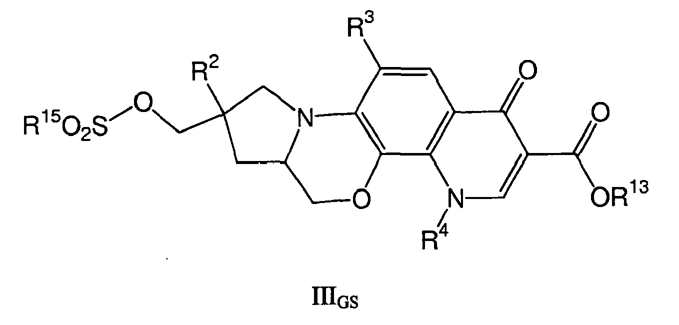 wherein R², R³, R⁴, R¹³ and R¹⁵ have the same meaning as in formula III_{G}.

According to a fourth variant, the compounds of formula III_{G} will also correspond to the formula: 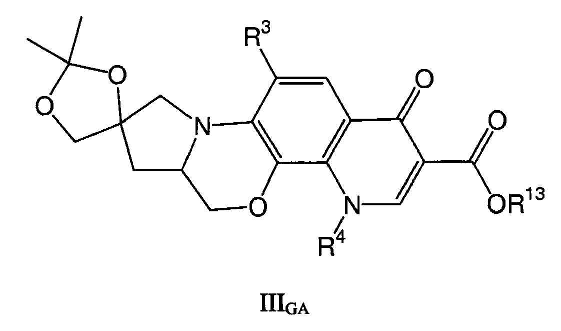 wherein R², R³, R⁴ and R¹³ have the same meaning as in formula III_{G}.

According to a fifth variant, the compounds of formula III_{G} will also correspond to the formula: 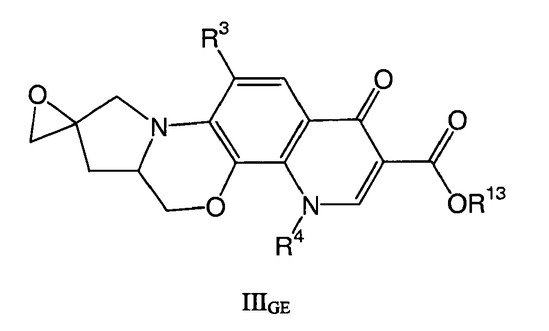 wherein R³, R⁴ and R¹³ have the same meaning as in formula III_{G} (and notably such a compound wherein R³ is fluorine, R⁴ is cyclopropyl and R¹³ is benzyl).

The following compounds of formula III_{G} will be preferred:
- (*13S*,*16S*)-1-cyclopropyl-7-fluoro-16-hydroxy-16-hydroxymethyl-4-oxo-1,4,13,15,16,17-hexahydro-12*H*-11-oxa-1,14-diaza-cyclopenta[*a*]phenanthrene-3-carboxylic acid ethyl ester;
- (*13S*,*16S*)-1-cyclopropyl-7-fluoro-16-hydroxy-16-methanesulfonyloxymethyl-4-oxo-1,4,13,15,16,17-hexahydro-12*H*-11-oxa-1,14-diaza-cyclopenta[*a*]phenanthrene-3-carboxylic acid ethyl ester;
- (*13S*,*16S*)-1-cyclopropyl-7-fluoro-16-hydroxy-16-hydroxymethyl-4-oxo-1,4,13,15,16,17-hexahydro-12*H*-11-oxa-1,14-diaza-cyclopenta[*a*]phenanthrene-3-carboxylic acid benzyl ester;
- (*13S*,*16S*)-1-cyclopropyl-7-fluoro-16-hydroxy-16-methanesulfonyloxymethyl-4-oxo-1,4,13,15,16,17-hexahydro-12*H*-11-oxa-1,14-diaza-cyclopenta[*a*]phenanthrene-3-carboxylic acid benzyl ester;
- (*13R*,*16S*)-1-cyclopropyl-7-fluoro-16-hydroxy-16-hydroxymethyl-4-oxo-1,4,13,15,16,17-hexahydro-12*H*-11-oxa-1,14-diaza-cyclopenta[*a*]phenanthrene-3-carboxylic acid ethyl ester;
- (*13R*,*16S*)-1-cyclopropyl-7-fluoro-16-hydroxy-16-methanesulfonyloxymethyl-4-oxo-1,4,13,15,16,17-hexahydro-12*H*-11-oxa-1,14-diaza-cyclopenta[*a*]phenanthrene-3-carboxylic acid ethyl ester;
- (*13S*,*16R*)-1-cyclopropyl-7-fluoro-16-hydroxy-16-hydroxymethyl-4-oxo-1,4,13,15,16,17-hexahydro-12*H*-11-oxa-1,14-diaza-cyclopenta[*a*]phenanthrene-3-carboxylic acid benzyl ester;
- (*13S*,*16R*)-1-cyclopropyl-7-fluoro-16-hydroxy-16-methanesulfonyloxymethyl-4-oxo-1,4,13,15,16,17-hexahydro-12*H*-11-oxa-1,14-diaza-cyclopenta[*a*]phenantbrene-3-carboxylic acid benzyl ester;
- (*13R*,*16R*)-1-cyclopropyl-7-fluoro-16-hydroxy-16-hydroxymethyl-4-oxo-1,4,13,15,16,17-hexahydro-12*H*-11-oxa-1,14-diaza-cyclopenta[*a*]phenanthrene-3-carboxylic acid benzyl ester;
- (*13R*,*16S*)-1-cyclopropyl-7-fluoro-16-hydroxy-16-hydroxymethyl-4-oxo-1,4,13,15,16,17-hexahydro-12*H*-11-oxa-1,14-diaza-cyclopenta[*a*]phenanthrene-3-carboxylic acid benzyl ester;
- (*13R*,*16R*)-1-cyclopropyl-7-fluoro-16-hydroxy-16-methanesulfonyloxymethyl-4-oxo-1,4,13,15,16,17-hexahydro-12*H*-11-oxa-1,14-diaza-cyclopenta[*a*]phenanthrene-3-carboxylic acid benzyl ester;
- (*13R*,*16S*)-1-cyclopropyl-7-fluoro-16-hydroxy-16-methanesulfonyloxymethyl-4-oxo-1,4,13,15,16,17-hexahydro-12*H*-11-oxa-1,14-diaza-cyclopenta[*a*]phenanthrene-3-carboxylic acid benzyl ester;
- 1-cyclopropyl-7-fluoro-4-oxo-1,4,13,15,16,17-hexahydro-12*H*-11-oxa-1,14-diaza-(1-oxaspiro[2.4]hepta)[*a*]phenanthrene-3-carboxylic acid benzyl ester;
- (*13S*,*16S*)-1-ethyl-7-fluoro-16-hydroxy-16-hydroxymethyl-4-oxo-1,4,13,15,16,17-hexahydro-12*H*-11-oxa-1,14-diaza-cyclopenta[*a*]phenanthrene-3-carboxylic acid benzyl ester;
- (*13S*,*16S*)-1-ethyl-7-fluoro-16-hydroxy-16-methanesulfonyloxymethyl-4-oxo-1,4,13,15,16,17-hexahydro-12*H*-11-oxa-1,14-diaza-cyclopenta[*a*]phenanthrene-3-carboxylic acid benzyl ester;
- (*13S*,*16S*)-1-cyclopropyl-16-hydroxy-16-hydroxymethyl-4-oxo-1,4,13,15,16,17-hexahydro-12*H*-11-oxa-1,14-diaza-cyclopenta[*a*]phenanthrene-3-carboxylic acid benzyl ester;
- (*13S*,*16S*)-1-cyclopropyl-16-hydroxy-16-methanesulfonyloxymethyl-4-oxo-1,4,13,15,16,17-hexahydro-12*H*-11-oxa-1,14-diaza-cyclopenta[*a*]phenanthrene-3-carboxylic acid benzyl ester;
- (*13S*,*16R*)-1-cyclopropyl-7-fluoro-16-hydroxymethyl-4-oxo-1,4,13,15,16,17-hexahydro-12*H*-11-oxa-1,14-diaza-cyclopenta[*a*]phenanthrene-3-carboxylic acid benzyl ester;
- (*13S*,*16S*)-1-cyclopropyl-7-fluoro-16-hydroxymethyl-4-oxo-1,4,13,15,16,17-hexahydro-12*H*-11-oxa-1,14-diaza-cyclopenta[*a*]phenanthrene-3-carboxylic acid benzyl ester;
as well as the salts of these compounds.

The invention also relates to intermediates useful in the preparation of the compounds of formula I, namely the compounds of formula IX*a* 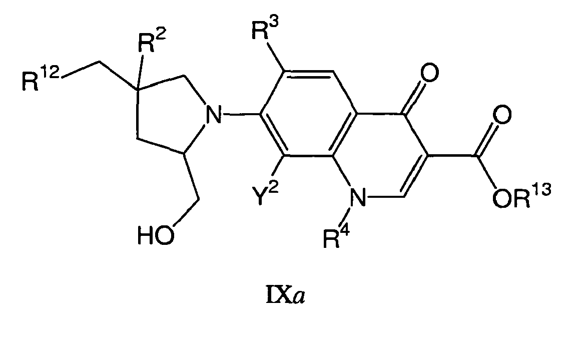 wherein
R² and R¹² are each OH or R² and R¹² form, together with the carbon atoms that they are attached to, an acetonide ring, or also R¹² and R² together are connected to form a double bond,
Y² is a halogen atom,
R³ and R⁴ are as defined in formula I, and
R¹³ is H, alkyl or arylalkyl;
and to salts of compounds of formula IX*a*.

According to a first variant, the compounds of formula IX*a* will also correspond to the formula: 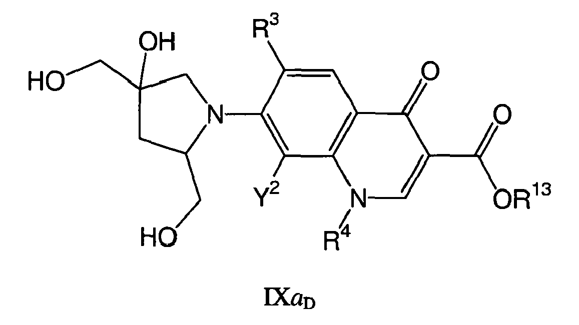 wherein R³, R⁴, Y² and R¹³ have the same meaning as in formula IX.

According to a second variant, the compounds of formula IX*a* will also correspond to the formula: 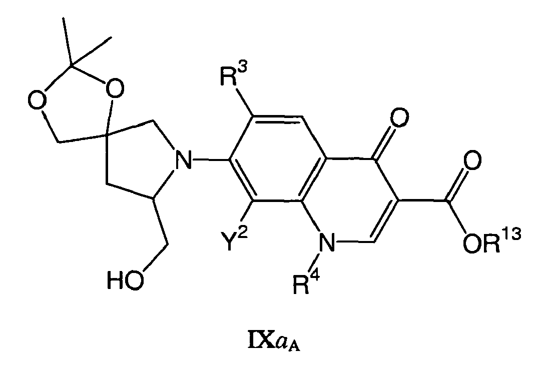 wherein R³, R⁴, Y² and R¹³ have the same meaning as in formula IX*a*.

According to a third variant, the compounds of formula IX*a* will also correspond to the formula: 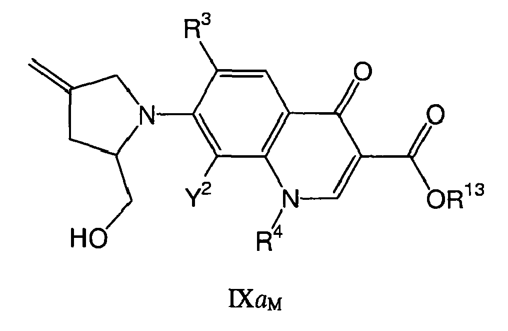 wherein R³, R⁴, Y² and R¹³ have the same meaning as in formula IX*a*.

The following compounds of formula IX*a* will be preferred:
- 1-cyclopropyl-6,8-difluoro-7-((*2S*)-2-hydroxymethyl-4-methylene-pyrrolidin-1-yl)-4-oxo-1,4-dihydro-quinoline-3-carboxylic acid ethyl ester;
- 1-cyclopropyl-6,8-difluoro-7-((*2R*)-2-hydroxymethyl-4-methylene-pyrrolidin-1-yl)-4-oxo-1,4-dihydro-quinoline-3-carboxylic acid ethyl ester;
as well as the salts of these compounds.

Other intermediates useful in the preparation of the compounds of formula I are the compounds of formula IX*b* 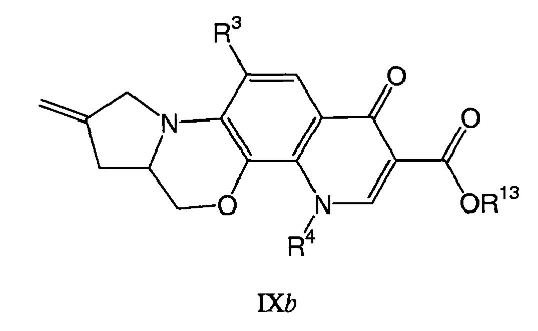 wherein
R³ and R⁴ are as defined in formula I, and
R¹³ is H, alkyl or arylalkyl;
as well as the salts of compounds of formula IX*b*.

The following compounds of formula IX*b* will be preferred:
- (*13S*)-1-cyclopropyl-7-fluoro-16-methylene-4-oxo-1,4,13,15,16,17-hexahydro-12*H*-11-oxa-1,14-diaza-cyclopenta[*a*]phenanthrene-3-carboxylic acid ethyl ester;
- (*13R*)-1-cyclopropyl-7-fluoro-16-methylene-4-oxo-1,4,13,15,16,17-hexahydro-12*H*-11-oxa-1,14-diaza-cyclopenta[*a*]phenanthrene-3-carboxylic acid ethyl ester;
as well as the salts of these compounds.

The invention further relates, as intermediates useful in the preparation of the compounds of formula I, to the compounds of formula X 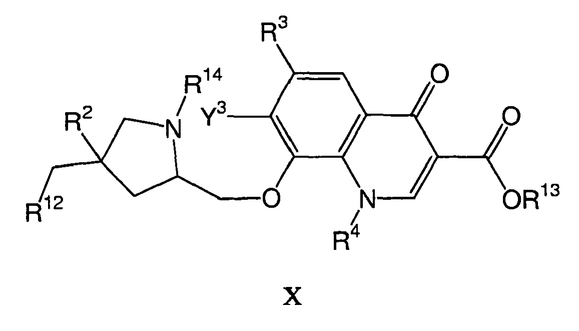 wherein
R² and R¹² are each OH or R² and R¹² form, together with the carbon atoms that they are attached to, an acetonide ring, or also R¹² and R² together are connected to form a double bond,
Y³ is a halogen atom,
R³ and R⁴ are as defined in formula I,
R¹³ is H, alkyl or arylalkyl, and
R¹⁴ is H, alkoxycarbonyl, alkenyloxycarbonyl or benzyloxycarbonyl;
and to salts of compounds of formula X.

According to a first variant, the compounds of formula X will also correspond to the formula: 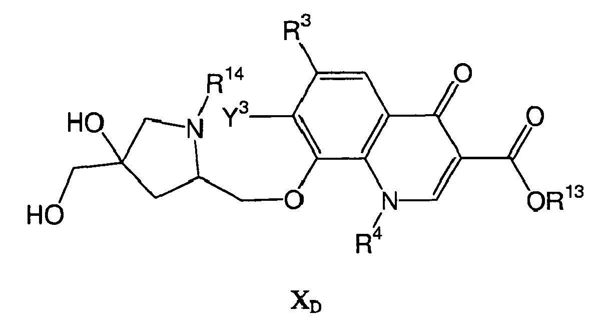 wherein Y³, R³, R⁴, R¹³ and R¹⁴ have the same meaning as in formula X.

According to a second variant, the compounds of formula X will also correspond to the formula: 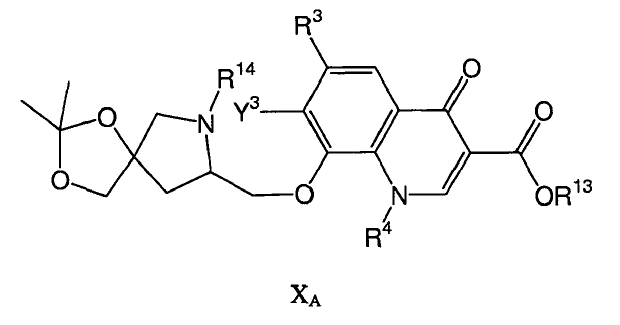 wherein Y³, R³, R⁴, R¹³ and R¹⁴ have the same meaning as in formula X.

According to a third variant, the compounds of formula X will also correspond to the formula: 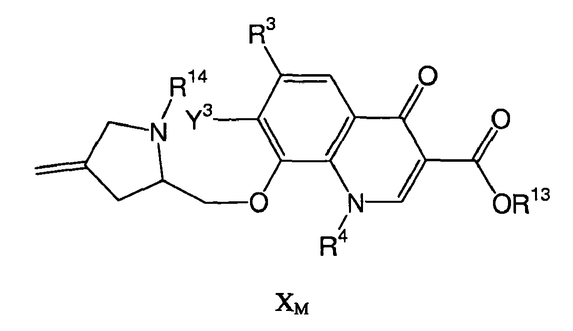 wherein Y³, R³, R⁴, R¹³ and R¹⁴ have the same meaning as in formula X.

The following compounds of formula X will be preferred:
- 8-((*2S*)-1-*tert*-butoxycarbonyl-4-methylene-pyrrolidin-2-ylmethoxy)-1-cyclopropyl-6,7-difluoro-4-oxo-1,4-dihydro-quinoline-3-carboxylic acid ethyl ester;
- 8-((*2S*)-1-*tert*-butoxycarbonyl-4-methylene-pyrrolidin-2-ylmethoxy)-1-cyclopropyl-6,7-difluoro-4-oxo-1,4-dihydro-quinoline-3-carboxylic acid;
- 8-((*2S*)-1-*tert*-butoxycarbonyl-4-methylene-pyrrolidin-2-ylmethoxy)-1-cyclopropyl-6,7-difluoro-4-oxo-1,4-dihydro-quinoline-3-carboxylic acid benzyl ester;
- 8-[(*2S*,*4S*))-(1-*tert*-butoxycarbonyl-4-hydroxy-4-hydroxymethyl-pyrrolidin-2-ylmethoxy)]-1-cyclopropyl-6,7-difluoro-4-oxo-1,4-dihydro-quinoline-3-carboxylic acid benzyl ester;
- 1-cyclopropyl-6,7-difluoro-8-[(*2S*,*4S*)-4-hydroxy-4-hydroxymethyl-pyrrolidin-2-ylmethoxy]-4-oxo-1,4-dihydro-quinoline-3-carboxylic acid benzyl ester;
- 8-[(*2S*,*4R*))-(1-*tert*-butoxycarbonyl-4-hydroxy-4-hydroxymethyl-pyrrolidin-2-ylmethoxy)]-1-cyclopropyl-6,7-difluoro-4-oxo-1,4-dihydro-quinoline-3-carboxylic acid benzyl ester;
- 1-cyclopropyl-6,7-difluoro-8-[(*2S*,*4R*)-4-hydroxy-4-hydroxymethyl-pyrrolidin-2-ylmethoxy]-4-oxo-1,4-dihydro-quinoline-3-carboxylic acid benzyl ester;
- 8-((*2R*)-1-*tert*-butoxycarbonyl-4-methylene-pyrrolidin-2-ylmethoxy)-1-cyclopropyl-6,7-difluoro-4-oxo-1,4-dihydro-quinoline-3-carboxylic acid ethyl ester;
- 8-((*2R*)-1-*tert*-butoxycarbonyl-4-methylene-pyrrolidin-2-ylmethoxy)-1-cyclopropyl-6,7-difluoro-4-oxo-1,4-dihydro-quinoline-3-carboxylic acid;
- 8-((*2R*)-1-*tert*-butoxycarbonyl-4-methylene-pyrrolidin-2-ylmethoxy)-1-cyclopropyl-6,7-difluoro-4-oxo-1,4-dihydro-quinoline-3-carboxylic acid benzyl ester;
- 8-[(*2R*,*4R*))-(1-*tert*-butoxycarbonyl-4-hydroxy-4-hydroxymethyl-pyrrolidin-2-ylmethoxy)]-1-cyclopropyl-6,7-difluoro-4-oxo-1,4-dihydro-quinoline-3-carboxylic acid benzyl ester;
- 8-[(*2R*,*4S*))-(1-*tert*-butoxycarbonyl-4-hydroxy-4-hydroxymethyl-pyrrolidin-2-ylmethoxy)]-1-cyclopropyl-6,7-difluoro-4-oxo-1,4-dihydro-quinoline-3-carboxylic acid benzyl ester;
- 1-cyclopropyl-6,7-difluoro-8-[(*2R*,*4R*)-4-hydroxy-4-hydroxymethyl-pyrrolidin-2-ylmethoxy]-4-oxo-1,4-dihydro-quinoline-3-carboxylic acid benzyl ester;
- 1-cyclopropyl-6,7-difluoro-8-[(*2R*,*4S*)-4-hydroxy-4-hydroxymethyl-pyrrolidin-2-ylmethoxy]-4-oxo-1,4-dihydro-quinoline-3-carboxylic acid benzyl ester;
- 8-((*S*)-1-*tert*-butoxycarbonyl-4-methylene-pyrrolidin-2-ylmethoxy)-1-ethyl-6,7-difluoro-4-oxo-1,4-dihydro-quinoline-3-carboxylic acid ethyl ester;
- 8-((*S*)-1-*tert*-butoxycarbonyl-4-methylene-pyrrolidin-2-ylmethoxy)-1-ethyl-6,7-difluoro-4-oxo-1,4-dihydro-quinoline-3-carboxylic acid;
- 8-((*S*)-1-*tert*-butoxycarbonyl-4-methylene-pyrrolidin-2-ylmethoxy)-1-ethyl-6,7-difluoro-4-oxo-1,4-dihydro-quinoline-3-carboxylic acid benzyl ester;
- 8-((*2S*,*4S*)-1-*tert*-butoxycarbonyl-4-hydroxy-4-hydroxymethyl-pyrrolidin-2-ylmethoxy)-1-ethyl-6,7-difluoro-4-oxo-1,4-dihydro-quinoline-3-carboxylic acid benzyl ester;
- 1-ethyl-6,7-difluoro-8-((*2S*,*4S*)-4-hydroxy-4-hydroxymethyl-pyrrolidin-2-ylmethoxy)-4-oxo-1,4-dihydro-quinoline-3-carboxylic acid benzyl ester;
- 8-((*2S*)-1-*tert*-butoxycarbonyl-4-methylene-pyrrolidin-2-ylmethoxy)-1-cyclopropyl-7-fluoro-4-oxo-1,4-dihydro-quinoline-3-carboxylic acid ethyl ester;
- (*2S*)-8-(1-*tert*-butoxycarbonyl-4-methylene-pyrrolidin-2-ylmethoxy)-1-cyclopropyl-7-fluoro-4-oxo-1,4-dihydro-quinoline-3-carboxylic acid;
- (*2S*)-8-(1-*tert*-butoxycarbonyl-4-methylene-pyrrolidin-2-ylmethoxy)-1-cyclopropyl-7-fluoro-4-oxo-1,4-dihydro-quinoline-3-carboxylic acid benzyl ester;
- (*2S*,*4S*)-8-(1-*tert*-butoxycarbonyl-4-hydroxy-4-hydroxymethyl-pyrrolidin-2-ylmethoxy)-1-cyclopropyl-7-fluoro-4-oxo-1,4-dihydro-quinoline-3-carboxylic acid benzyl ester;
- (*2S*,*4S*)-2-(3-benzyloxycarbonyl-1-cyclopropyl-7-fluoro-4-oxo-1,4-dihydro-quinolin-8-yloxymethyl)-4-hydroxy-4-hydroxymethyl-pyrrolidine;
- 8-[(*2S*,*4R*)-1-*tert*-butoxycarbonyl-4-(*tert*-butyl-dimethyl-silanyloxymethyl)-pyrrolidin-2-ylmethoxy]-1-cyclopropyl-6,7-difluoro-4-oxo-1,4-dihydro-quinoline-3-carboxylic acid ethyl ester;
- 8-[(*2S*,*4S*)-1-*tert*-butoxycarbonyl-4-(*tert*-butyl-dimethyl-silanyloxymethyl)-pyrrolidin-2-ylmethoxy]-1-cyclopropyl-6,7-difluoro-4-oxo-1,4-dihydro-quinoline-3-carboxylic acid ethyl ester;
- 8-[(*2S*,*4R*)-1-*tert*-butoxycarbonyl-4-(*tert*-butyl-dimethyl-silanyloxymethyl)-pyrrolidin-2-ylmethoxy]-1-cyclopropyl-6,7-difluoro-4-oxo-1,4-dihydro-quinoline-3-carboxylic acid;
- 8-[(*2S*,*4S*)-1-*tert*-butoxycarbonyl-4-(*tert*-butyl-dimethyl-silanyloxymethyl)-pyrrolidin-2-ylmethoxy]-1-cyclopropyl-6,7-difluoro-4-oxo-1,4-dihydro-quinoline-3-carboxylic acid;
- 8-[(*2S*,*4R*)-1-*tert*-butoxycarbonyl-4-(*tert*-butyl-dimethyl-silanyloxymethyl)-pyrrolidin-2-ylmethoxy]-1-cyclopropyl-6,7-difluoro-4-oxo-1,4-dihydro-quinoline-3-carboxylic acid benzyl ester;
- 8-[(*2S*,*4S*)-1-*tert*-butoxycarbonyl-4-(*tert*-butyl-dimethyl-silanyloxymethyl)-pyrrolidin-2-ylmethoxy]-1-cyclopropyl-6,7-difluoro-4-oxo-1,4-dihydro-quinoline-3-carboxylic acid benzyl ester;
- 8-[(*2S*,*4R*)-1-*tert*-butoxycarbonyl-4-hydroxymethyl-pyrrolidin-2-ylmethoxy)-1-cyclopropyl-6,7-difluoro-4-oxo-1,4-dihydro-quinoline-3-carboxylic acid benzyl ester;
- 8-[(*2S*,*4S*)-1-*tert*-butoxycarbonyl-4-hydroxymethyl-pyrrolidin-2-ylmethoxy)-1-cyclopropyl-6,7-difluoro-4-oxo-1,4-dihydro-quinoline-3-carboxylic acid benzyl ester;
- 1-cyclopropyl-6,7-difluoro-8-((*2S*,*4R*)-4-hydroxymethyl-pyrrolidin-2-ylmethoxy)-4-oxo-1,4-dihydro-quinoline-3-carboxylic acid benzyl ester;
- 1-cyclopropyl-6,7-difluoro-8-((*2S*,*4S*)-4-hydroxymethyl-pyrrolidin-2-ylmethoxy)-4-oxo-1,4-dihydro-quinoline-3-carboxylic acid benzyl ester;
as well as the salts of these compounds.

Besides, any preferences indicated for the compounds of formula I (whether for the compounds themselves, salts thereof, compositions containing the compounds or salts thereof, uses of the compounds or salts thereof, etc.) apply *mutatis mutandis* to compounds of formula I_{CE}, compounds of formula I_{T1}, compounds of formula I_{T2}, compounds of formula I_{C1}, compounds of formula I_{C2}, compounds of formula III_{G}, compounds of formula III_{GH}, compounds of formula III_{GP}, compounds of formula III_{GS}, compounds of formula III_{GA}, compounds of formula III_{GE}, compounds of formula IX*a*, compounds of formula IX*a*_{D}, compounds of formula IX*a*_{A}, compounds of formula IX*a*_{M}, compounds of formula IX*b*, compounds of formula X, compounds of formula X_{D}, compounds of formula X_{A} and compounds of formula X_{M}.

According to the invention, the compounds of formula I can be prepared by the process described below.

### Preparation of the compounds of formula I

### Abbreviations:

The following abbreviations are used throughout the specification and the examples:

| | |
|---|---|
| AcOH | acetic acid |
| AD-mix α | 1,4-*bis*(dihydroquinine)phthalazine, K₃Fe(CN)₆, K₂CO₃ and K₂OsO₄.2H₂O |
| AD-mix β | 1,4-*bis*(dihydroquinidine)phthalazine, K₃Fe(CN)₆, K₂CO₃ and K₂OsO₄.2H₂O |
| Alloc | allyloxycarbonyl |
| aq. | aqueous |
| atm | atmosphere |
| 9-BBN | 9-borabicyclo[3.3.1]nonane |
| BnBr | benzyl bromide |
| Boc | *tert*-butoxycarbonyl |
| *t*-BuOK | potassium *tert*-butylate |
| Cbz | benzyloxycarbonyl |
| DBU | 1,8-diazabicyclo[5.4.0]undec-7-ene |
| DCC | dicyclohexylcarbodiimide |
| DCM | dichloromethane |
| (DHQ)₂PHAL | 1,4-*bis*(dihydroquinine)phthalazine |
| DIAD | diisopropyl azo dicarboxylate |
| DIPEA | *N*,*N*-diisopropylethylamine |
| DMA | dimethylacetamide |
| DMF | *N*,*N*-dimethylformamide |
| DMSO | dimethylsulfoxide |
| dr | diastereomeric ratio |
| EA | ethyl acetate |
| EDC | 1-(dimethylaminopropyl)-3-ethylcarbodiimide hydrochloride |
| ESI | electron spray Ionisation |
| ether or Et₂O | diethyl ether |
| h | hour |
| HATU | *O*-(7-azabenzotriazol-1-yl)-*N*,*N*,*N'*,*N'*-tetramethyluronium hexafluorophosphate |
| Hept | heptane |
| Hex | *n*-hexane |
| HOBT | 1-hydroxybenzotriazole |
| HV | high vacuum |
| LDA | lithium diisopropylamide |
| ML | mother liquor |
| MeCN | acetonitrile |
| MeOH | methanol |
| MgSO₄ | magnesium sulfate |
| min | minutes |
| MS | mass spectroscopy |
| NaOMe | sodium methylate |
| NMP | *N*-methylpyrrolidinone |
| org. | organic |
| Pd/C | palladium on charcoal |
| PPh₃ | triphenylphosphine |
| rt | room temperature |
| sat. | saturated |
| SiO₂ | silica gel |
| TBDMSCl | *tert*-butyldimethylsilyl chloride |
| TEA | triethylamine |
| TFA | trifluoroacetic acid |
| THF | tetrahydrofuran |
| THP | tetrahydropyranyl |

### General preparation routes:

The novel compounds of formula I can be manufactured in accordance with the present invention by
a) reacting a compound of formula II 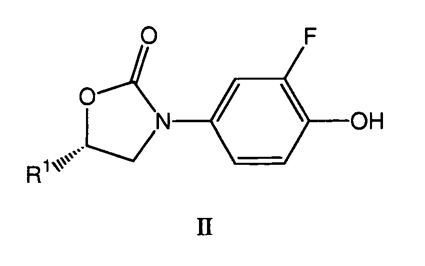 with a compound of formula III 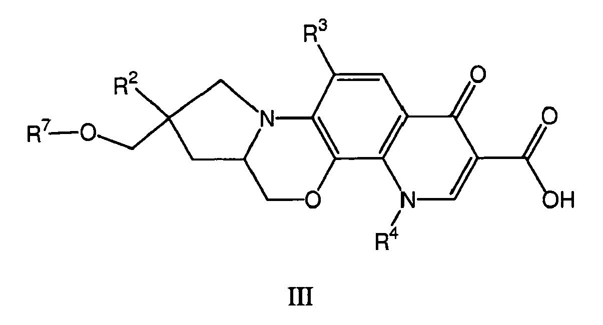 wherein R⁷ is C₁-C₃ alkylsulfonyl (e.g. methylsulfonyl), trifluoromethylsulfonyl or arylsulfonyl (e.g. phenyl- or *p*-tolyl-sulfonyl) and R² is OH or H, or R² and R⁷ together form also a bond (i.e. R² and OR⁷ form, together with the carbon atoms that carry them, an epoxide ring), or R² and OR⁷ form a cyclic carbonate, sulfate or phosphate, and the other symbols are as before, preferably between about 10°C and 100°C (more preferably between about 40°C and 80°C), in the presence of an inorganic base such as K₂CO₃ or an organic base such as TEA in an organic solvent (e.g. DMF), or, wherein R⁷ is H and R² is OH or H, under Mitsunobu conditions, R¹, R³ and R⁴ being as defined in formula I;
   or
b) ring closing a compound of formula IV 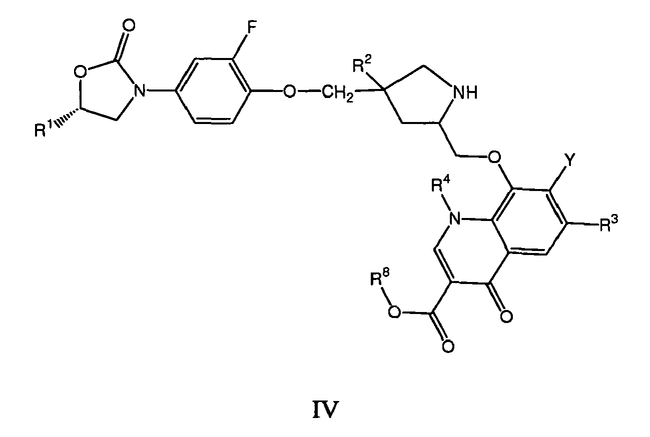 wherein Y is halogen, R⁸ is hydrogen, BF₂ or B(OC(=O)(C₁-C₄)akyl)₂, (C₁-C₅)alkyl (*e.g*. methyl, ethyl, *n*-propyl, *iso*-propyl or *tert*-butyl), (C₃-C₅)alkenyl (e.g. allyl), aryl-(C₁-C₅)alkyl (e.g. benzyl, *p*-nitrobenzyl or *p*-methoxybenzyl), tri-(C₁-C₅)alkylsilyl (e.g. trimethylsilyl or *tert*-butyldimethylsilyl) or aryl-di(C₁-C₅)alkylsilyl (e.g. phenyldimethylsilyl), and the other symbols are as before, preferably between about 10°C and 100°C, more preferably between about 40°C and 80°C in the presence of an organic base, such as TEA or DIPEA, in an organic solvent, e.g. NMP;
   R¹, R², R³ and R⁴ being as defined in formula I;
   or
c) ring closing a compound of formula V 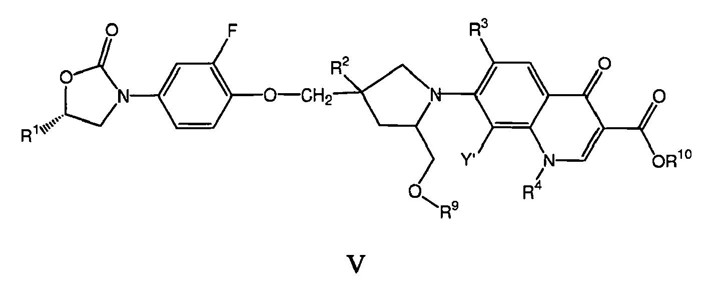 wherein Y' is halogen, R⁹ is an alkali metal such as Na, Li or K, R¹⁰ is H, (C₁-C₅)alkyl (e.g. methyl, ethyl, *n*-propyl, *iso*-propyl or *tert*-butyl), (C₃-C₅)alkenyl (e.g. allyl), aryl-(C₁-C₅)alkyl (e.g. benzyl, *p*-nitrobenzyl or *p*-methoxybenzyl), tri-(C₁-C₅)alkylsilyl (e.g. trimethylsilyl or *tert*-butyldimethylsilyl) or aryl-di(C₁-C₅)alkylsilyl (e.g. phenyldimethylsilyl), and R¹, R², R³ and R⁴ are as defined in formula I,
   or wherein Y' is OH, R⁹ is H and R¹⁰ and R¹, R², R³ and R⁴ are as defined in formula I, provided that in this case Mitsunobu conditions are used;
   or
d) converting the group W in a compound of formula VI 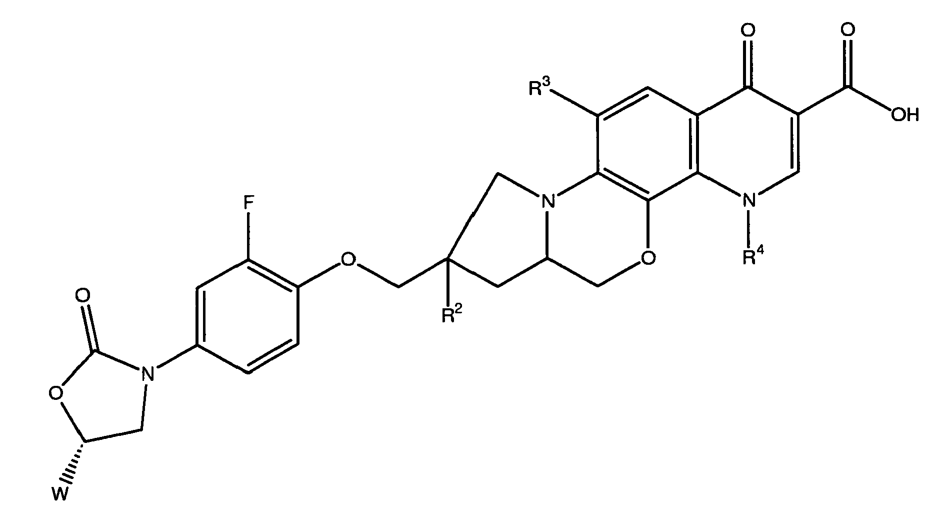 wherein R², R³ and R⁴ are as defined in formula I, into the group R¹ as follows:
   d1) converting a compound of formula VI wherein W is [(C₁-C₃)alkyl]-SO₂O-CH₂-, CF₃-SO₂O-CH₂-, aryl-SO₂O-CH₂- (e.g. phenyl-SO₂O-CH₂- or *p*-tolyl-SO₂O-CH₂-) or halogen-CH₂- into a compound of the formula I wherein R¹ is heteroaryl-methyl wherein the heteroaryl contains a basic nitrogen atom, or also heteroaryloxy-methyl or heteroarylamino-methyl, by reaction with the corresponding heteroaryl, hydroxyl-heteroaryl or amino-heteroaryl derivative in a solvent like THF, MeCN, DMF in presence of an organic base such as TEA at a temperature between about 0°C and 80°C;
      or
   d2) converting a compound of formula VI wherein W is N₃-CH₂- into a compound of the formula I wherein R¹ is 1,2,3-triazol-1-yl-methyl by cycloaddition with norbomadiene or an alkyne derivative in the presence of copper iodide between about 10°C and 60°C in an aprotic organic solvent like THF, MeCN, DMF, toluene or EA at a pressure between 1 and 10 bars;
      or
   d3) converting a compound of formula VI wherein W is -CH₂-NH₂ into a compound of the formula I wherein R¹ is -CH₂NHCO-alkyl or -CH₂NHCO-haloalkyl by reaction with an activated form of the corresponding alkylcarboxylic or haloalkylcarboxylic acid obtained using an agent such as DCC, EDC, HOBT or HATU (G. Benz in Comprehensive Organic Synthesis, B.M. Trost, I. Fleming, Eds, Pergamon Press: New York (1991), vol. 6, p. 381), between -20°C and 60°C in an dry aprotic solvent like DCM, MeCN or DMF or by reaction with a carboxylic (e.g. acetic) acid anhydride in a solvent such as DCM, DMA or NMP in presence of an organic base such as pyridine to give the corresponding acylamino compounds (further activating agents are described by in Comprehensive Organic Transformations, 2nd Ed., RC. Larock Ed, (1999), Wiley-VCH p. 1932-1940);
      or
   d4) converting a compound of formula VI wherein W is NC-CH₂- into a compound of the formula I wherein R¹ is 1,2,3,4-tetrazol-5-ylmethyl by reaction with sodium azide in a solvent like MeCN or THF, at a temperature between about 0°C and 80°C, or wherein R¹ is 4-methylthiazol-2-ylinethyl by reaction with H₂S followed by methyl-bromomethylketone in a solvent like MeCN or THF, at a temperature between about 0°C and 80°C (e.g. as described in J. Chem. Research, Synopses (1995), 11, 444-5);
   or
e) converting a compound of formula VII 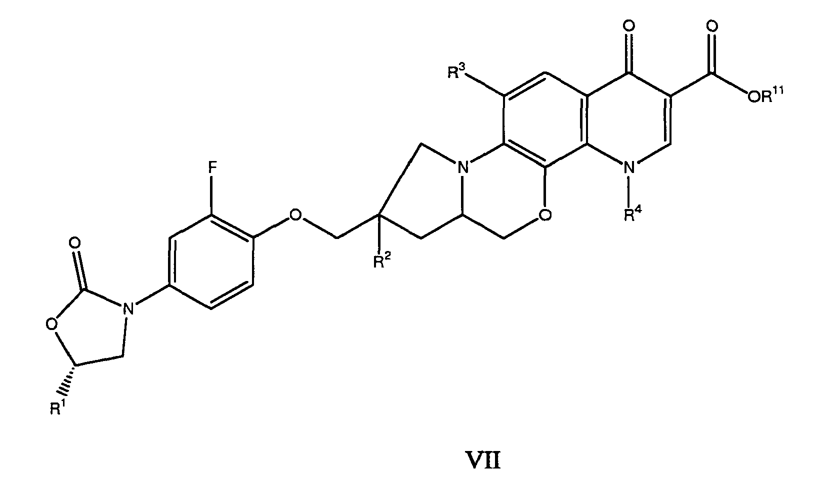 wherein R¹¹ is (C₁-C₅)alkyl (*e.g*. methyl, ethyl, *n*-propyl, *iso*-propyl or *tert*-butyl), aryl-(C₁-C₅)alkyl (*e.g*. benzyl, p-nitrobenzyl or p-methoxybenzyl), alkenyl (e.g. allyl), tri-(C₁-C₅)alkylsilyl (e.g. trimethylsilyl or *tert*-butyl dimethyl silyl) or aryl-di(C₁-C₅)alkylsilyl (e.g. phenyldimethylsilyl) and R¹, R², R³ and R⁴ are as defined in formula I into the corresponding compound of formula I by hydrolysis, saponification or hydrogenolysis (e.g. as reviewed in Protecting groups, Kocienski, P.J., Thieme (1994));
   or
f) converting a compound of formula I wherein R² is OH into a compound of formula I wherein R² is OSO₃H, OPO₃H₂, OCH₂OPO₃H₂, OCOCH₂CH₂COOH or OCOR⁶, R⁶ being the residue of a naturally occurring amino acid or of dimethylaminoglycine.

The compounds of formula I obtained according to the abovementioned general preparation methods may then, if desired, be converted into their salts, and notably into their pharmaceutically acceptable salts.

Besides, whenever the compounds of formula I are obtained in the form of mixtures of enantiomers, the enantiomers can be separated using methods known to one skilled in the art (e.g. by formation and separation of diastereomeric salts or by chromatography over a chiral stationary phase). Whenever the compounds of formula I are obtained in the form of mixtures of diasteromers they may be separated by an appropriate combination of silica gel chromatography, HPLC and crystallization techniques.

Concerning the above process, the following should be noted:
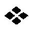 regarding variants a) and c), it is meant by "Mitsunobu conditions" the conditions described in Synthesis (1981), 1, 1-28, and notably conditions wherein the reaction is carried out in the presence of DIAD and PPh₃;
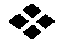 regarding variant a), the compound of formula III could also be replaced by an ester thereof (i.e. a compound of formula III_{G} as defined above wherein R¹³ represents alkyl or arylalkyl), in which case an ester hydrolysis step would follow the reaction with the compound of formula II (general methods to perform such reactions have been reviewed in Protecting groups, Kocienski, P.J., Thieme (1994));
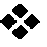 regarding variant b), when R⁸ is not H, an additional deprotection step is required (general methods to perform such reactions have been reviewed in Protecting groups, Kocienski, P.J., Thieme (1994));
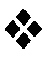 concerning variant c):
   ○ when R¹⁰ is not H, an additional deprotection step is required (general methods to perform such reactions have been reviewed in Protecting groups, Kocienski, P.J., Thieme (1994));
   ○ when R⁹ is H and Y is OH, the reaction is performed under Mitsunobu conditions (as described in Synthesis (1981), 1, 1-28);
   ○ when R⁹ is Na, Li or K and Y is halogen, the reaction is performed in a solvent such as THF, *N*,*N*-dimethylimidazoline-2-one or DMF at a temperature between about 40°C and 80°C;
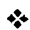 regarding variant d), the free acid function of the compound of formula VI could be protected as an alkyl or arylalkyl ester which would then be removed under standard conditions after the conversion of the group W (general methods to perform such reactions have been reviewed in Protecting groups, Kocienski, P.J., Thieme (1994)); and
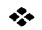 regarding variant f):
   ○ compounds of formula I wherein_R² is OSO₃H can be obtained by reaction of the corresponding compounds of formula I wherein R² is OH with a SO₃.DMF or SO₃.pyridine complex in a solvent such as DMF or THF and subsequent hydrolysis;
   ○ compounds of formula I wherein R² is OPO₃H₂ can be obtained by deprotection of the corresponding compounds wherein R² is OPO(OR)₂ and R is allyl or benzyl (according to the nature of R, various methods for deprotection may be used as reviewed in Protecting Groups, Kocienski, P., J., Thieme (1994), like for example catalytic hydrogenation over a noble catalyst such as palladium or hydrolysis with hydrobromic acid in a solvent such as AcOH when R is benzyl), the latter compounds being referred to as "compounds of formula I.P";
   ○ compounds of formula I wherein R² is OCH₂OPO₃H₂ can be obtained by reaction of compounds of formula I wherein R² is OH with di-*tert*-butyl chloromethyl phosphate in presence of a base such as NaH in a solvent such as DMF or THF and subsequent treatment with a mineral acid such as hydrochloric acid in an organic solvent such as EA (as described in J. Med. Chem. (2004), 47, 188-195);
   ○ compounds of formula I wherein R² is OCOCH₂CH₂COOH or OCOR⁶ can be obtained for example by reaction of compounds of formula I wherein R² is OH with succinic anhydride or an acid chloride of formula ClCOR⁶ under standard conditions known to one skilled in the art.

The invention also offers a new process for making key intermediates in the synthesis of certain compounds of formula I, namely the compounds of formula XIV 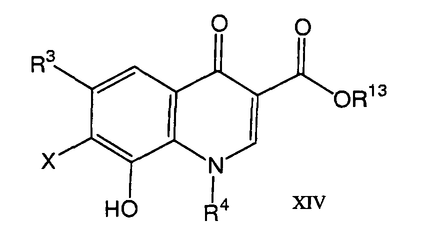 wherein X and R³ both represent fluorine, R⁴ represents cyclopropyl and R¹³ represents alkyl, alkenyl, trialkylsilyl, phenyldialkylsilyl, benzyl, p-methoxybenzyl or p-nitrobenzyl,
said process comprising the following steps:
i) reaction of 1-cyclopropyl-6,7-difluoro-8-methoxy-4-oxo-1,4-dihydro-quinoline-3-carboxylic acid with hydrobromic acid followed by addition of water to yield 1-cyclopropyl-6,7-difluoro-8-hydroxy-4-oxo-1,4-dihydro-quinoline-3-carboxylic acid;
ii) treatment of 1-cyclopropyl-6,7-difluoro-8-hydroxy-4-oxo-1,4-dihydroquinoline-3-carboxylic acid with a base followed by addition of BnBr to yield 8-benzyloxy-1-cyclopropyl-6,7-difluoro-4-oxo-1,4-dihydro-quinoline-3-carboxylic acid;
iii) treatment of 8-benzyloxy-1-cyclopropyl-6,7-difluoro-4-oxo-1,4-dihydroquinoline-3-carboxylic acid with alkyl bromide, alkenyl bromide, trialkylsilyl bromide or chloride, phenyldialkylsilyl bromide or chloride, benzyl bromide or chloride, *p*-methoxybenzyl bromide or *p*-nitrobenzyl bromide in the presence of a base to yield 8-benzyloxy-1-cyclopropyl-6,7-difluoro-4-oxo-1,4-dihydroquinoline-3-carboxylic acid ethyl ester; and
iv) hydrogenation of 8-benzyloxy-1-cyclopropyl-6,7-difluoro-4-oxo-1,4-dihydroquinoline-3-carboxylic acid ethyl ester in the presence of a catalyst to yield 1-cyclopropyl-6,7-difluoro-1,4-dihydro-8-hydroxy-4-oxo-3-quinolinecarboxylic acid ethyl ester.

Concerning the process for making the compounds of formula XIV as defined above, it is preferred that at least one of the following conditions is used:
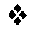 for step i), the solvent is preferably AcOH;
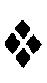 for step i), the reaction with hydrobromic acid is preferably carried out with hydrobromic acid in water or AcOH (in particular with aqueous hydrobromic acid) at a temperature above room temperature, and more preferably at a temperature above 50 or even 80°C (e.g. about 100°C);
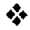 for step i), the addition of water is preferably carried out at a temperature below room temperature, and more preferably at a temperature above 10 or even 5°C (e.g. about 0°C);
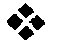 for step ii), the base used is preferably LiOH, NaOH or KOH;
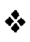 for step ii), the solvent used is preferably carried out in a polar aprotic solvent, for example DMF or THF;
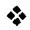 for step ii), the addition of BnBr is preferably carried out at a temperature that is about room temperature;
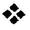 for step iii), the base used is preferably K₂CO₃ or Na₂CO₃;
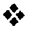 for step iii), the solvent used for carrying out the reaction is preferably a polar aprotic solvent, for example DMF or THF;
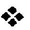 for step iii), the reaction is preferably carried out at a temperature between 40 and 80°C;
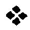 for step iv), the hydrogenation catalyst is preferably Pd(OH)₂;
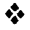 for step iv), the hydrogenation reaction is preferably carried out in a mixture of tetrahydrofuran and ethanol (which mixture is preferably a THF-EtOH mixture having a volume proportion from 10-1 to 5-1, for example 9-1).

Preferably, the process for making the compounds of formula XIV as defined above will be used for making 1-cyclopropyl-6,7-difluoro-1,4-dihydro-8-hydroxy-4-oxo-3-quinolinecarboxylic acid ethyl ester.

The compounds of formula II are obtained from (*5R*)-3-(4-benzyloxy-3-fluorophenyl)-5-hydroxymethyloxazolidin-2-one VII*a* (WO 2004/096221; see Scheme 1 below), which is transformed into its corresponding sulfonates VII*b* (wherein R' represents (C₁-C₃)alkyl-, trifluoromethyl- or aryl- (e.g. phenyl- or *p*-tolyl)) by reaction with a (C₁-C₃)alkyl-, trifluoromethyl- or aryl-sulfonyl halide such as methanesulfonyl chloride between about -30°C and 60°C, preferably between about -10°C and +30°C, in a solvent like THF or DCM in the presence of an organic base like TEA or pyridine.

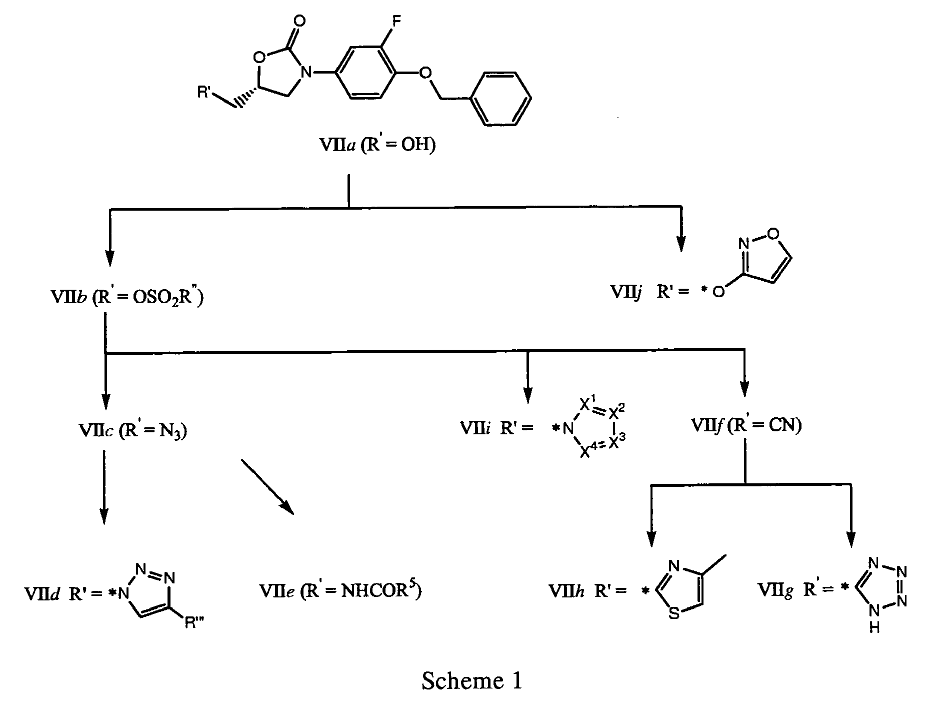

The sulfonates VII*b* are subsequently reacted between about 30°C and 150°C, preferably between about 40°C and 80°C with sodium azide in DMF. The azide derivatives VII*c* are further processed into compounds of formula VII*d* (wherein R"' is hydrogen, alkyl or hydroxymethyl; e.g. R' = [1,2,3]triazol-1-yl) by dipolar cycloaddition with norbomadiene or an alkyne derivative like acetylene, propyne or propargyl alcohol in the presence of copper iodide between about 10°C and 60°C in a solvent like THF, toluene or EA at a pressure between 1 and 10 bars. In another approach, the azides VII*c* are either reduced catalytically over noble metals such as Pd/C under hydrogen between about 10°C and 60°C in a solvent like THF, MeOH or EA or reacted with PPh₃ and water between about 10°C and 40°C in a solvent like THF. The resulting primary amine is acylated by reaction with an activated form of the corresponding alkyl- or haloalkyl-carboxylic acid obtained using an activating agent as described above (e.g. DCC, EDC, HOBT, HATU) or a carboxylic (e.g. acetic) acid anhydride to give the compounds of formula VII*e* (wherein R⁵ is alkyl or haloalkyl).

The sulfonate VII*b* can be converted into the corresponding nitrile derivative VII*f* after reaction with NaCN between 20°C and 100°C in a dry polar solvent such as MeCN or DMF. The corresponding nitrile derivative VII*f* is reacted with sodium azide to give the 1,2,3,4-tetrazol-5-yl derivative VII*g* as described in J. Org. Chem. (1950), 15, 1082-92. The intermediate nitrile VII*f* can also be transformed into the corresponding thioamide by reaction with H₂S and subsequently reacted with methyl bromomethylketone leading to compounds of formula VII*h* (R¹= 4-methylthiazol-2-yl-methyl) as described in J. Chem. Soc. (1943), Abstracts, 419-20.

The sulfonates VII*b*, can be further converted into compounds of formula VII*i* in which R¹ = pyrazol-1-yl (X¹ = N and X² = X³ = X⁴ = CH), tetrazol-1-yl (X¹ = X² = X³ = N and X⁴ = CH) or tetrazol-2-yl (X¹ = X² = X⁴ = N and X³ = CH) by reaction with pyrazole or tetrazole, respectively, as described in Synthesis (1999), 9, 1613-1624.

Compounds of formula II wherein R¹ is heteroaryloxymethyl are prepared from the intermediate hydroxymethyl-oxazolidin-2-one derivative VII*a* (Scheme 1) with a hydroxy-heteroaryl (*e.g*. 3-isoxazolol prepared according to J. Med. Chem. (2002), 45, 2454-68) under Mitsunobu conditions as reviewed in Synthesis (1981), 1 to give compound of formula VII*j*.

Compounds of formula II are obtained from compounds VII*d-j* through catalytic hydrogenation over noble catalyst such as platinum or palladium on charcoal in a solvent like EA or MeOH between about 20°C and 60°C or by treatment with aq. hydrobromic acid in a solvent such as AcOH between 20 and 80°C.

Compounds of formula III*b* wherein R² is OH, R⁷ is SO₂R¹⁵, R¹⁵ being alkyl, trifluoromethyl or aryl like phenyl or *p*-tolyl are obtained (Scheme 2) from compounds of formula III*a* wherein R⁷ is H by reaction with the corresponding sulfonyl chlorides in presence of an organic base such as TEA in a solvent such as DCM or THF between -10°C and 50°C. Compounds of formula III*b* can be used for the preparation of the spiro oxirane derivatives of formula III*c*, which reaction is carried out under basic conditions (e.g. with Na₂CO₃ in a solvent like DMF). Compounds of formula III*a* wherein R⁷ = H are obtained from compounds of formula III*a* wherein R⁷ is PG. Typical protecting groups (PG) that can be used are THP ethers, methoxymethyl or 2-methoxyethoxymethyl ethers, allyl ethers, trialkylsilyl ethers or alkyl esters; their formation and removal are described in Protecting groups, Kocienski, P.J., Thieme (1994). Compounds of formula III*a* (wherein R¹³ is H, alkyl or arylalkyl and R⁷ is PG) are prepared by intramolecular ring closure, either under Mitsunobu conditions (when Y² is OH) or, after transformation of the primary alcohol function of compound of formula IX*a* (wherein Y² is OH) into its alkyl- or aryl-sulfonate, under basic conditions such as Na₂CO₃ or DBU in a solvent like THF or DMF between 20°C and 100°C. Compounds of formula III*a* can also be prepared from compounds of formula IX*a* wherein Y² = F by intramolecular ring closure in presence of a strong base such as NaH, LDA, DBU or an alkali alkoxylate. In the particular case wherein R¹² and R² together are connected to form a double bond, the resulting compound of formula IX*b* is subjected to a subsequent asymmetric cis dihydroxylation to yield the corresponding compound of formula III*a*. When R¹² and R² together form an acetonide, the resulting acetonide derivative of formula III*a* is treated with an acid to give the corresponding compound of formula III*a* wherein R² = OH and R⁷ = H. Alternatively, compounds of formula III*a* wherein R⁷ is H or PG can be obtained from compounds of formula X*b* (wherein R¹³ is H, alkyl or arylalkyl and R¹⁴ is H) after intramolecular ring closure in a solvent like THF, NMP or DMF between 20°C and 100°C. If R¹³ is alkyl or arylalkyl, the free acid of formula III is liberated according to standard procedures as described in Protecting groups, Kocienski, P.J., Thieme (1994) (e.g. hydrogenation over Pd/C for R¹³ = benzyl; acidic treatment with TFA or a solution of HCl in an organic solvent such as THF or MeOH for R¹³ = *tert*-butyl; acidic or basic hydrolysis for R¹³ = methyl or ethyl).

Compounds of formula IX*a* are obtained (Scheme 2) from the 7,8-dihalo-1,4-dihydro-4-oxoquinoline-3-carboxylic acid derivatives XIII (wherein Y¹ and Y² are both halogen atoms and R¹³ is H, alkyl or arylalkyl) by reaction with the pyrrolidine derivatives of formula XI (wherein R¹² is O-PG and R² is H or OH or R¹² and R² together are connected to form a double bond or R¹² and R² together form an acetonide and the other symbols are as before), in a polar solvent such as NMP in the presence of an organic base like TEA or DIPEA, between about 30°C and 100°C, preferably between about 50°C and 80°C.

Compounds of formula X*a* are obtained (Scheme 2) by reacting 8-hydroxy-7-halo-1,4-dihydro-4-oxo-quinoline-3-carboxylic acid derivatives XIV either with alcohol derivatives of formula XII (wherein PG can be e.g. Boc, Alloc or Cbz - a variety of other protecting groups may however be used as reviewed in Protecting groups, Kocienski, P.J., Thieme (1994)) under Mitsunobu conditions or with the corresponding alkyl or aryl sulfonates of the alcohol XII obtained after reaction with a (C₁-C₃)alkylsulfonyl halide (e.g. methylsulfonyl chloride) or aryl-sulfonyl halide (like phenyl- or *p*-toluenesulfonyl chloride) between about -30°C and 60°C, preferably between about -10°C and +30°C, in a solvent like THF or DCM in the presence of an organic base like TEA or pyridine. Compounds of formula X*b* (R¹⁴ = H) are obtained by removal of the protecting group of the compounds of formula X*a* (R¹⁴ = PG) using standard methods. According to the nature of the protecting group, several strategies may be used to unmask the amino group, such as using TFA in the case of Boc and Cbz or hydrogenolysis using a catalyst such as Pd/C and hydrogen in the case of the Cbz group.

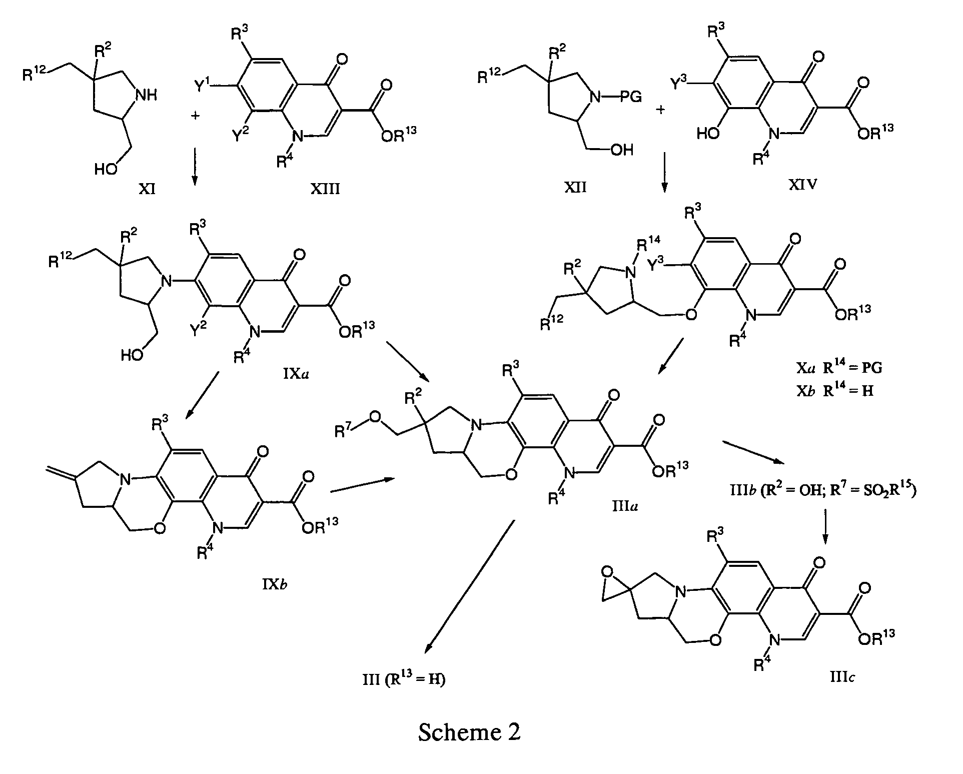

Compounds of formula XI wherein R¹² and R² are both OH are prepared from the corresponding 4-methylidene derivatives by Sharpless asymmetric dihydroxylation using AD-mix α or β. (J. Org. Chem. (1992), 57, 2768). The primary alcohol function is optionally transformed into its corresponding sulfonate by reaction with an alkyl or aryl sulfonyl chloride as described above.

Compounds of formula XI wherein R¹² and R² form a double bond are prepared from 2-(hydroxymethyl)-4-methylene-1-pyrrolidinecarboxylic acid 1,1-dimethylethyl ester (prepared according to Tetrahedron (1997), 53(2), 539-556) after treatment with HCl in an organic solvent such as THF or EA, or with TFA neat or in an organic solvent such as DCM.

Compounds of formula XI wherein R¹² and R² form an acetonide are prepared from 8-[[[(1,1-dimethylethyl)diphenylsilyl]oxy]methyl]-2,2-dimethyl-7-(phenylmethyl)-1,3-dioxa-7-azaspiro[4.4]nonane (prepared according to Carbohydrate Research (1995), 279, 307-14) after removal of the silyl protecting group with e.g. HF in MeCN and removal of the benzyl protecting group (e.g. by hydrogenation over a noble metal).

Compounds of formula XI wherein R¹² is a protecting group and R² is OH are prepared from 2-[[[(1,1-dimethylethyl)dimethylsilyl]oxy]methyl]-4-methylene-1-pyrrolidinecarboxylic acid 1,1-dimethylethyl ester (prepared according to J. Org. Chem. (2003), 68(10), 3923-3931) after Sharpless asymmetric dihydroxylation using AD-mix α or β (J. Org. Chem. (1992), 57, 2768), selective protection of the primary alcohol with a acid stable protecting group such as benzyl or Alloc and removal of both the silyl and Boc protecting groups under acidic conditions.

Compounds of formula XI wherein R² is H and R¹² is OH are obtained by hydroboration of the corresponding 4-methylidene derivatives of formula XII wherein R¹² and R² form a bond with borane-dimethyl sulfide complex or 9-BBN as described in J. Am. Chem. Soc. (1968), 90, 5281, followed by removal of the nitrogen protecting group.

Compounds of formula IV are obtained (Scheme 3) by *N*-deprotecting compounds of formula XV using standard conditions (*e.g*. TFA neat or diluted in an organic solvent such as DCM or HCl in an organic solvent such as ether or THF for Boc). Compounds of formula XV are obtained by coupling 8-hydroxy-7-halo-1,4-dihydro-4-oxo-quinoline-3-carboxylic acid derivatives of formula XIV with a compound of formula XVI either under Mitsunobu conditions or after prior transformation of the primary alcohol function of XVI into its corresponding mesylate and treatment with an inorganic base such as Na₂CO₃ or an organic base such as DBU.

Compounds of formula XVI are obtained by reacting a compound of formula XII, wherein R¹² and R² form an epoxide or R¹² is OH and R² is H, with a compound of formula II under the conditions described for the reaction of compounds of formula II with compounds of formula III.

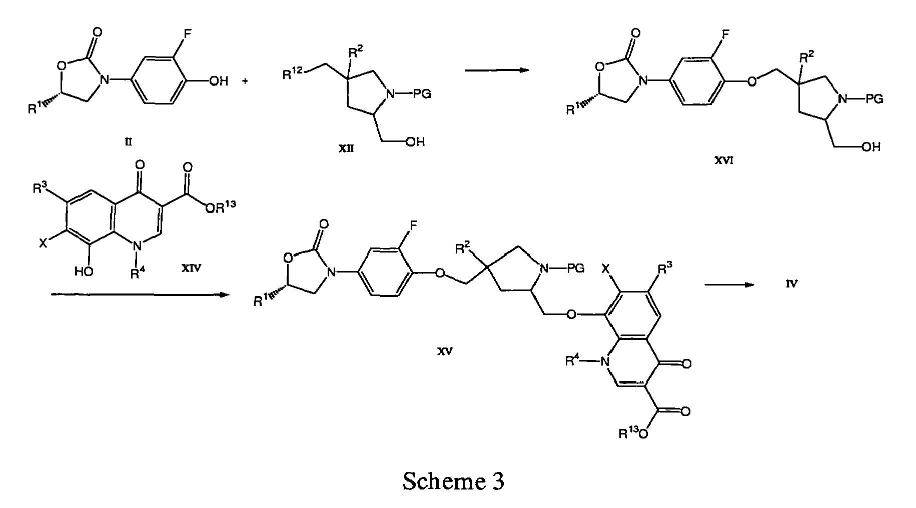

Compounds of formula V are obtained (Scheme 4) by reacting compounds of formula XVII with 7,8-dihalo-1,4-dihydro-4-oxoquinoline-3-carboxylic acid derivatives of formula XIII under the same conditions as for the preparation of compounds of formula IX*a*. Compounds of formula XVII are prepared by deprotection of compounds of formula XVI using standard methods.

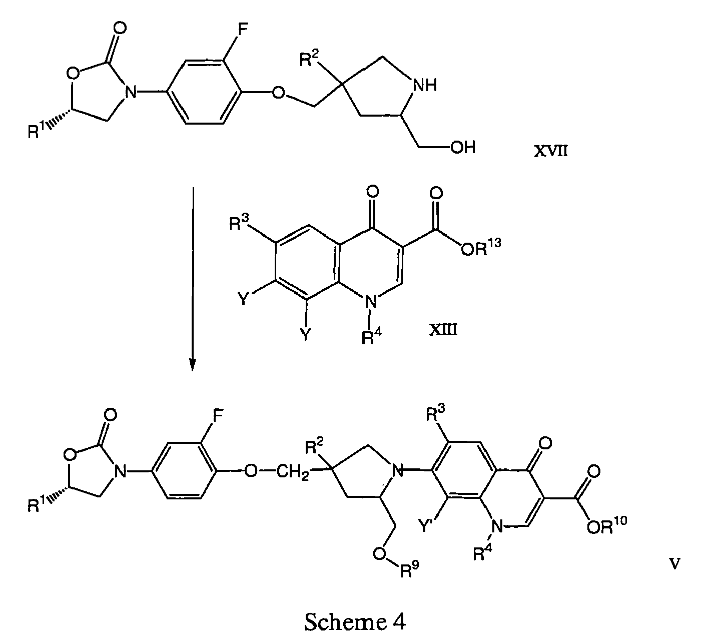

Intermediates of formula I.P 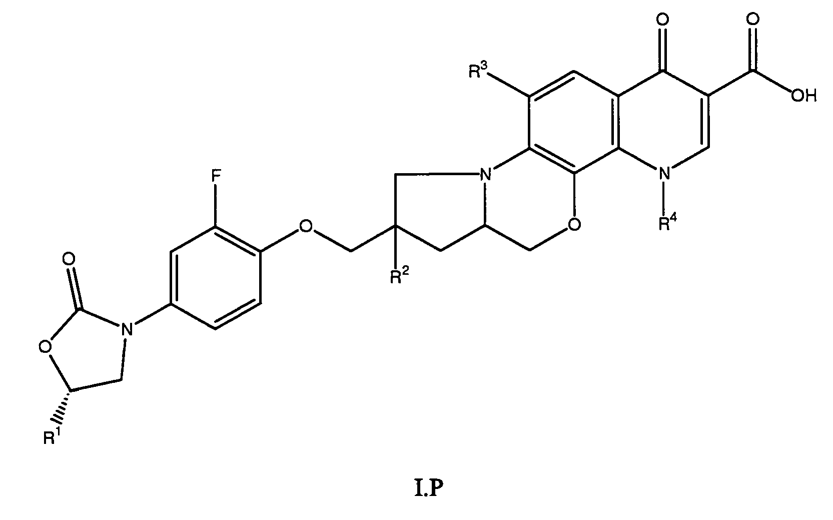 wherein R² is OPO(OR)₂ and R¹, R³ and R⁴ are as defined in formula I are obtained by reaction of compound of formula I wherein R² is OH with either ω-PO(OR)₂ wherein ω is diisopropylamino or halogen and R is benzyl or allyl in presence of an organic base such as TEA or with diisopropyl-phosphoramidic acid dibenzyl or diallyl ester in presence of tetrazole or 4,4-dicyanoimidazole in a solvent such as DCM between -10°C and 50°C followed by oxidation with *tert*-butyl hydroperoxide.

The following examples further illustrate the preparation of the pharmacologically active compounds of the invention but do not limit the scope thereof.

### EXAMPLES

All temperatures are stated in °C. All analytical and preparative HPLC investigations on non-chiral phases are performed using RP-C18 based columns. Analytical HPLC investigations are performed on two different instruments with cycle-times of ∼2.5 min and ∼3.5 min respectively. Unless otherwise stated, the values indicated for MS correspond to the main peaks ((M+H)⁺ with a variation of +/- 0.5 unit). In NMR spectra, coupling constants J are given in Hz and quint. refers to a quintuplet.

### Example 1: (13S,16S)-1-cyclopropyl-7-fluoro-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

*Note: two synthetic approaches, i.e. Approach A and Approach B described hereafter, have been used for preparing the compound of Example 1.*

### APPROACH A:

### 1.A.i. (5R)-3-(4-benzyloxy-3-fluoro-phenyl)-5-[1,2,3]triazol-1-ylmethyl-oxazolidin-2-one:

A solution of (*5R*)-5-azidomethyl-3-(4-benzyloxy-3-fluoro-phenyl)-oxazolidin-2-one (1 g, 2.92 mmol; prepared according to WO 2004/096221) and 2,5-norbomadiene (1 ml, 9.93 mmol) in 10 ml dioxane was refluxed for 24 h. The dioxane was evaporated under reduced pressure and the residue was stirred in EA. The crystals were collected by filtration and dried in vacuum to afford 0.906 g (84%) of pink solid.

¹H NMR (DMSO_{d6}; δ ppm): 3.85-3.88 (dd, 1H, J = 8 and J = 6, N-CH₂); 4.15-4.23 (dd, 1H, J1 = J2 = 8, N-CH₂); 4.81-4.83 (d, 2H, J = 6, triazole-CH₂); 5.07-5.14 (m, 1H, O-CH); 5.17 (s, 2H, O-CH₂); 7.09-7.42 (m, 1H, H-phenyl); 7.26-7.31 (m, 2H, H-phenyl); 7.35-7.50 (m, 5H, H-benzyl); 7.76 (s, 1H, H-triazole); 8.16 (s, 1H, H-triazole). MS (ESI+): 369.5.

### 1.A.ii. (5R)-3-(3-fluoro-4-hydroxy-phenyl)-5-[1,2,3]triazol-1-ylmethyl-oxazolidin-2-one:

A suspension of 10% Pd(OH)₂ (1 g) and intermediate 1.A.i (13.68 g; 37.2 mmol) in 500 ml of THF/MeOH 8:2 was stirred under 1 atm hydrogen. The reaction was further stirred overnight, warmed to 40°C and the catalyst was filtered off. The filtrate was evaporated under reduced pressure and the residue was stirred in EA and filtered. The off-white solid was dried under HV to afford 4.55 g of material. The grey catalyst cake was stirred in DMF (100 ml) and filtered. The filtrate was evaporated and the residue was stirred in EA. The dark solid was collected and combined to the first crop to afford 8.14 g (78.7%) of the expected compound.

¹H NMR (DMSO_{d6}; δ ppm): 3.85 and 4.17 (2xdd, 2x1H, N-CH₂, J = 9 and J = 5); 4.82 (d, 2H, J = 2, CH₂-triazole); 5.08 (m, 1H, O-CH-CO); 6.91-7.03 (m, 2H, H-phenyl); 7.37 (dd, 1H, J = 2, H-phenyl); 7.77 (s, 1H, triazole); 8.16 (s, 1H, triazole); 9.75 (s, 1H, phenol).

MS (ESI+): 279.1.

### 1.A.iii. (2S)-(4-methylene-pyrrolidin-2-yl)-methanol hydrochloride:

A solution of (*2S*)-2-(*tert*-butyl-dimethyl-silanyloxymethyl)-4-methylene-pyrrolidine-1-carboxylic acid *tert*-butyl ester (7.7 g; prepared according to Goodman et al., J. Org. Chem. (2003), 68, 3923-31) in 80 ml of 1.25*M* HCl in MeOH was treated with 80 ml of 1.25 M HCl in MeOH. The reaction was stirred at 40°C for 2 h. The reaction mixture was evaporated under reduced pressure and the residue was taken up in ether and stirred for 1 h. The crystals were collected by filtration under a stream of dry nitrogen and dried under HV affording 3.36 g (95%) of colourless crystals.

¹H NMR (DMSO_{d6}; δ ppm): 2.35-2.44 (1H, m); 2.60-2.68 (1H, m); 3.54-3.84 (5H, m); 5.4 (1H, s); 9.2 (s, 1H); 9.95 (s, 1H).

MS (ESI+): 114.1 (base).

### 1.A.iv. 1-cyclopropyl-6,8-difluoro-7-((2S)-2-hydroxymethyl-4-methylene-pyrrolidin-1-yl)-4-oxo-1,4-dihydro-quinoline-3-carboxylic acid ethyl ester:

A solution of 3.11 g of 1-cyclopropyl-6,7,8-trifluoro-4-oxo-1,4-dihydro-quinoline-3-carboxylic acid ethyl ester (commercial) and intermediate 1.A.iii (1.64 g) in NMP (8 ml) and 1,3-dimethyl-2-imidazolone (2 ml) was treated with NaHCO₃ (1.68 g) and DIPEA (0.856 ml) and heated at 110°C under stirring. The reaction was monitored by HPLC and the reaction mixture cooled after 27 h. The NMP was evaporated under reduced pressure, the residue dissolved in DCM and washed with water and brine. The org. layer was dried over MgSO₄, filtered and the filtrate evaporated under reduced pressure. The residue was stirred in an ether/Hex mixture. The solid was collected by filtration and dried *in vacuo* to afford 2.456 g of beige solid. The ML was purified by chromatography over SiO₂, using EA then DCM/MeOH 95/5 as eluent. The relevant fractions were collected and evaporated under reduced pressure. The residue was stirred in EA/Hex to afford a second crop of 0.3 g of beige crystals, identical to the first crop by LC/MS analysis. Total yield: 2.75 g (68%).

¹H NMR (DMSO_{d6}; δ ppm): 1-1.79 (4H, m); 1.27 (3H, t, J = 7.5); 2.55 (1H, m); 2.75 (1H, dd, J = 8 and J = 12); 3.40 (2H, m); 3.90 (1H, bd, J = 12); 3.95 (1H, m); 4.22 (2H, q, J = 7.5); 4.27 (1H, m); 4.45 (1H, bd, J = 12); 4.75 (1H, t, J = 4), 5.02 (2H, s); 7.62 (1H, dd, J = 2 and J = 11); 8.42 (1H, s).

### 1.A.v. (13S)-1-cyclopropyl-7-fluoro-16-methylene-4-oxo-1,4,13,15,16,17-hexahydro-12H-11-oxa-1,14-diaza-cyclopenta[a]phenanthrene-3-carboxylic acid ethyl ester:

A solution of intermediate 1.A.iv (2.75 g) in 1,3-dimethyl-2-imidazolidinone (6.8 ml) was treated at rt with NaH (230 mg). The reaction was stirred at rt and monitored by HPLC. The mixture was diluted with water and the solid filtered and dried under reduced pressure to afford 0.87 g of pale yellow solid. The ML was further purified by chromatography on SiO₂, using an EA/DCM 1/1 mixture as eluent. The relevant fractions were collected and evaporated under reduced pressure. The residue was crystallized from an EA/Hex mixture to afford 158 mg of off-white solid. Total yield: 1.03 g (40%).

¹H NMR (DMSO_{d6}; δ ppm): 0.9-1.10 (4H, m); 1.26 (3H, t, J = 7.5); 2.40 (1H, bdd, J = 12 and J = 4); 2.85 (1H, bd, J =12 and J = 4); 3.52 (1H, t, J = 8); 3.65 (1H, m); 4.05 (1H, m); 4.20 (2H, q, J = 7.5); 4.40 (1H, bdd, J = 4 and J = 12); 4.70 (1H, dd, J = 3 and J = 8), 5.10 (2H, s); 7.44 (1H, d, J = 12); 8.41 (1H, s).

MS (ESI+): 385.3.

### 1.A.vi. (13S,16S)-1-cyclopropyl-7-fluoro-16-hydroxy-16-hydroxymethyl-4-oxo-1,4,13,15,16,17-hexahydro-12H-11-oxa-1,14-diaza-cyclopenta[a]phenanthrene-3-carboxylic acid methyl ester:

A mixture of *tert*-butanol (10 ml) and water (10 ml) was stirred with potassium ferricyanide III (1.56 g), potassium osmate dehydrate (0.006 g), K₂CO₃ (0.65 g) and (DHQ)₂PHAL (0.025 g) until two clear phases were formed. Intermediate 1.A.v (0.607 g) was added and the reaction mixture stirred at 0°C and monitored by HPLC. The reaction was stirred during 3 days and treated carefully at rt with sodium pyrosulfide (2.3 g). The mixture was diluted with DCM, the water layer washed twice with DCM. The combined org. layers were washed with brine, dried over MgSO₄/Fuller's earth and filtered. The filtrate was evaporated to dryness under reduced pressure. The residue was purified by chromatography over SiO₂, using DCM/MeOH 95/5 as eluent. The relevant fractions were collected and evaporated under reduced pressure to afford a foam (0.227 g; 34.4%).

¹H NMR (DMSO_{d6}; δ ppm): 0.9-1.10 (4H, m); 1.26 (3H, t, J =7.5); 1.65 (1H, dd, J = 10 and J = 11); 1.85 (1H, dd, J = 4 and J = 8); 3.3-4.1 (7H, m); 4.2 (2H, q, J = 7.5); 4.5 (1H, dd, J = 3 and J = 8); 4.90(1H, s); 5.05(1H, t, J = 4); 7.42 (1H, d, J = 12); 8.40 (1H, s).

MS (ESI+): 419.3.

### 1.A.vii. (13S,16S)-1-cyclopropyl-7-fluoro-16-hydroxy-16-methanesulfonyloxymethyl-4-oxo-1,4,13,15,16,17-hexahydro-12H-11-oxa-1,14-diaza-cyclopenta[a]phenanthrene-3-carboxylic acid ethyl ester:

A solution of intermediate 1.A.vi (570 mg) in pyridine (5 ml) was treated with mesyl chloride (0.118 ml). The reaction was monitored by HPLC. The pyridine was evaporated under reduced pressure and the residue was dissolved in DCM. The org.

layer was washed with water, 0.1*N* HCl and brine, dried over MgSO₄, filtered and the filtrate evaporated. The residue was purified by chromatography over SiO₂, using a DCM/MeOH 95/5 mixture as eluent. The relevant fractions were pooled and evaporated under reduced pressure. The residue was crystallized from an EA/Hex mixture to afford 517 mg (76%) of white solid.

¹H NMR (DMSO_{d6}; δ ppm): 0.9-1.01 (4H, m); 1.26 (3H, t, J =7.5); 1.80 (1H, dd, J = 3 and J = 12); 2.35 (1H, dd, J = 12 and J = 8); 3.24 (3H, s); 3.47 (1H, dd, J = 11 and J = 8); 3.65 (1H, m); 3.75 (1H, dd, J =8); 3.95 (1H, dd, J =8 and J = 2); 4.08 (1H, m); 4.19 (2H, q, J = 7.5); 4.32 (2H, s); 4.51 (1H, dd, J = 2 and J = 7); 5.52 (1H, s); 7.44 (1H, d, J = 12); 8.40 (1H, s).

MS (ESI+): 497.2.

### 1.A.viii. (13S,16S)-1-cyclopropyl-7-fluoro-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid ethyl ester:

A solution of intermediate 1.A.vii (99.3 mg) and intermediate 1.A.vii (56 mg) in DMF (2 ml) was treated with K₂CO₃ (55 mg). The reaction was stirred at 80°C for 5 h and monitored by HPLC/MS. The solvent was evaporated under reduced pressure and the residue was dissolved in DCM/MeOH 9/1. The org. layer was washed with water and brine, dried over MgSO₄ and filtered. The filtrate was evaporated under reduced pressure and the residue purified by chromatography on SiO₂, using a 9/1 DCM/MeOH mixture as eluent The relevant fractions were evaporated and the residue crystallized from an EA/Hex mixture to afford 73 mg (54%) of a beige solid.

¹H NMR (DMSO_{d6}; δ ppm): 0.9-1.10 (4H, m); 1.25 (3H, t, J =7.5); 1.85 (1H, dd, J=3 and J = 12); 2.45 (1H, dd, J = 12 and J = 8); 3.55 (1H, dd, J = 4 and J = 8; 3.60-3.95 (3H, m); 4.0-4.25 (7H, m); 4.55 (1H, dd, J = 2 and J = 8); 4.85 (2H, d, J = 3); 5.13 (1H, m); 5.38 (1H, s); 7.15 (1H, dd, J = 3 and J = 8); 7.25 (1H, dd, J = 8); 7.45 (1H, d, J = 12); 7.50 (1H, dd, J = 3 and J = 12); 7.88 (1H, d, J =0.5); 8.18 (1H, d, J = 0.5); 8.42 (1H, s).

MS (ESI+): 679.5.

### 1.A.ix. (13S,16S)-1-cyclopropyl-7-fluoro-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

A solution of intermediate 1.A.viii (153 mg) in dioxane (1 ml) was treated with 0.2*N* aq. LiOH. The reaction was stirred at 90°C during 48 h while the pH was kept at pH 9.1 to achieve a complete conversion. The mixture was diluted with water and acidified with 1*N* HCl to pH 4. The solid was collected by filtration and purified twice by chromatography over SiO₂ using a DCM/MeOH /AcOH 95/5/0.5 mixture as eluent. The relevant fractions were evaporated under reduced pressure and crystallized from MeOH, affording 18.5 mg (13%) of off-white solid.

¹H NMR (DMSO_{d6}; δ ppm): 1.0-1.20 (4H, m); 1.90 (1H, dd, J =3 and J =11); 2.45 (1H, dd, J =12 and J = 8); 3.60-3.95 (4H, m); 4.07 (1H, dd, J = 4 and J = 8); 4.10 (1h, s); 4.25 (2H, m); 4.62 (1H, br. dd, J = 2 and J = 8); 4.83 (2H, d, J = 6); 5.12 (1H, m); 5.40 (1H, s); 7.14 (1H, dd, J = 3 and J = 8); 7.26 (1H, t , J =8); 7.48 (1H, dd, J = 3 and J= 12); 7.58 (1H, d, J= 12); 7.76 (1H, d, J=0.5); 8.16 (1H, d, J=0.5); 8.63 (1H, s); 15.22 (1H,s).

MS (ESI+): 651 (M+H)⁺; 649.2 (M-H)⁻.

### APPROACH B:

### 1.B.i. 1-cyclopropyl-6,-7-difluoro-8-hydroxy-4-oxo-1,4-dihydro-quinoline-3-carboxylic acid:

A solution of 1-cyclopropyl-6,7-difluoro-8-methoxy-4-oxo-1,4-dihydro-quinoline-3-carboxylic acid (13.02 g) in HBr in AcOH (33%; 100 ml) was stirred at 100°C for 1 day. The reaction mixture was cooled to 0°C and diluted with water (400 ml). The resulting crystals were collected by filtration affording after drying 12.4 g of colourless material.

¹H NMR (DMSO_{d6}; δ ppm): 1.09-1.23 (4H, m); 4.32 (1H,m); 7.70 (dd, 1H, J = 8 and J= 10); 8.75 (1H, s); 11.66 (1H, broad); 14.78 (1H, s).

### 1.B.ii. 8-benzyloxy-1-cyclopropyl-6,7-difluoro-4-oxo-1,4-dihydro-quinoline-3-carboxylic acid:

A solution of intermediate 1.B.i (1 g) in DMF (15 ml) was treated with 1*N* NaOH (3.56 ml). After stirring for 15 min, the yellow solution was treated with BnBr (486 µl). The reaction mixture was stirred at rt for 1 h, diluted with water (50 ml) and the resulting colourless crystals were filtered affording after drying 1.2 g of solid.

MS(ESI⁺): 372.1 (M+H)⁺.

### 1.B.iii. 8-benzyloxy-1-cyclopropyl-6,7-difluoro-4-oxo-1,4-dihydro-quinoline-3-carboxylic acid ethyl ester:

A solution of intermediate 1.B.ii (59.23g) in DMF (300 ml) was treated with K₂CO₃ (24.24 g) and ethyl bromide (14.28 ml) and heated at 50°C for 1 h. The solvents were removed under reduced pressure and the residue was dissolved in DCM and washed with brine. The org. layer was dried over MgSO₄, filtered and evaporated under reduced pressure. The residue was suspended in EA/ether and stirred at 0°C before filtration and drying under reduced pressure, affording 51.72°g of colourless crystals.

MS(ESI⁺): 399.8.

### 1.B.iv. 1-cyclopropyl-6,7-difluoro-1,4-dihydro-8-hydroxy-4-oxo-3-quinolinecarboxylic acid ethyl ester:

A solution of intermediate 1.B.iii (52.38 g) in THF (900 ml) and EtOH (100 ml) was hydrogenated over Pd(OH)₂ (3 g) for 2 h. The suspension was diluted with DCM (1 1) and EtOH (100 ml), heated to 35°C and filtered. The ML was concentrated *in vacuo* and the crystals were collected by filtration. The solid was suspended in hot EA (300 ml) and stirred for 1 h. The suspension was filtered to afford colourless crystals (37 g).

MS(ESI⁺): 310.1.

### 1.B.v. 8-((2S)-1-tert-butoxycarbonyl-4-methylene-pyrrolidin-2-ylmethoxy)-1-cyclopropyl-6,7-difluoro-4-oxo-1,4-dihydro-quinoline-3-carboxylic acid ethyl ester:

A solution of (*2S*)-2-(hydroxymethyl)-4-methylene-1-pyrrolidinecarboxylic acid *tert*-butyl ester (2.58 g; J. Org. Chem. (2003), 68, 3923-3931), intermediate 1.B.iv (3.56 g) and PPh₃ (4.44 g) in THF (100 ml) was treated dropwise over 1.5 h with a solution of DIAD (2.85 ml) in THF (7 ml). The reaction was further stirred at rt for 16 h. The solvent was removed under reduced pressure and the residue was stirred in a mixture of ether/Hex (150 mL 1/1). The solid was filtered off and the filtrate was concentrated *in vacuo.* The residue was again stirred in the same solvent mixture and the second crop of crystals was filtered off. The filtrate was concentrated *in vacuo* and the residue was purified by chromatography over SiO₂ (EA/Hex 1:9). The relevant fractions were pooled, evaporated under reduced pressure and crystallized from EA/Hex (1:1) affording 4.48 g of title compound as colourless solid.

MS (ESI+): 505.5.

### 1.B.vi. 8-((2S)-1-tert-butoxycarbonyl-4-methylene-pyrrolidin-2-ylmethoxy)-1-cyclopropyl-6,7-difluoro-4-oxo-1,4-dihydro-quinoline-3-carboxylic acid:

A solution of intermediate 1.B.v (1.01 g) in dioxane (10 ml) was treated with lithium hydroxide monohydrate (0.17 mg) and water (1.5 ml). The reaction mixture was stirred at rt for 1 day. The organic solvent was removed under reduced pressure and the aq. residue was diluted with water (2 ml) and acidified to pH 2 with 1*N* HCl. The resulting solid was collected by filtration and dried under HV to afford 0.84 g of colourless solid.

¹H NMR (DMSO_{d6}; δ ppm): 1.08-1.25 (4H, m); 1.4 (9H, s); 2.7 (1H, m); 2.9 (1H, m); 3.83 (1H, m); 4.0 (1H, m); 4.1-4.24 (3H, m); 4.3 (1H, m); 5.07 (2H, m); 8.05 (1H, m); 8.78 (1H, s); 14.5 (1H, s).

MS (ESI+): 477.2.

### 1.B.vii. 8-((2S)-1-tert-butoxycarbonyl-4-methylene-pyrrolidin-2-ylmethoxy)-1-cyclopropyl-6,7-difluoro-4-oxo-1,4-dihydro-quinoline-3-carboxylic acid benzyl ester:

A solution of intermediate 1.B.vi (899 mg) in DMF (5 ml) was treated with K₂CO₃ (365 mg) and BnBr (0.23 ml). The reaction mixture was stirred at 60°C for 3 h. The solvent was removed under reduced pressure and the residue was dissolved in DCM and washed with brine. The org. layer was dried over MgSO₄, filtered and evaporated. The residue was crystallized from ether/Hex to give a pale yellow solid (867 mg).

¹H NMR (DMSO_{d6}; δ ppm): 1.0-1.15 (4H, m); 1.4 (9H, s); 2.65 (1H, m); 2.87 (1H, m); 3.80 (1H, m); 3.93-4.26 (4H, m); 4.3 (1H, m); 5.06 (2H, m); 5.28 (2H, s); 7.30-7.42 (3H, m); 7.45-7.51 (2H, m) 7.87 (1H, m); 8.57 (1H, s).

MS (ESI+): 567.5.

### 1.B.viii. 8-[(2S,4S))-(1-tert-butoxycarbonyl-4-hydroxy-4-hydroxymethyl-pyrrolidin-2-ylmethoxy)]-1-cyclopropyl-6,7-difluoro-4-oxo-1,4-dihydro-quinoline-3-carboxylic acid benzyl ester:

The compound was obtained in 98% yield as a colourless foam, using the procedure of Example 1, step 1.A.vi and starting from intermediate 1.B.vii (867 mg), potassium ferricyanide III (1.51 g), potassium osmate dihydrate (0.006 g), K₂CO₃ (0.64 g) and (DHQ)₂PHAL (0.024 g).

MS (ESI+): 601.1.

### 1.B.ix. 1-cyclopropyl-6,7-difluoro-8-[(2S,4S)-4-hydroxy-4-hydroxyntethyl-pyrrolidin-2-ylmethoxy]-4-oxo-1,4-dihydro-quinoline-3-carboxylic acid benzyl ester hydrochloride:

Intermediate 1.B.viii (900 mg) was dissolved in a 3.7*M* HCl solution in dioxane (10 ml). The solution was treated with a few drops of water and the mixture was stirred at rt for 30 min. The solvent was removed under reduced pressure and the residue was stirred in EA. The crystals were collected by filtration and dried under HV, affording a colourless solid (802 mg).

MS (ESI+): 501.2.

### 1.B.x. (13S,16S)-1-cyclopropyl-7-fluoro-16-hydroxy-16-hydroxymethyl-4-oxo-1,4,13,15,16,17-hexahydro-12H-11-oxa-1,14-diaza-cyclopenta[a]phenanthrene-3-carboxylic acid benzyl ester:

A solution of intermediate 1.B.ix (802 mg) in NMP (4 ml) was treated with NaHCO₃ (313 mg) and DIPEA (0.256 ml). The mixture was stirred at 80°C for 1 h. The solvent was removed under reduced pressure and the residue, dissolved in a DCM/MeOH (9:1) mixture, was washed with water and brine. The org. layer was dried over MgSO₄, filtered and concentrated *in vacuo.* The residue was crystallized from MeCN to afford a solid (215 mg).

¹H NMR (DMSO_{d6}; δ ppm): 0.9-1.10 (4H, m); 1.65 (1H, dd, J = 13 and J = 10); 2.34 (1H, dd, J = 13 and J = 8); 3.35 (1H, m); 3.44 (2H, d, J = 4); 3.58 (1H, m); 3.75 (1H, m); 3.93 (1H, dd, J = 10 and J = 7); 4.07 (1H, m); 4.48 (1H, dd, J = 10 and J = 7); 4.86 (1H, s); 4.96 (1H, t, J = 4); 5.15 (2H, s); 7.26-7.50 (6H, m); 8.45 (1H, s).

MS (ESI+): 481.3.

### 1.B.xi. (13S,16S)-1-cyclopropyl-7-fluoro-16-hydroxy-16-methanesulfonyloxymethyl-4-oxo-1,4,13,15,16,17-hexahydro-12H-11-oxa-1,14-diaza-cyclopenta[a]phenanthrene-3-carboxylic acid benzyl ester:

This compound was obtained as a colourless foam in 82% yield, using the procedure of Example 1, step 1.A.vii and starting from intermediate 1.B.x (215 mg), mesyl chloride (56 mg) and pyridine (0.7 ml).

¹H NMR (DMSO_{d6}; δ ppm): 0.9-1.10 (4H, m); 1.82 (1H, dd, J = 14 and J = 3); 2.33 (1H, dd, J = 14 and J = 8); 3.24 (3H, s); 3.47 (1H, dd, J = 10 and J = 3); 3.65 (1H, m); 3.72 (1H, m); 3.93 (1H, dd, J = 14 and J = 3); 4.07 (1H, m); 4.30 (2H, s); 4.51 (1H, dd, J= 10 and J = 3); 5.16 (2H, s); 7.28-7.50 (6H, m); 8.47 (1H, s).

MS (ESI+): 559.1.

### 1.B.xii. (13S,16S)-1-cyclopropyl-7-fluoro-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid benzyl ester:

The compound was obtained as a beige solid in 26% yield, using the procedure of Example 1, step 1.B.viii and starting from intermediate 1.B.xi (203 mg) and intermediate 1.A.ii (111 mg).

¹H NMR (DMSO_{d6}; δ ppm): 0.9-1.10 (4H, m); 1.84 (1H, dd, J = 14 and J = 3); 2.43 (1H, dd, J = 14 and J = 8); 3.53 (1H, dd, J = 10 and J = 3); 3.7 (1H, m); 3.78 (1H, m); 3.87 (1H, dd, J = 10 and J = 8); 3.98-4.14 (3H, m); 4.21 (1H, m); 4.57 (1H, dd, J = 10 and J = 3); 4.83 (2H,d, J = 4); 5.13 (1H, m); 5.28 (2H, s); 5.37(1H, s); 7.12-7.52 (9H, m); 8.47 (1H, d, J= 1); 8.18 (1H, d, J= 1); 8.46 (1H, s).

MS (ESI+): 741.2.

### 1.B.xiii. (13S,16S)-1-cyclopropyl-7-fluoro-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

A solution of intermediate 1.B.xii (70 mg) was dissolved in MeOH (3 ml) and hydrogenated overnight at rt over 10% Pd/C (10 mg). The suspension was diluted with DCM (5 ml) and the catalyst was filtered off. The filtrate was concentrated *in vacuo* and the residue stirred in MeOH (5 ml). The crystals were collected by filtration, washed with MeOH and Hex and dried under HV, affording 57 mg (93%) of title compound.

¹H NMR and MS identical with those obtained for Example 1, step 1.A.ix.

### Example 2: (13S,16S)-16-{4-[(5S)-5-(acetylamino-methyl)-2-oxo-oxazolidin-3-yl]-2-fluoro-phenoxymethyl}-1-cyclopropyl-7-fluoro-16-hydroxy-4-oxo-1,4,13,15,16,17-hexahydro-12H-11-oxa-1,14-diaza-cyclopenta[a]phenanthrene-3-carboxylic acid:

### 2.i. (135,16S)-16-{4-[(5S)-5-(acetylamino-methyl)-2-oxo-oxazolidin-3-yl]-2-fluoro-phenoxymethyl}-1-cyclopropyl-7-fluoro-16-hydroxy-4-oxo-1,4,13,15,16,17-hexahydro-12H-11-oxa-1,14-diaza-cyclopenta[a]phenanthrene-3-carboxylic acid ethyl ester:

Using the procedure of Example 1, step 1.A.viii and starting from intermediate 1.A.vii (99 mg) and *N*-[(5*S*)-3-(3-fluoro-4-hydroxy-phenyl)-2-oxo-oxazolidin-5-ylmethyl]-acetamide (54 mg; described in WO 2004/096221), the title compound was obtained in 49% yield after chromatography on SiO₂ (eluent DCM/MeOH 9/1) and crystallization from EA/Hex.

¹H NMR (DMSO_{d6}; δ ppm): 0.9-1.10 (4H, m); 1.25 (3H, t, J =7.5); 1.83 (3H, s); 1.85 (1H, m); 2.42 (1H, m); 3.45 (2H, m); 3.53 (1H, dd, J = 3 and J = 8); 3.60-3.8 (3H, m); 4.0-4.25 (7H, m); 4.55 (1H, dd, J = 3 and J = 8); 4.70 (1H, m); 5.13 (1H, m); 5.38 (1H, s); 7.15-7.30 (2H, m); 7.46 (1H, d, J = 12); 7.59 (1H, dd, J = 3 and J = 12); 8.5 (1H, t, J = 4); 8.42 (1H, s).

MS (ESI+): 669.5.

### 2.ii. (13S,16S)-16-{4-[(SS)-5-(acetylamino-methyl)-2-oxo-oxazolidin-3-yl]-2-fluoro-phenoxymethyl}-1-cyclopropyl-7-fluoro-16-hydroxy-4-oxo-1,4,13,15,16,17-hexahydro-12H-11-oxa-1,14-diaza-cyclopenta[a]phenanthrene-3-carboxylic acid:

Starting from intermediate 2.i (67 mg) and using the procedure of Example 1, step 1.A.ix, the title compound was obtained in 18% yield after chromatography on SiO₂ (eluent DCM/MeOH /AcOH 95/5/0.5).

¹H NMR (DMSO_{d6}; δ ppm): 1-1.20 (4H, m); 1.84 (3H, s); 1.9 (1H, m); 2.48 (1H, m); 3.43 (2H, m); 3.6-3.85 (3H, m); 4.04-4.18 (4H, m); 4.26 (1H, m); 4.63 (1H, m); 4.72 (1H, m); 5.42 (1H, s); 7.16-7.33 (2H, m); 7.57 (1H, dd, J = 3 and J = 12); 7.6 (1H, d, J = 12); 8.26 (1H, t, J = 4); 8.63 (1H, s); 15.24 (1H, s).

MS (ESI+): 641.3.

Example 3: (*13R,16S*)-16-{4-[(*5S*)-5-(acetylamino-methyl)-2-oxo-oxazolidin-3-yl]-2-fluoro-phenoxymethyl}-1-cyclopropyl-7-fluoro-16-hydroxy-4-oxo-1,4,13,15,16,17-hexahydro-12*H*-11-oxa-1,14-diaza-cyclopenta[*a*]phenanthrene-3-carboxylic acid:

### 3.i. (2R)-(4-methylene-pyrrolidin-2-yl)-methanol hydrochloride:

This compound was obtained in 95% yield as a colourless solid, starting from (2*R*)-2-(*tert*-butyl-dimethyl-silanyloxymethyl)-4-methylene-pyrrolidine-1-carboxylic acid *tert*-butyl ester (50.3 g; prepared starting from (2*R*,*4R*)-4-hydroxy-pyrrolidine-2-carboxylic acid in analogy to J. Org. Chem. (2003), 68, 3923-31) and using the procedure of Example 1, step 1.A.iii.

¹H NMR and MS identical with those obtained for intermediate 1.A.iii.

### 3.ii. 1-cyclopropyl-6,8-difluoro-7-((2R)-2-hydroxymethyl-4-methylenepyrrolidin-1-yl)-4-oxo-1,4-dihydro-quinoline-3-carboxylic acid ethyl ester:

The compound was obtained in 33% yield, starting from intermediate 3.i (2.09 g) and 1-cyclopropyl-6,7,8-trifluoro-4-oxo-1,4-dihydro-quinoline-3-carboxylic acid ethyl ester (4.35 g) and using the procedure of Example 1, step 1.A.iv.

¹H NMR (DMSO_{d6}; δ ppm): 1-2 (4H, m); 1.27 (3H, t, J = 7.5); 2.53 (1H, m); 2.75 (1H, dd, J = 8 and J = 12); 3.40 (2H, m); 3.90 (1H, bd, J = 12); 3.95 (1H, m); 4.22 (2H, q, J = 7.5); 4.27 (1H, m); 4.45 (1H, bd, J = 12 Hz); 4.75 (1H, t, J = 4), 5.02 (2H, s); 7.62 (1H, dd, J = 2 and J = 11); 8.42 (1H, s).

### 3.iii. (13R)-1-cyclopropyl-7-fluoro-16-methylene-4-oxo-1,4,13,15,16,17-hexahydro-12H-11-oxa-1,14-diaza-cyclopenta[a]phenanthrene-3-carboxylic acid ethyl ester:

This compound was obtained in 40% yield after crystallization from EA, starting from intermediate 3.ii and using the procedure of Example 1, step 1.A.v.

¹H NMR (DMSO_{d6}; δ ppm): 0.9-1.1 (4H, m); 1.27 (3H, t, J = 7.5); 2.4 (1H, m); 2.85 (1H, dd, J = 8 and J = 14); 3.51 (1H, m); 3.64 (1H, m); 4.02-4.08 (2H, m); 4.22 (2H, q, J = 7.5); 4.3 (1H, dd, J =15 and J = 3); 4.58 (1H, dd, J =10 and J = 3); 5.10 (2H, d, J = 2), 7.44 (1H, d, J = 12); 8.42 (1H, s).

MS: 385.3 (M+H)⁺.

### 3.iv. (13R,16S)-1-cyclopropyl-7-fluoro-16-hydroxy-16-hydroxymethyl-4-oxo-1,4,13,15,16,17-hexahydro-12H-11-oxa-1,14-diaza-cyclopenta[a]phenanthrene-3-carboxylic acid ethyl ester:

This compound was obtained in 98% yield, starting from intermediate 3.iii (308 mg) and AD-mix α and using the procedure of Example 1, step 1.A.vi.

¹H NMR (DMSO_{d6}; δ ppm): 0.9-1.1 (4H, m); 1.27 (3H, t, J = 7.5); 1.68 (1H, dd, J = 15 and J = 12); 1.85(1H, dd, J = 15 and J = 8); 3.34-3.49 (3H, m); 3.60 (1H, dd, J = 14 and J = 8 Hz); 3.75 (1H, m); 3.88 (1H, m); 4.06 (1H, m); 4.22 (2H, q, J = 7.5); 4.65 (1H, dd, J = 14 and J = 3); 4.95 (1H, t, J = 6); 5.02 (1H, s), 7.44 (1H, d, J = 12); 8.42 (1H, s).

MS: 419.2.

### 3.v. (13R,16S)-1-cyclopropyl-7-fluoro-16-hydroxy-16-methanesulfonyloxymethyl-4-oxo-1,4,13,15,16,17-hexahydro-12H-11-oxa-1,14-diaza-cyclopenta[a]phenanthrene-3-carboxylic acid ethyl ester:

The compound was obtained as a colourless solid in 60% yield, starting from intermediate 3.iv (334 mg) and using the procedure of Example 1, step 1.A.vii.

MS: 496.8.

### 3.vi. (13R,16S)-16-{4-[(5S)-5-(acetylamino-methyl)-2-oxo-oxazolidin-3-yl]-2-fluoro-phenoxymethyl}-1-cyclopropyl-7-fluoro-16-hydroxy-4-oxo-1,4,13,15,16,17-hexahydro-12H-11-oxa-1,14-diaza-cyclopenta[a]phenanthrene-3-carboxylic acid ethyl ester:

The compound was obtained in 41% yield as a beige solid, starting from intermediate 3.v (89 mg) and *N*-[(*5S*)-3-(3-fluoro-4-hydroxy-phenyl)-2-oxo-oxazolidin-5-ylmethyl]-acetamide (48 mg) and using the procedure of Example 1, step 1.A.viii.

MS: 668.9.

### 3.vii. (13R,16S)-16-{4-[(5S)-5-(acetylamino-methyl)-2-oxo-oxazolidin-3-yl]-2-fluoro-phenoxymethyl}-1-cyclopropyl-7-fluoro-16-hydroxy-4-oxo-1,4,13,15,16,17-hexahydro-12H-11-oxa-1,14-diaza-cyclopenta[a]phenanthrene-3-carboxylic acid:

Starting from intermediate 3.vi (50 mg) and using the procedure of Example 1, step 1.A.ix, the title compound was obtained as a yellow foam in 39% yield after chromatography on SiO₂ (eluent DCM/MeOH /AcOH 95/5/0.5).

¹H NMR (DMSO_{d6}; δ ppm): 1.05-1.18 (4H, m); 1.80 (1H, m); 1.85 (3H, s); 2.14 (1H, dd, J = 15 and J = 10); 3.40 (2H, m); 3.50 (1H, dd, J = 12 and J = 10); 3.70 (1H, dd, J = 8 and J = 10); 3.80 (1H, dd, J = 12 and J = 4); 3.83-4.29 (5H, m); 4.66-4.80 (2H, m); 5.53 (1H, s); 7.15-7.30 (2H, m); 7.59 (1H, dd, J = 3 and J = 14); 7.61 (1H, d, J = 14); 8.25 (1H, t, J = 4); 8.60 (1H, s); 15.25 (1H, s).

MS (ESI+): 641.1.

### Example 4: (13R,16S)-1-cyclopropyl-7-fluoro-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

### 4.i. (13R,16S)-1-cyclopropyl-7-fluoro-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid ethyl ester.

This compound was obtained as a colourless solid in 96% yield, starting from intermediate 1.A.ii (55 mg) and intermediate 3.v (90 mg) and using the procedure of Example 1 step 1.A.viii.

MS: 679.3.

### 4.ii. (13R,16S)-1-cyclopropyl-7-fluoro-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

Starting from intermediate 4.i (174 mg) and using the procedure of Example 1, step 1.A.ix, the title compound was obtained in 20% yield.

¹H NMR (DMSO_{d6}; δ ppm): 1.05-1.18 (4H, m); 1.80 (1H, m); 2.14 (1H, dd, J = 15 and J = 10); 3.50 (1H, dd, J = 12 and J = 10); 3.73-4.3 (7H, m); 4.70-4.88 (3H, m); 5.12 (1H, m); 5.53 (1H, s); 7.10-7.30 (2H, m); 7.50 (1H, dd, J = 3 and J = 14); 7.61 (1H, d, J= 14); 7.77 (1H, d, J= 1); 8.17 (1H, d, J= 1); 8.63 (1H, s); 15.22 (1H, broad).

MS: 651.0 (M+H)⁺; 649.2 (M-H)⁻.

### Example 5: (13S,16R)-1-cyclopropyl-7-fluoro-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diazacyclopenta[a]phenanthrene-3-carboxylic acid:

### 5.i. 8-[(2S,4R))-(1-tert-butoxycarbonyl-4-hydroxy-4-hydroxymethyl-pyrrolidin-2-ylmethoxy)]-1-cyclopropyl-6,7-difluoro-4-oxo-1,4-dihydro-quinoline-3-carboxylic acid benzyl ester:

The step 1.B.viii of Example 1 was repeated on a larger scale starting from 56.84 g K₂CO₃, 135.4 g potassium ferricyanide, 252 mg potassium osmate dihydrate, 1.067 mg (DHQ)₂PHAL, 1_1 *tert*-butanol, 11 water and 77.6 g intermediate 1.B.vii. The oxidation was complete after 6 day stirring at -1.5 °C. The reaction was treated carefully with 128 g sodium bisulfite, stirred at rt for 20 min and diluted with 1.21 EA. The org. layer was washed with water and brine. The water layers were backwashed with 0.5 1 EA.

The combined org. layers dried over MgSO₄, filtered and the filtrate was evaporated. The solid was dissolved in a 0.51 DCM/MeOH (9/1) and filtered over 150 g SiO₂. The SiO₂ pad was washed with 11 of DCM/MeOH (9/1), the filtrate was collected and concentrated to 250 ml. The slurry was diluted with 11 EA and concentrated to a volume of 11 and stirred for 16 h at rt. The crystals were collected and washed with cold EA, affording 65.35 g of intermediate 1.B.viii as a white solid. The mother liquor was evaporated and purified by chromatography over SiO₂, using a DCM/MeOH (95/5) as eluent. The relevant fractions were evaporated and the residue dried to afford 7.41 g of a colourless foam.

¹H NMR (DMSO_{d6}; δ ppm): 0.95-1.15 (4H, m); 1.25 (9H, s); 1.8-1.9 (1H, m); 2.2-2.3 (1H, t broad, J = 11); 3.1-3.4 (4H, m); 4.0 (1H, m); 4.2 (1H, m); 4.35 (1H, m); 4.45 (1H, m); 4.8 (1H, s broad); 4.9 (1H, t, J=7); 5.15 (2H, s); 7.25-7.50 (5H, m); 7.9 (1H, m); 8.6 (1H, s).

MS (ESI): 600.9.

### 5.ii. 1-cyclopropyl-6,7-difluoro-8-[(2S,4R)-4-hydroxy-4-hydroxymethyl-pyrrolidin-2-ylmethoxy]-4-oxo-1,4-dihydro-quinoline-3-carboxylic acid benzyl ester hydrochloride:

This compound was prepared as a colourless solid in 98.9% yield (6.25 g), starting from intermediate 5.i (6.25 g) and 3.7*M* HCl and using the procedure of Example 1, step 1.B.ix.

MS (ESI): 501.1.

### 5.iii. (13S,16R)-1-cyclopropyl-7-fluoro-16-hydroxy-16-hydroxymethyl-4-oxo-1,4,13,15,16,17-hexahydro-12H-11-oxa-1,14-diaza-cyclopenta[a]phenanthrene-3-carboxylic acid benzyl ester:

This compound was obtained as a colourless solid in 39% yield (2.15 g), starting from intermediate 5.ii (5.75 g), NaHCO₃ and DIPEA and using the procedure of Example 1, step 1.B.x.

MS (ESI): 480.7.

### 5.iv. (13S,16R)-1-cyclopropyl-7fluoro-16-hydroxy-16-methanesulfonyloxymethyl-4-oxo-1,4,13,15,16,17-hexahydro-12H-11-oxa-1,14-diaza-cyclopenta[a]phenanthrene-3-carboxylic acid benzyl ester:

A solution of intermediate 5.iii (1.2 g) in THF (2 ml) was cooled to 0°C and treated with TEA (0.7 ml). The resulting solution was treated with methanesulfonic anhydride (653 mg) in THF (1 ml). After 30 min the reaction mixture was further treated portionwise (0.1 ml portions) with a solution of methanesulfonic anhydride (70 mg) in THE (0.7 ml) until the reaction was over. The reaction mixture was diluted with water (5 ml) and EA (10 ml). The org. layer was treated 3 times with aq. NaHCO₃ solution.

The org. layer was sequentially washed with water and brine, dried over MgSO₄, filtered and evaporated under reduced pressure. The resulting solid was stirred in EA (5 ml), filtered and dried under reduced pressure to afford 1.18 g of a colourless solid (85% yield).

¹H NMR (DMSO_{d6}; δ ppm): 0.95-1.1 (4H, m); 1.67 (1H, dd, J = 10 and J = 12.5); 2.07 (1H, dd, J = 6 and J = 12.5); 3.23 (3H, s); 3.46 (1H, t, J = 10); 3.6 (1H, dd, J = 3 and J = 8); 3.83-3.94 (2H, m); 4.03-4.10 (1H, m); 4.3 (2H, s); 4.66 (1H, dd, J = 3.5 and 10.5); 5.26 (2H, s); 5.61 (1H, s); 7.3-7.5 (6H, m); 8.45 (1H, s).

MS (ESI): 558.6.

### 5.v. (13S,16R)-1-cyclopropyl-7-fluoro-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diazacyclopenta[a]phenanthrene-3-carboxylic acid benzyl ester:

This compound was obtained as a colourless solid in 69% yield, starting from intermediates 5.iv (0.500 g) and 1.A.ii (0.249 g) and using the procedure of Example 1, step 1.A.viii.

MS (ESI): 650.7.

### 5.vi. (13S,16R)-1-cyclopropyl-7-fluoro-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diazacyclopenta[a]phenanthrene-3-carboxylic acid:

A solution of intermediate 5.v (440 mg) was dissolved in MeOH:THF (1:1; 10 ml) and hydrogenated for 3 h at rt over 10 mg 10% Pd/C. The solvents were evaporated under reduced pressure and the residue was taken up in a mixture of CH₂Cl₂ (50 ml) and MeOH (5 ml). The suspension was filtered and the filtrate evaporated under reduced pressure. The oily residue was taken up in a CH₂Cl₂ (about 1 ml) and diluted with MeOH (50 ml). The solid was collected by filtration and washed with MeOH (5 ml), affording 29 mg (75.7%) of a pale yellow solid.

¹H NMR (DMSO_{d6}; δ ppm): 0.99-1.14 (4H, m); 1.78 (1H, m); 2.14 (1H, dd, J = 6.5 and 13); 3.76 (1H, t, J = 10); 3.77 (1H, dd, J = 3.5 and J = 11); 3.87 (1H, dd, J = 6 and J = 9); 3.97-4.26 (7H, m); 4.75 (1H, dd, J = 3.5 and J = 10); 4.83 (1H, d, J = 5); 5.08-5.16 (1H, m); 5.58 (1H,s); 7.13-7.30 (1H, dt, J = 1 and J = 9); 7.25 (1H, t, J = 9); 7.50 (1H, dd, J = 2.6 and J = 13.5); 7.58 (1H, d, J = 13.5); 7.77 (1H, d, J = 1); 8.17 (1H, d, J = 1); 8.60 (1H, s); 15.22 (1H, s).

MS (ESI): 651.0 (M+H)⁺; 649.2 (M-H)⁻.

### Example 6: (13R,16R)-1-cyclopropyl-7-fluoro-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diazacyclopenta[a]phenanthrene-3-carboxylic acid:

### 6.i. 8-((2R)-1-tert-butoxycarbonyl-4-methylene-pyrrolidin-2-ylmethoxy)-1-cyclopropyl-6,7-difluoro-4-oxo-1,4-dihydro-quinoline-3-carboxylic acid ethyl ester:

This compound was obtained as a colourless material (88.8% yield), starting from intermediates 1.B.iv (5.8 g) and (2R)-2-(hydroxymethyl)-4-methylene-1-pyrrolidinecarboxylic acid *tert*-butyl ester (4.0 g; prepared in analogy to J. Org. Chem. (2003), 68, 3923-31, starting from 4-hydroxy-D-proline instead of its L-enantiomer) and using the procedure of Example 1, step 1.B.v.

¹H NMR (DMSO_{d6}; δ ppm): 1.05-1.09 (4H, m); 1.25 (3H, t, J = 7); 1.27 (9H, s); 2.65 (1H, m); 2.87-2.89 (1H, m); 3.81 (1H, m); 4.0-4.04 (3H, m); 4.19 (1H, m); 4.21 (2H, q, J = 7), 4.26 (1H, m); 5.07 (2H, m); 7.89 (1H, m); 8.52 (1H, s).

MS (ESI): 505.24.

### 6.ii. 8-((2R)-1-tert-butoxycarbonyl-4-methylene-pyrrolidin-2-ylmethoxy)-1-cyclopropyl-6,7-difluoro-4-oxo-1,4-dihydro-quinoline-3-carboxylic acid:

This compound was obtained as a colourless material (92.8% yield) from intermediate 6.i (8.17 g) and LiOH (20.3 g), using the procedure of Example 1, step 1.B.vi.

MS (ESI): 476.8.

### 6.iii. 8-((2R)-1-tert-butoxycarbonyl-4-methylene-pyrrolidin-2-ylmethoxy)-1-cyclopropyl-6,7-difluoro-4-oxo-1,4-dihydro-quinoline-3-carboxylic acid benzyl ester.

This compound was obtained as a colourless material (90.9% yield) from intermediate 6.ii (7.14 g) and BnBr (2.8 g), using the procedure of Example 1, step 1.B.vii.

¹H NMR (DMSO_{d6}; δ ppm): 1.05-1.09 (4H, m); 1.39 (9H, s); 2.65 (1H, m); 2.73-2.86 (1H, m); 3.82 (1H, m); 3.98 (3H, m); 4.03 (1H, m); 4.27 (1H, m); 5.06 (2H, m), 5.29 (2H, s); 7.03-7.58 (5H,m), 7.89 (1H, m); 8.57 (1H, s).

MS (ESI): 567.1.

### 6.iv. 8-[(2R,4R))-(1-tert-butoxycarbonyl-4-hydroxy-4-hydroxymethyl-pyrrolidin-2-ylmethoxy)]-1-cyclopropyl-6,7-difluoro-4-oxo-1,4-dihydro-quinoline-3-carboxylic acid benzyl ester and 8-[(2R,4S))-(1-tert-butoxycarbonyl-4-hydroxy-4-hydroxymethyl-pyrrolidin-2-ylmethoxy)]-1-cyclopropyl-6,7-difluoro-4-oxo-1,4-dihydro-quinoline-3-carboxylic acid benzyl ester:

These compounds were obtained as a colourless material (86.2% yield) from intermediate 6.iii (7.57 g), potassium ferricyanide (III) (13.2 g), potassium osmate dehydrate (24 mg) and (DHQ)₂PHAL (0.102 g), using the procedure of Example 1, step 1.B.viii.

MS (ESI): 601.39.

### 6.v. 1-cyclopropyl-6,7-difluoro-8-[(2R,4R)-4-hydroxy-4-hydroxymethyl-pyrrolidin-2-ylmethoxy]-4-oxo-1,4-dihydro-quinoline-3-carboxylic acid benzyl ester hydrochloride and 1-cyclopropyl-6,7-difluoro-8-[(2R,4S)-4-hydroxy-4-hydroxymethyl-pyrrolidin-2-ylmethoxyl-4-oxo-1,4-dihydro-quinoline-3-carboxylic acid benzyl ester hydrochloride:

These compounds were obtained as a colourless material (78.9% yield) from intermediates 6.iv (6.95 g) in 3.7*M* HCl in dioxane, using the procedure of Example 1, step 1.B.ix.

MS (ESI): 501.11.

### 6.vi. (13R,16R)-1-cyclopropyl-7-fluoro-16-hydroxy-16-hydroxymethyl-4-oxo-1,4,13,15,16,17-hexahydro-12H-11-oxa-1,14-diaza-cyclopenta[a]phenanthrene-3-carboxylic acid benzyl ester and (13R,16S)-1-cyclopropyl-7-fluoro-16-hydroxy-16-hydroxymethyl-4-oxo-1,4,13,15,16,17-hexahydro-12H-11-oxa-1,14-diaza-cyclopenta[a]phenanthrene-3-carboxylic acid benzyl ester:

A solution of intermediates 6.v. (4.35 g) in MeCN (100 ml) was treated with DIPEA (4.16 ml). The mixture was stirred at 60°C for 3 h. The solvent was removed under reduced pressure and the residue, dissolved in DCM, was sequentially washed with aq. 0.1*N* HCl, saturated NaHCO₃, water and brine. The org. layer was dried over MgSO₄, filtered and concentrated in vacuo. The residue was purified by chromatography over SiO₂ (eluent DCM/MeOH, 95:5). The relevant fractions were pooled and crystallized from EtOH, affording 1.6 g (41% yield) of the major isomer as a colourless material.

The mother liquor was evaporated and crystallized from EtOH affording 270 mg (7% yield) of the minor isomer as yellowish material.

### Major isomer (intermediate 6.vi.a): (13R,16R)-1-cyclopropyl-7-fluoro-16-hydroxy-16-hydroxymethyl-4-oxo-1,4,13,15,16,17-hexahydro-12H-11-oxa-1,14-diaza-cyclopenta[a]phenanthrene-3-carboxylic acid benzyl ester:

¹H NMR (DMSO_{d6}; δ ppm): 0.9-1.08 (4H, m); 1.65 (1H, dd, J = 3.5 and J = 13.5); 2.34 (1H, dd, J= 8.5 and J = 13.5); 3.38 (1H, d, J = 3), 3.42 (2H, d, J=5.7); 3.56-3.62 (1H, m); 3.72 (1H,dd, J = 10 and J = 11); 3.91 (1H, dd, J = 3.5 and J = 11); 4.07 (1H, m); 4.48 (1H, dd, J = 3 and J = 10); 4.88(1H,s); 4.99 (1H, t, J = 5.7); 5.26 (2H, s); 7.30-7.50 (6H, m); 8.45 (1H, s).

MS (ESI): 480.9.

### Minor isomer (intermediate 6.vi.b): (13R,16S)-1-cyclopropyl-7-fluoro-16-hydroxy-16-hydroxymethyl-4-oxo-1,4,13,15,16,17-hexahydro-12H-11-oxa-1,14-diaza-cyclopenta[a]phenanthrene-3-carboxylic acid benzyl ester:

¹H NMR (DMSO_{d6}; δ ppm): 0.9-1.08 (4H, m); 1.64-1.71 (1H, dd, J = 10 and J = 12); 2.34 (1H, dd, J = 6.5 and J = 6.9); 3.4-3.48 (2H, m), 3.60 (1H, dd, J = 3.5 and J = 10); 3.76-3.90 (2H, m); 4.03-4.08 (1H, m); 4.64 (1H, dd, J = 3.5 and J = 10); 4.92 (1H, t, J = 5.7); 5.02 (2H, s); 5.25 (2H, s); 7.03-7.50 (6H, m); 8.45 (1H, s).

MS (ESI): 480.9.

### 6.vii. (13R,16R)-1-cyclopropyl-7-fluoro-16-hydroxy-16-methanesulfonyloxymethyl-4-oxo-1,4,13,15,16,17-hexahydro-12H-11-oxa-1,14-diaza-cyclopenta[a]phenanthrene-3-carboxylic acid benzyl ester:

A solution of intermediate 6.vi.a (1.40 g) in THF (14 ml) was cooled to 0°C and treated with TEA (0.81 ml). The resulting solution was treated with methanesulfonic anhydride (759 mg) in THF (1 ml). After 30 min the reaction mixture was further treated portionwise (0.1 ml portions) with a solution of methanesulfonic anhydride (0.7 g) in THF (0.7 ml) until the reaction was over. The reaction mixture was diluted with water (10 ml) and EA (10 ml). The org. layer was treated three times with aq. NaHCO₃ solution. The org. layer was sequentially washed with water and brine, dried over MgSO₄, filtered and evaporated under reduced pressure. The resulting solid was stirred in EA (50 ml), filtered and dried under reduced pressure to afford 1.32 g (81% yield) of a colourless solid.

MS (ESI): 558.4.

### 6.viii. (13R,16R)-1-cyclopropyl-7-fluoro-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid benzyl ester:

This compound was obtained as a beige solid in 57.3% yield, starting from intermediate 6.vii (300 mg) and intermediate 1.A.ii (149 mg) and using the procedure of Example 1, step 1.A.viii.

MS (ESI): 740.9.

### 6.ix. (13R,16R)-1-cyclopropyl-7-fluoro-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

This compound was obtained as a yellow solid in 95% yield, starting from intermediate 6.viii (180 mg) and using the procedure of Example 1, step 1.B.xiii.

¹H NMR (DMSO_{d6}; δ ppm): 1.00-1.14 (4H, m); 1.85-1.91 (1H, m); 2.42-2.50 (1H, m); 3.61-3.67 (1H, dd, J = 5 and J = 11); 3.73-3.89 (3H, m); 4.04-4.09 (1H, dd, J = 3.5 and J = 11); 4.13 (2H, s); 4.22-4.26 (2H, m); 4.60 (1H, d, J = 7); 4.83-4.84(2H, d, J = 5); 5.10-5.15 (1H, m); 5.41 (1H,s); 7.13-7.17 (1H, dt, J =1.5 and J = 9); 7.26 (1H, t, J = 9); 7.50 (1H, dd, J = 2.6 and J = 13.5); 7.60 (1H, d, J = 13); 7.77 (1H, d, J = 1); 8.17 (1H, d, J = 1); 8.60 (1H, s); 15.22 (1H, s).

MS (ESI): 650.8.

### Example 7: (13R,16R)-1-cyclopropyl-7-fluoro-16-[2-fluoro-4-((5S)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

### 7.i. (3S)-5-azidomethyl-3-(4-benzyloxy-3-fluoro-phenyl)-oxazolidin-2-one:

This compound was prepared in analogy to WO 2004/096221 starting from 1-benzyloxy-2-fluoro-4-nitrobenzene (WO 03/064413) using (S)-glycidyl butyrate and subsequent mesylation and reaction with sodium azide. The title compound and the intermediates displayed the same physicochemical properties (MS, NMR) as their corresponding (R)-enantiomers.

MS (ESI): 343.1.

### 7.ii. (5S)-3-(4-benzyloxy-3-fluoro-phenyl)-5-[1,2,3]triazol-1-ylmethyl-oxazolidin-2-one:

A solution of intermediate 7.i (5.0 g) in toluene (125 ml) was treated with TEA (4.87 ml). Nitrogen was bubbled through the solution for 15 min. Copper iodide (34 mg) was added to the solution and the mixture was heated to 50°C under a gentle stream of acetylene for 17 h. The reaction mixture was allowed to cool and the resulting crystals were collected by filtration and washed with ether (50 ml). The solid was suspended in water and stirred for 1 h. The resulting crystals were collected by filtration and dried *in vacuo,* affording 4.78 g (74.2% yield) of a pink solid.

MS (ESI): 343.1.

### 7.iii. (5S)-3-(3-fluoro-4-hydroxy-phenyl)-5-[1,2,3]triazol-1-ylmethyl-oxazolidin-2-one:

A suspension of intermediate 7.ii (4.0 g) in AcOH (20 ml) was treated with HBr (62% in water; 20.3 ml) and stirred at 25°C over night. The dark blue solution was poured into water (240 ml). The resulting suspension was stirred for 15 min and the solid was filtered and air-dried. The solid was stirred in ether (50 ml), collected by filtration and dried in vacuo to afford 3.07 g (99.6% yield) of a colourless solid.

MS (ESI): 279.3.

### 7.iv. (13R,16R)-1-cyclopropyl-7-fluoro-16-[2-fluoro-4-((5S)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid benzyl ester:

This compound was obtained as a pale yellow solid in 59.8% yield, starting from intermediate 7.iii (300 mg) and intermediate 6.vii (149 mg) and using the procedure of Example 1, step 1.A.viii.

¹H NMR (DMSO_{d6}; δ ppm): 0.91-1.08 (4H, m); 1.82-1.88 (1H, dd, J = 3.5 and J = 13); 2.40-2.50 (1H, dd, J = 8.61 and J = 13.5); 3.51-3.56 (1H, dd, J = 5 and J = 11); 3.64-3.70 (1H, m); 3.77 (1H, t, 9.5); 3.84-3.89 (1H, dd, J = 6 and J = 9.5); 4.01-4.11 (4H, m); 4.21 (1H, t, J = 9); 4.55 (1H, dd, J = 3 and J = 10); 4.83 (2H, d, J = 5); 5.08-5.17 (1H, m); 5.26 (2H, s); 5.37 (1H, s); 7.13-7.17 (1H, dt, J = 1.5 and J=9); 7.26 (1H, t, J=9); 7.28 (7H, m); 7.77 (1H, d, J= 1); 8.17 (1H, d, J= 1); 8.47 (1H, s).

MS (ESI): 740.8.

### 7.v. (13R,16S)-1-cyclopropyl-7-fluoro-16-[2-fluoro-4-((5S)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

The title compound (105 mg) was obtained in 60% yield as a pale yellow solid, starting from intermediate 7.iv (200 mg) and using the procedure of Example 1, step 1.B.xiii.

¹H NMR (DMSO_{d6}; δ ppm): 1.00-1.18 (4H, m); 1.85-1.91 (1H, dd, J = 3.5 and J = 13); 2.40-2.50 (1H, dd, J = 7.5 and J = 13.5); 3.63-3.70 (1H, dd, J = 5 and J = 11); 3.74-3.90 (3H, m); 4.07 (1H, dd, J= 3 and J = 11); 4.13 (2H, s); 4.19-4.25 (2H,m); 4.50 (1H, dd, J = 1.7 and J=7); 4.83 (2H, d, J = 5); 5.08-5.15 (1H, m); 5.41 (1H, s); 7.14 (1H, dd, J = 1.5 and J = 9); 7.26 (1H, t, J = 9); 7.50 (1H, dd, J = 2.6 and 13.5); 7.59 (1H, d, J=12.6); 7.77 (1H, d, J = 1); 8.17 (1H, d, J = 1); 8.47 (1H, s); 15.22 (1H, s).

MS (ESI): 650.8.

### Example 8: (13S,16S)-1-cydopropyl-7-fluoro-16-[2-fluoro-4-((5S)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

### 8.i. (13S,16S)-1-cyclopropyl-7-fluoro-16-[2-fluoro-4-((5S)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid benzyl ester:

This compound was obtained as a pale yellow solid in 45% yield, starting from intermediate 1.B.xi (200 mg) and intermediate 7.iii (149.7 mg) and using the procedure of Example 1, step 1.A.viii.

MS (ESI): 740.8.

### 8.ii. (13S,16S)-1-cyclopropyl-7-fluoro-16-[2-fluoro-4-((5S)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

The title compound (65 mg) was obtained in 62.5% yield as a yellow solid by hydrogenation of intermediate 8.i (200 mg) using the procedure of Example 5, step 5.ii.

¹H NMR (DMSO_{d6}; δ ppm): 1.00-1.18 (4H, m); 1.85-1.91 (1H, dd, J = 3.5 and J = 13); 2.40-2.50 (1H, dd, J = 7.5 and J =13.5); 3.63-3.70 (1H, dd, J = 5 and J = 11); 3.74-3.90 (3H, m); 4.07 (1H, dd, J=3 and J=11); 4.13 (2H, s); 4.19-4.25 (2H, m); 4.50 (1H, dd, J = 1.7 and J = 7); 4.83 (2H, d, J = 5); 5.08-5.15 (1H, m); 5.41 (1H, s); 7.14 (1H, dd, J = 1.5 and J = 9); 7.26 (1H, t, J = 9); 7.50 (1H, dd, J = 2.6 and J = 13.5); 7.59 (1H, d, J = 12.6); 7.77 (1H, d, J = 1); 8.17 (1H, d, J = 1); 8.47 (1H, s); 15.22 (1H, s).

MS (ESI): 650.8.

### Example 9: (13S,16R)-1-cyclopropyl-7-fluoro-16-[2-fluoro-4-((5S)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diazacyclopenta[a]phenanthrene-3-carboxylic acid:

### 9.i. (13S,16R)-1-cyclopropyl-7-fluoro-16-[2-fluoro-4-((5S)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diazacyclopenta[a]phenanthrene-3-carboxylic acid benzyl ester:

This compound was obtained as a beige solid in 41.1% yield, starting from intermediates 5.iv (2.0 g) and 7.iii (0.996 mg) and using the procedure of Example 1, step 1.A.viii.

MS (ESI): 740.9.

### 9.ii. (13S,16R)-1-cyclopropyl-7-fluoro-16-[2-fluoro-4-((5S)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diazacyclopenta[a]phenanthrene-3-carboxylic acid:

The title compound (74 mg) was obtained in 84.2% yield as a yellow solid by hydrogenation of intermediate 9.i (1.0 g) using the procedure of Example 5, step 5.ii.

¹H NMR (DMSO_{d6}; δ ppm): 0.98-1.20 (4H, m); 1.74-1.82 (1H, dd, J = 10 and J = 12.5); 2.10-2.16 (1H, dd, J = 6 and J = 12.5); 3.33-3.50 (1H, t, J = 10); 3.77 (1H, dd, J = 3.5 and J = 11); 3.87 (1H, dd, J = 5.7 and J = 9) 4.02-4.24 (6H, m); 4.78 (1H, dd, J = 3.5 and J = 10.5); 4.83 (2H, d, J = 5); 5.08-5.17 (1H,m); 5.41 (1H, s); 7.14 (1H, dt, J = 1 and J = 9); 7.25 (1H, t, J = 9); 7.50 (1H, dd, J = 2.6 and J = 13.5); 7.57 (1H, d, J = 13.5); 7.77 (1H, d, J= 1); 8.17 (1H, d, J= 1); 8.59 (1H, s); 15.26 (1H, s).

MS (ESI): 650.8.

### Example 10: (13R,16S)-1-cyclopropyl-7-fluoro-16-[2-fluoro-4-((5S)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diazacyclopenta[a]phenanthrene-3-carboxylic acid:

### 10.i. (13R,16S)-1-cyclopropyl-7-fluoro-16-hydroxy-16-methanesulfonyloxymethyl-4-oxo-1,4,13,15,16,17-hexahydro-12H-11-oxa-1,14-diaza-cyclopenta[a]phenanthrene-3-carboxylic acid benzyl ester:

This compound was prepared as a colourless solid in 69% yield, starting from intermediate 6.vi.b (243 mg) and using the procedure of Example 6, step 6.vii.

MS (ESI): 558.8.

### 10.ii. (13R,16S)-1-cyclopropyl-7-fluoro-16-[2-fluoro-4-((5S)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diazacyclopenta[a]phenanthrene-3-carboxylic acid benzyl ester:

This compound was obtained as a pale yellow solid in 53.5% yield, starting from intermediates 10.i (100 mg) and 7.iii (49 mg) and using the procedure of Example 1, step 1.A.viii.

MS (ESI): 740.9.

Alternatively, the title compound was obtained as a pale yellow solid in 72% yield, starting from intermediates 10.i (97.5 mg) and 7.iii (48.6 mg) and using the procedure of Example 1, step 1.A.viii. (Same MS data). As a by-product, the intermediate epoxide, 1-cyclopropyl-7-fluoro-4-oxo-1,4,13,15,16,17-hexahydro-12*H*-11-oxa-1,14-diaza-(1-oxaspiro[2.4]hepta)[*a*]phenanthrene-3-carboxylic acid benzyl ester (i.e. the compound of formula III_{G} wherein R³ is fluorine, R⁴ is cyclopropyl, R¹³ is benzyl and R² and OR⁷ form, together with the carbon atoms that carry them, an epoxide ring), was obtained as a colourless powder in 19.90% yield (15 mg; MS (ESI): 462.9).

### 10.iii. (13R,16S)-1-cyclopropyl-7-fluoro-16-[2-fluoro-4-((5S)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diazacyclopenta[a]phenanthrene-3-carboxylic acid:

The title compound (32 mg) was obtained in 38.7% yield as a yellow solid by hydrogenation of intermediate 10.ii (71 mg) using the procedure of Example 5, step 5.ii.

¹H NMR (DMSO_{d6}; δ ppm): 0.98-1.20 (4H, m); 1.74-1.82 (1H, dd, J = 10 and J = 12); 2.04-2.17 (1H, dd, J = 6 and J = 12.5); 3.51 (1H, t, J = 10); 3.9(1H, dd, J = 3.5 and J = 11); 3.87 (1H, dd, J = 5.5 and J = 9); 4.02-4.24 (6H, m); 4.78 (1H, dd, J = 3.5 and J = 10.5); 4.83 (2H, d, J= 5); 5.08-5.17 (1H, m); 5.53 (1H, s); 7.14 (1H, dt, J = 1 and J = 9); 7.25 (1H, t, J = 9); 7.50 (1H, dd, J = 2.6 and J = 13.5); 7.57 (1H, d, J = 13.5); 7.77 (1H, d, J = 1); 8.17 (1H, d, J = 1); 8.59 (1H, s); 15.26 (1H, s).

MS (ESI): 650.7.

### Example 11: (13S,16S)-1-cyclopropyl-7-fluoro-16-[2-fluoro-4-((5R)-2-oxo-5-pyrazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

### 11.i. (5R)-3-(4-benzyloxy-3-fluoro-phenyl)-5-pyrazol-1-ylmethyl-oxazolidin-2-one:

A solution of pyrazole (1.03 g) in 2,3-dimethylimidazolidinone (20 ml) was cooled to 0°C and treated with NaH (555 mg; 60% in mineral oil). The reaction mixture was stirred at rt for 30 min and treated with (5*R*)-methanesulfonic acid 3-(4-benzyloxy-3-fluoro-phenyl)-2-oxo-oxazolidin-5-ylmethyl ester (5 g; prepared according to WO 2004/096221). The reaction mixture was further stirred at rt overnight. The solvent was evaporated *in vacuo.* The residue was taken up in water (50 ml) and the resulting crystals were collected by filtration affording, after recrystallization from EA/Hex, 4.45 g (95.8% yield) of a colourless material.

¹H NMR (DMSO_{d6}; δ ppm): 3.83-3.88 (1H,dd, J = 6 and J = 9); 4.11-4.17 (1H, dd, J1 = J2 = 9); 4.50-4.52 (2H, d, J = 5); 5.00-5.07 (1H, m); 5.16 (2H,s); 6.26-6.28 (1H, dd, J1 = J2 = 2), 7.09-7.14 (1H, m); 7.26-7.31 (3H, m); 7.35-7.50 (5H, m); 7.76 (1H, d, J = 0.6).

### 11.ii. (5R)-3-(3-fluoro-4-hydroxy-phenyl)-5-pyrazol-1-ylrnethyl-oxazolidin-2-one:

A solution of intermediate 11.i (5.0 g) dissolved in EA:MeOH (1:1, 20 ml) was hydrogenated for 12 h at rt over 30 mg 10% Pd/C. The catalyst was removed by filtration. The catalyst was washed with EA (5 ml). The filtrate was evaporated *in vacuo* affording 3.66 g (97% yield) of a colourless material.

¹H NMR (DMSO_{d6}; δ ppm): 3.80 and 4.11 (2H, 2xdd, J = 6 and J = 9); 4.49 (2H, d, J = 2); 4.97-5.05 (1H, m); 6.26-6.28 (1H, dd, J1 = J2 = 2); 6.91-7.03 (2H, m); 7.37 (1H, dd, J = 2 and J = 14); 7.47 (1H, d); 7.67 (1H, dd, J = 2 and J = 5); 9.75 (1H, s).

MS (ESI): 278.4.

### 11.iii. (13S,16S)-1-cyclopropyl-7-fluoro-16-[(5R)-2-fluoro-4-(2-oxo-5-pyrazol-1-ylmethyl-oxazolidin-3-yl)phenoxynethyl]-16-hydroxy-4-oxo-1,4,13,15,16,17-hexahydro-12H-11-oxa-1,14-diaza-cyclopenta[a]phenanthrene-3-carboxylic acid benzyl ester:

This compound was obtained as an orange solid in 58.1% yield, starting from intermediate 1.B.xi (111 mg) and intermediate 11.ii (61 mg) and using the procedure of Example 1, step 1.A.viii.

MS (ESI): 739.8.

### 11.iv. (135,16S)-1-cyclopropyl-7-fluoro-16-[2-fluoro-4-((5R)-2-oxo-5-pyrazol-1-ylmethyl-oxazolidin-3-yl)-phenoxvniethyl]-16-hydroxy-4-oxo-1,4,13,15,16,17-hexahydro-12H-11-oxa-1,14-diaza-cyclopenta[a]phenanthrene-3-carboxylic acid:

The title compound (38 mg) was obtained in 81.4% yield as a yellow solid, starting from intermediate 11.iii (53 mg) and using the procedure of Example 5, step 5.ii.

¹H NMR (DMSO_{d6}; δ ppm): 1.00-1.18 (4H, m); 1.85-1.91 (1H,dd, J = 3.5 and J = 13); 2.40-2.50 (1H, m); 3.63-3.70 (1H, dd, J = 5 and J = 9); 3.74-3.90 (3H, m); 4.07-4.25 (5H, m); 4.5 (2H, d, J = 6); 4.6 (1H, dd, J = 1 and J = 7.5); 5.08-5.15 (1H, m); 5.41 (1H, s); 6.25 (1H, t, J = 1); 7.14 (1H, ddd, J = 1, J = 2.5 and J = 9); 7.25 (1H, t, J = 9); 7.42 (1H, s); 7.50 (1H, dd, J = 2.6 and J = 13); 7.59 (1H, d, J = 12.5); 7.80 (1H, s); 8.6 (1H, s), 15.25 (1H, s).

MS (ESI): 649.9.

### Example 12: (13S,16S)-1-cyclopropyl-7-fluoro-16-[2-fluoro-4-((5S)-5-imidazol-1-ylmethyl-2-oxo-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

### 12.i. (5S)-3-(4-benzyloxy-3-fluoro-phenyl)-5-imidazol-1-ylmethyl-oxazolidin-2-one:

This compound (3.3 g) was obtained in 71.0% yield as a colourless solid, starting from (5*R*)-methanesulfonic acid 3-(4-benzyloxy-3-fluoro-phenyl)-2-oxo-oxazolidin-5-ylmethyl ester (5.0 g) and imidazole (5.99 g) and using the procedure of Example 11, step 11.i.

¹H NMR (DMSO_{d6}; δ ppm): 3.73-3.80 (1H, dd, J= 6 and J = 9); 4.10-4.17 (1H, t, J = 9); 4.36-4.50 (2H, m); 4.92-5.01 (1H, m); 5.17 (2H, s); 6.90-6.91(1H, d, J1 = 1), 7.09-7.14 (1H, m); 7.26-7.31 (2H, m); 7.35-7.50 (6H, m); 7.68 (1H,s).

MS (ESI): 368.5.

### 12.ii. (5S)-3-(3-fluoro-4-hydroxy-phenyl)-5-imidazol-1-ylmethyl-oxazolidin-2-one:

This compound (1.91 g) was obtained in 76.7% yield as a colourless solid, starting from intermediate 12.i (3.33 g) and using the procedure of Example 11, step 11.ii.

¹H NMR (DMSO_{d6}; δ ppm): 3.75 and 4.11 (2H, 2xdd, J = 6 and J = 9); 4.40 (2H, m); 4.90-5.00 (1H, m); 6.91 (1H, s); 6.91-7.07 (2H, m); 7.22 (1H, s); 7.35-7.79 (1H, dd, J = 2.5 and J = 14); 7.67 (1H, s); 9.77 (1H, s).

MS (ESI): 278.3.

### 12.iii. (13S,16S)-1-cyclopropyl-7-fluoro-16-[2-fluoro-4-((5S)-5-imidazol-1-ylntethyl-2-oxo-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid benzyl ester:

The compound (79 mg) was obtained as an orange solid in 53.4% yield, starting from intermediates 1.B.xi (112 mg) and 12.ii (61 mg) and using the procedure of Example 1, step 1.A.viii.

MS (ESI): 739.8.

### 12.iv. (13S,16S)-1-cyclopropyl-7-fluoro-16-[2-fluoro-4-((5S)-5-imidazol-1-ylmethyl-2-oxo-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

The title compound (30 mg) was obtained in 68.5% yield as a yellow solid, starting from intermediate 12.iii (50 mg) and using the procedure of Example 5, step 5.ii.

¹H NMR (DMSO_{d6}; δ ppm): 1.00-1.18 (4H, m); 1.85-1.91 (1H, dd, J = 3.5 and J = 13); 2.40-2.50 (1H, m); 3.63-3.70 (1H, dd, J = 5 and J = 9); 3.74-3.90 (3H, m); 4.07-4.25 (5H, m); 4.4 (2H, d, J = 6); 4.83 (1H, d, J = 8); 5.08-5.15 (1H, m); 5.41 (1H, s); 6.80 (1H, s); 7.14-7.26 (3H, m); 7.50 (1H, dd, J = 2.6 and J = 13); 7.59 (1H, d, J = 12.5); 7.70 (1H, s); 8.6 (1H, s), 15.25 (1H, s).

MS (ESI): 649.8.

### Example 13: (13S,16S)-1-cyclopropyl-7-fluoro-16-{2-fluoro-4-[(5R)-5-(4-methyl-[1,2,3]triazol-1-ylmethyl)-2-oxo-oxazolidin-3-yl]-phenoxymethyl}-16-hydroxy-4-oxo-1,4,13,15,16,17-hexahydro-12H-11-oxa-1,14-diaza-cyclopenta[a]phenanthrene-3-carboxylic acid:

### 13.i. (5S)-5-aminomethyl-3-(4-benzyloxy-3-fluoro-phenyl)-oxazolidin-2-one:

A solution of (5*R*)-5-azidomethyl-3-(4-benzyloxy-3-fluoro-phenyl)-oxazolidin-2-one (5.0g; prepared according to WO 2004/096221) in THF (80 ml) was sequentially treated with PPh₃ (4.4 g) and water (2.62 ml). The solution was stirred at 80°C overnight. The solvent was removed under reduced pressure and the residue was purified by chromatography over SiO₂ using DCM:MeOH (9:1) as eluent, affording 4.32 g (93.5% yield) of a colourless solid.

MS (ESI): 317.0.

### 13.ii. (5R)-3-(4-benzyloxy-fluoro-phenyl)-5-(4-naethyl-[1,2,3]triazol-1-ylmethyl)-oxazolidin-2-one:

A suspension of intermediate 13.i (4.3 g) in dry MeOH (200 ml) was treated with DIPEA (9 ml) and *N*-[(1*E*)-1-(dichloromethyl)propylidene]-4-methylbenzenesulfonohydrazide (5.04 g; prepared according to J. Med. Chem. (2005), 48, 499-506) at 0°C. The reaction mixture was further stirred at RT overnight. The solvent was evaporated under reduced pressure and the residue was dissolved in DCM/MeOH (9/1) and purified by chromatography over SiO₂ (eluent DCM/MeOH (95/5). The relevant fractions were pooled and evaporated affording 3.87 g (74.1% yield) of a colourless solid.

¹H NMR (DMSO_{d6}; δ ppm): 2.22 (3H, s); 3.84 (1H, dd, J = 6 and J = 9); 4.19 (1H, t , J = 9); 4.74 (2H, d, J = 5); 5.07 (1H, m); 5.17 (2H, s); 7.12 (1H, ddd, J = 1.5, J = 2.5 and J = 9); 7.27 (1H, t, J = 12.5); 7.30-7.50 (6H, m); 7.76 (1H, d, J = 1).

MS (ESI): 383.1.

### 13.iii. (5R)-3-(3-fluoro-4-hydroxy-phenyl)-5-(4-methyl-[1,2,3]triazol-1-ylmethyl)-oxazolidin-2-one:

This compound (2.85 g) was obtained as a colourless solid in 100% yield, starting from intermediate 13.ii (3.33 g) and using the procedure of Example 11, step 11.ii.

¹H NMR (DMSO_{d6}; δ ppm): 2.23 (3H, s); 3.80 (1H, dd, J = 6 and J = 9); 4.16 (1H, t, J = 9); 4.73 (2H, d, J = 5.3); 5.01-5.10 (1H, m); 6.94 (1H, t, J = 9.5); 7.02 (1H, ddd, J = 1, J = 2.7 and J = 9); 7.37 (1H, dd, J = 2.5 and J = 13.5); 7.87 (1H, s); 9.76 (1H, s).

MS (ESI): 293.1.

### 13.iv. (13S,16S)-1-cyclopropyl-7-fluoro-16-{2-fluoro-4[(5R)-5-(4-methyl-[1,2,3]triazol-1-ylmethyl)-2-oxo-oxazolidin-3-yl]-phenoxymethyl}-16-hydroxy-4-oxo-1,4,13,15,16,17-hexahydro-12H-11-oxa-1,14-diaza-cyclopenta[a]phenanthrene-3-carboxylic acid benzyl ester:

This compound (78 mg) was obtained as an orange solid in 51.6% yield, starting from intermediates 1.B.xi (112 mg) and 13.iii (64 mg) and using the procedure of Example 1, step 1.A.viii.

MS (ESI): 754.8.

### 13.v. (13S,16S)-1-cyclopropyl-7-fluoro-16-{2-fluoro-4-[(5R)-5-(4-methyl-[1,2,3]triazol-1-ylmethyl)-2-oxo-oxazolidin-3-yl]-phenoxymethyl}-16-hydroxy-4-oxo-1,4,13,15,16,17-hexahydro-12H-11-oxa-1,14-diaza-cyclopenta[a]phenanthrene-3-carboxylic acid:

The title compound (29 mg) was obtained as a yellow solid in 68.8% yield, starting from intermediate 13.iv (50 mg) and using the procedure of Example 5, step 5.ii.

¹H NMR (DMSO_{d6}; δ ppm): 1.00-1.18 (4H, m); 1.85-1.91 (1H, dd, J = 3.5 and J = 13); 2.25 (3H, s); 2.40-2.50 (1H, m); 3.63-3.70 (1H, dd, J = 5 and J = 9); 3.74-3.90 (3H, m); 4.07-4.25 (5H, m); 4.5 (1H, dd, J = 1 and J = 7.5); 4.6 (2H, d, J = 6); 5.08-5.15 (1H,m); 5.41 (1H, s); 7.14 (1H, ddd, J = 1 and 2.5 and 9); 7.25 (1H, t, J = 9); 7.50 (1H, dd, J = 2.6 and 13); 7.59 (1H, d, J = 12.5); 7.80 (1H, s); 8.6 (1H, s), 15.25 (1H,s).

MS (ESI): 754.9.

### Example 14: (13S,16S)-1-cyclopropyl-7-fluoro-16-[2-fluoro-4-((5R)-2-oxo-5-tetrazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

### 14.i. (5R)-3-(4-benzyloxy-3-fluoro-phenyl)-5-tetrazol-1-ylmethyl-oxazolidin-2-one:

A 0.45M solution of tetrazole in MeCN (33.72 ml) was diluted with 2,3-dimethylimidazolidinone (20 ml). MeCN was removed *in vacuo* and the resulting solution was treated at 0°C with NaH (55 mg, 60% dispersion in mineral oil). The suspension was stirred at rt for 30 min and treated with (5*R*)-methanesulfonic acid 3-(4-benzyloxy-3-fluoro-phenyl)-2-oxo-oxazolidin-5-ylmethyl ester (5 g). The solution was stirred at 60°C for 2 days. The solvent was evaporated under reduced pressure and the residue was taken up in water (50 ml) and stirred for 1 h. The resulting crystals were collected by filtration and washed with ether (50 ml). The solid was purified by chromatography over SiO₂ using EA and EA:DCM (9:1) as eluent, affording 1.75 g (37.5% yield) of a colourless material.

¹H NMR (DMSO_{d6}; δ ppm): 3.89 (1H, dd, J = 5.5 and J = 9.5); 4.22 (1H, t, J = 9.5); 4.93 (2H, d, J = 5); 5.1-5.2 (3H, m); 7.13 (1H, ddd, J = 1.5, J = 2.5 and J = 9); 7.29 (1H, t, J = 9); 7.3-7.52 (6H, m); 9.5 (1H, s).

MS (ESI): 370.3.

### 14.ii. (5R)-3-(3-fluoro-4-hydroxy-phenyl)-5-tetrazol-1-ylmethyl-oxazolidin-2-one:

This compound (1.80 g) was obtained as a colourless solid in 89.2% yield, starting from intermediate 14.i (2.67 g) and using the procedure of Example 11, step 11.ii.

¹H NMR (DMSO_{d6}; δ ppm): 3.87 (1H, dd, J = 5.5 and J = 9.5); 4.20 (1H, t, J = 9.5); 4.92 (2H, d, J = 5.5); 5.09-5.1 (1H, m); 6.95 (1H, t, J = 9); 7.05(1H, ddd, J = 1, J = 2.5 and J = 9); 7.36 (1H, dd, J = 2.5 and J = 13.5); 9.47 (1H, s); 9.78 (1H, s).

MS (ESI): 230.3.

### 14.iii. (13S,16S)-1-cyclopropyl-7-fluoro-16-[2-fluoro-4-((5R)-2-oxo-5-tetrazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid benzyl ester:

This compound (38 mg) was obtained as a yellow solid in 25.6% yield, starting from intermediates 1.B.xi (112 mg) and 14.ii (61.4 mg) and using the procedure of Example 1, step 1.A.viii.

MS (ESI): 742.0.

### 14.iv. (13S,16S)-1-cyclopropyl-7-fluoro-16-[2-fluoro-4-((5R)-2-oxo-5-tetrazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

The title compound (12 mg) was obtained as an off-white solid in 39% yield, starting from intermediate 14.iii (50 mg) and using the procedure of Example 5, step 5.ii.

¹H NMR (DMSO_{d6}; δ ppm): 0.95-1.15 (4H, m); 1.95 (1H, d, J = 11); 2.2 (1H, m); 3.6-3.85 (3H, m); 3.90-3.95 (1H, m); 4.0-4.3 (5H, m); 4.6 (1H, d, J = 8); 5.0 (2H, d, J=6); 5.2 (1H, m); 5.4 (1H, s); 7.15 (1H, ddd, J= 1, J=2.5 and J=9); 7.25 (1H, t, J=9); 7.42 (1H, d, J=13.5); 7.6 (1H, d, J = 12.5); 8.6 (1H, s); 9.45 (1H, s); 15.25 (1H, s).

MS (ESI): 651.8.

### Example 15: (13S,16S)-1-cyclopropyl-7-fluoro-16-[2-fluoro-4-((5R)-2-oxo-5-[1,2,4]triazol-1-ylmethyl-oxazolidin-3-yl)-phenoxymethyl]-16-hydroxy-4-oxo-1,4,13,15,16,17-hexahydro-12H-11-oxa-1,14-diazacyclopenta[a]phenanthrene-3-carboxylic acid:

### 15.i. (5R)-3-(4-benzyloxy-3-fluoro-phenyl)-5-[1,2,4]triazol-1-ylmethyl-oxazolidin-2-one:

This compound (650 mg) was obtained as a colourless solid in 69.8% yield, starting from (5*R*)-methanesulfonic acid 3-(4-benzyloxy-3-fluoro-phenyl)-2-oxo-oxazolidin-5-ylmethyl ester (1.0 g) and 1,2,4-triazole (187 mg) and using the procedure of Example 11, step 11.i.

¹H NMR (DMSO_{d6}; δ ppm): 3.88 (1H, dd, J = 5.5 and J = 9); 4.18 (1H, t, J = 9); 4.61 (2H, m); 5.02-5.11 (1H, m); 5.17 (2H, s); 7.11(1H, ddd, J = 1.5, J = 2.5 and J = 10); 7.29 (1H, t, J = 9); 7.35-7.52 (6H, m); 8.0 (1H, s); 8.57 (1H, s).

MS (ESI): 369.2.

### 15.ii. (5R)-3-(3-fluoro-4-hydroxy-phenyl)-5-[1,2,4]triazol-1-ylmethyl-oxazolidin-2-one:

A solution of intermediate 15.i (630 mg) in AcOH (3 ml) was treated with a mixture of HBr (3 ml; 62% in water) and AcOH (3 ml). The solution was stirred at rt for 8 h and poured into water (15 ml). The precipitate was stirred for 15 min and filtered. The residue was washed with water (2 x 10 ml) and dried *in vacuo* to afford 373 mg (78.35% yield) of a white powder.

¹H NMR (DMSO_{d6}; δ ppm): 3.85 (1H, dd, J = 5.7 and J = 9); 4.15 (1H, t, J = 9); 4.62 (2H, m); 5.05 (1H, m); 6.94 (1H, t, J = 9.5); 7.04 (1H, ddd, J = 1.5, J = 2.5 and J = 9.5); 7.38 (1H, dd, J = 2.5 and J = 13.5); 8.0 (1H, s); 8.57 (1H, s); 9.75 (1H, s).

MS (ESI): 279.01.

### 15.iii. (13S,16S)-1-cyclopropyl-7-fluoro-16-[2-fluoro-4-((5R)-2-oxo-5-[1,2,4]triazol-1-ylmethyl-oxazolidin-3-yl)-phenoxymethyl]-16-hydroxy-4-oxo-1,4,13,15,16,17-hexahydro-12H-11-oxa-1,14-diazacyclopenta[a]phenanthrene-3-carboxylic acid benzyl ester:

This compound (90 mg) was obtained as a yellow solid in 9% yield, starting from intermediates 1.B.xi (749 mg) and 15.ii (373 mg) and using the procedure of Example 1, step 1.A.viii.

MS (ESI): 740.9.

### 15.iv. (13S,16S)-1-cyclopropyl-7-fluoro-16-[2-fluoro-4-((5R)-2-oxo-5-[1,2,4]triazol-1-ylmethyl-oxazolidin-3-yl)-phenoxyniethyl]-16-hydroxy-4-oxo-1,4,13,15,16,17-hexahydro-12H-11-oxa-1,14-diazacyclopenta[a]phenanthrene-3-carboxylic acid:

The title compound (68 mg) was obtained as a yellow solid in 89% yield, starting from intermediate 15.iii (86 mg) and using the procedure of Example 5, step 5.ii.

¹H NMR (DMSO_{d6}; δ ppm): 0.98-1.03 (4H, m); 1.87 (1H, dd, J = 4 and J = 13.5); 2.43 (1H, dd, J = 8.3 and J = 13.5); 3.62 (1H, dd, J = 5 and J = 11); 3.69-3.76 (2H, m); 3.88 (1H, dd, J = 6 and J = 9.3); 4.04 (1H, dd, J = 3 and J = 11); 4.10 (2H, s); 4.17 (1H, t, J = 9); 4.22-4.25 (1H, m); 4.53-4.66 (2H, m); 5.01-5.09 (1H, m); 5.38 (1H, s); 7.12 (1H, d broad, J = 9); 7.25 (1H, t, J = 9); 7.48 (1H, dd, J = 2.6 and J = 13.5); 7.57 (1H, d, J = 12.6); 7.98 (1H, s); 8.55 (1H, s); 8.59 (2H, s); 15.2 (1H, s).

MS (ESI): 650.7.

### Example 16: (13S,16S)-1-cyclopropyl-7-fluoro-16-{2-fluoro-4-[(5R)-5-(isoxazol-3-yloxymethyl)-2-oxo-oxazolidin-3-yl]-phenoxymethyl}-16-hydroxy-4-oxo-1,4,13,15,16,17-hexahydro-12H-11-oxa-1,14-diaza-cyclopenta[a]phenanthrene-3-carboxylic acid:

### 16.i. (5R)-3-(4-benzyloxy-3-fluoro-phenyl)-5-(isoxazol-3-yloxymethyl)-oxazolidin-2-one:

A solution of (5*R*)-3-(4-benzyloxy-3-fluoro-phenyl)-5-hydroxymethyl-oxazolidin-2-one (6.34 g), isoxazol-3-ol (1.87 g; prepared according to Chem. Pharm. Bull. (1966), 14, 1277-86) and PPh₃ (6.81 g) in THF (200 ml) was treated dropwise with DIAD (5.04 ml). The reaction was stirred at rt for 2 h. The solvent was evaporated under reduced pressure and the residue was purified by chromatography over SiO₂ using DCM/EA/Hex (1/5/4) as eluent. The relevant fractions were pooled and evaporated under reduced pressure. The residue was crystallized from EA/Hex affording 7.32 g of a colourless solid.

¹H NMR (DMSO_{d6}; δ ppm): 3.90 (1H; dd, J = 6.6 and J = 9); 4.16 (1H, t; J = 9); 4.42-4.52 (2H, m); 5.02-5.10 (1H, m); 5.18 (2H, s); 6.40 (1H, d, J = 2); 7.19 (1H, ddd, J = 1, J = 2.5 and J = 9.5); 7.22 (1H, t, J = 9); 7.30-7.50 (5H, m); 7.60 (1H, dd, J = 2.5 and J = 13.5); 8.70 (1H, d, J = 2).

MS (ESI): 385.3.

### 16.ii. (5R)-3-(3-fluoro-4-hydroxy-phenyl)-5-(isoxazol-3-yloxymethyl)-oxazolidin-2-one:

This compound (1.27 g) was prepared as a colourless solid in 79.5% yield, starting from intermediate 16.i (2 g) and using the procedure of Example 15, step 15.ii.

¹H NMR (DMSO_{d6}; δ ppm): 3.83 (1H, dd, J = 6.5 and J = 9); 4.14 (1H, t, J = 9); 4.41-4.51 (2H, m); 5.0-5.08 (1H, m); 6.4 (1H, s); 6.96 (1H, dd, J = 9 and J = 9.8); 7.10(1H, ddd, J = 1.2, J = 2.7 and J = 9); 7.49 (1H, dd, J = 2.5 and J = 13.5); 8.70 (1H, s); 9.73 (1H, s).

MS (ESI): 295.3.

### 16.iii. (13S,16S)-1-cyclopropyl-7-fluoro-16-{2-fluoro-4-[(5R)-5-(isoxazol-3-yloxymethyl)-2-oxo-oxazolidin-3-yl]-phenoxymethyl}-16-hydroxy-4-oxo-1,4,13,15,16,17-hexahydro-12H-11-oxa-1,14-diaza-cyclopenta[a]phenanthrene-3-carboxylic acid benzyl ester:

This compound (567 mg) was obtained as a beige solid in 84.8% yield, starting from intermediates 1.B.xi (494 mg) and 16.ii (260 mg) and using the procedure of Example 1, step 1.A.viii.

MS (ESI): 756.7.

### 16.iv. (13S,16S)-1-cyclopropyl-7-fluoro-16-{2-fluoro-4-[(5R)-5-(isoxazol-3-yloxymethyl)-2-oxo-oxazolidin-3-yl]-phenoxymethyl}-16-hydroxy-4-oxo-1,4,13,15,16,17-hexahydro-12H-11-oxa-1,14-diaza-cyclopenta[a]phenanthrene-3-carboxylic acid:

A solution of intermediate 16.iii (150 mg) in AcOH (0.5 ml) was treated with HBr (0.5 mL; 62 % in water). The reaction mixture was stirred at rt for 5 days. The reaction mixture was poured into water (20 ml) and the resulting crystals were collected by filtration, washed with water (5 ml) and air dried. The solid was suspended in ether (10 ml), stirred for 10 min, filtered and washed with ether (5 ml). The solid was dissolved in DCM/MeOH (50 ml; 4:1). The DCM was removed under reduced pressure and the residue diluted with MeOH (10 ml). The resulting crystals were collected by filtration and washed with MeOH, affording 89 mg (67.4% yield) of a yellow solid.

¹H NMR (DMSO_{d6}; δ ppm): 0.98-1.15 (4H, m); 1.78 (1H, dd, J = 3 and J= 9); 2.50 (1H, m); 3.63 (1H, dd, J = 3 and J = 8); 3.70-3.90 (2H, m); 3.91 (1H, dd, J = 6.7 and J = 9); 4.05 (1H, dd, J = 3 and J = 11); 4.10 (2H, s), 4.14-4.23 (2H, m); 4.26-4.4.53 (2H, m); 4.56-4.62 (1H, d broad, J = 9); 5.03-5.11 (1H, m); 5.40 (1H, s); 6.40 (1H, s); 7.10-7.30 (2H, m); 7.60 (2H, d, J = 12.6); 8.62 (1H, s); 8.70 (1H, s); 15.20 (1H, s).

MS (ESI): 666.6.

### Example 17: (13S,16S)-1-cydopropyl-7-fluoro-16-[2-fluoro-4-((5S)-2-oxo-5-[(2,2,2-trifluoro-acetylamino)methyl]-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

### 17.i. N-[(S)-3-(4-benzyloxy-3-fluoro-phenyl)-2-oxo-oxazolidin-5-ylmethyl]-2,2,2-trifluoro-acetamide:

A solution of intermediate 13.i (1.58 g) in DCM (30 ml) was sequentially treated at 0°C with pyridine (0.805 ml) and trifluoroacetic anhydride (0.847 ml). The reaction was further stirred at rt for 4 h. The reaction was diluted with EA (250 mL) and sequentially washed with water, sat. aq. CuSO₄, sat. NaHCO₃, water and brine (each 100 ml). The org. layer was dried over MgSO₄ and filtered. The filtrate was evaporated under reduced pressure to afford a white solid (2 g; 97% yield).

¹H NMR (DMSO_{d6}; δ ppm): 3.57 (2H, t, J = 5.5); 3.78 (1H, dd, J = 6 and J = 9); 4.14 (1H, t, J=9); 4.77-4.86 (1H, m); 5.17 (1H, s); 7.16 (1H, ddd, J = 1, J=2.5 and J = 9); 7.28 (1H, t, J = 9); 7.34-7.47 (6H, m); 7.54 (1H, dd, J = 2.5 and J = 13.5); 9.79 (1H, t broad, J = 5.5).

MS (ESI): 412.8.

### 17.ii. 2,2,2-trifluoro-N-[(S)-3-(3-fluoro-4-hydroxy-phenyl)-2-oxo-oxazolidin-5-ylmethyl]-acetamide:

A solution of intermediate 17.i (1.90 g) in DMA (15 ml) was hydrogenated over 10 % Pd/C (200 mg). The catalyst was filtered off and washed with DMA (5 ml) and the filtrate was evaporated under reduced pressure. The resulting oil crystallized by addition of water (100 ml). The solid was collected by filtration and sequentially washed with water (20 ml) and Hex affording 1.3 g (87.5%) of a colourless solid.

¹H NMR (DMSO_{d6}; δ ppm): 3.56 (2H, t, J = 6); 3.75 (1H, dd, J = 6 and J = 9); 4.11 (1H, t, J = 9); 4.75-4.84 (1H, m); 6.96 (1H, t, J = 9); 7.08 (1H, ddd, J = 1, J = 2.5 and J = 9); 7.43 (1H, dd, J = 2.5 and J = 13.5); 9.74 (1H, s); 9.79 (1H, t broad, J = 6).

MS (ESI): 323.1.

### 17.iii. (135,16S)-1-cyclopropyl-7-fluoro-16-[2-fluoro-4-((5S)-2-oxo-5-[(2,2,2-trifluoro-acetylamino)methyl]-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid benzyl ester:

This compound (127 mg) was obtained as a yellow solid in 60.3% yield, starting from intermediates 1.A.vii (150 mg) and 17.ii (86 mg) and using the procedure of Example 1, step 1.A.viii.

MS (ESI): 784.9.

### 17.iv. (13S,16S)-1-cyclopropyl-7-fluoro-16-[2-fluoro-4-((5S)-2-oxo-5-[(2,2,2-trifluoro-acetylamino)methyl]-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

A solution of intermediate 17.iii (121 mg) in DMA (5 ml) was hydrogenated over 10 % Pd/C (20 mg). The catalyst was filtered off and washed with DMA (1 ml). The filtrate was evaporated under reduced pressure. The resulting oil was dissolved in DCM/MeOH (9/1) and filtered. The filtrate was evaporated under reduced pressure, affording 50 mg (46.7% yield) of a yellow solid.

¹H NMR (DMSO_{d6}; δ ppm): 1.00-1.20 (4H, m); 1.90 (1H, m); 2.48 (1H, m); 3.56-3.81 (6H, m); 4.04-4.18 (5H, m); 4.60 (1H, m); 5.42 (1H, s); 5.76 (1H, s); 7.21-7.28 (2H, m); 7.57 (1H, dd, J = 2.5 and J = 13.5); 7.6 (1H, d, J = 12.5); 8.6 (1H, s); 9.80 (1H, t, J = 6); 15.24 (1H, s).

MS (ESI): 694.88.

### Example 18: (13S,16R)-1-cyclopropyl-7-fluoro-16-[2-fluoro-4-((5S)-2-oxo-5-[(2,2,2-trifluoro-acetylamino)methyl]-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid: 18.i. (13S,16R)-1-cyclopropyl-7-fluoro-16-[2-fluoro-4-((5S)-2-oxo-5-[(2,2,2-trifluoro-acetylamino)methyl]-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid benzyl ester:

This compound (118 mg) was obtained as a yellow solid in 56% yield, starting from intermediates 5.iv (150 mg) and 17.ii (86 mg) and using the procedure of Example 1, step 1.A.viii.

MS (ESI): 784.7.

### 18.ii. (13S,16R)-1-cyclopropyl-7-fluoro-16-[2-fluoro-4-((5S)-2-oxo-5-[(2,2,2-trifluoro-acetylamino)methyl]-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

This compound (65 mg) was obtained as a yellow solid in 62% yield, starting from intermediate 18.i (118 mg) and using the procedure of Example 17, step 17.ii.

¹H NMR (DMSO_{d6}; δ ppm): 1.0-1.14 (4H, m); 1.79 (1H, t broad, J = 10); 2.10-2.16 (1H, dd, J = 5.5 and J = 12); 3.51 (1H, t, J = 10); 3.58 (2H, t, J = 5.5); 3.76-3.81 (2H, m); 3.96-4.24 (6H, m); 4.74-4.85 (2H, m); 5.53 (1H, s); 7.20 (1H, dd, J = 2 and J = 9); 7.27 (1H, t, J = 9); 7.53 (1H, dd, J = 2.5 and J = 12.5); 7.57 (1H, d, J = 13.5); 8.60 (1H, s); 9.80 (1H, t broad, J = 5.8); 15.20 (1H, s).

MS (ESI): 695.0.

### Example 19: (13S,16S)-1-ethyl-7-fluoro-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

### 19.i. 1-ethyl-6,7-difluoro-8-hydroxy-4-oxo-1,4-dihydro-quinoline-3-carboxylic acid:

48% aq. HBr (35 ml) was added to a solution of 1-ethyl-6,7-difluoro-8-methoxy-4-oxo-1,4-dihydro-quinoline-3-carboxylic acid ethyl ester (9.34 g; prepared according to EP 241 206) in AcOH (30 ml). The orange solution was stirred at 110 °C for 24 h. It was poured into water (200 mL) and the grey-white precipitate was filtered and dried to provide a beige solid (6.374 g, 79% yield).

¹H NMR (DMSO_{d6}; δ ppm): 1.43 (3H, t, J = 7); 4.85 (2H, q, J = 7); 7.76 (1H, dd, J = 8 and J = 10); 8.91 (1H, s); 12.02 (1H, broad); 14.90 (1H, broad).

MS (ESI): 269.8.

### 19.ii. 1-ethyl-6,7-difluoro-8-hydroxy-4-oxo-1,4-dihydro-quinoline-3-carboxylic acid ethyl ester:

Chlorotrimethylsilane (30 ml) was added to a suspension of intermediate 19.i (6.256 g) in DCM (55 ml) and EtOH (55 ml). The reaction mixture was stirred at 60°C for 6 days. The reaction mixture was concentrated under reduced pressure and the residue taken up in water (100 ml). It was stirred, filtered and the precipitate washed with ether (4 x 25 ml) and dried to afford a brownish solid (6.21 g, 90% yield).

¹H NMR (DMSO_{d6}; δ ppm): 1.28 (3H, t, J = 7); 1.37 (3H, t, J = 7); 4.22 (2H, q, J = 7); 4.66 (2H, q, J = 7); 7.63 (1H, dd, J = 9 and J = 11); 8.55 (1H, s); 11.51 (1H, s).

MS (ESI): 298.1.

### 19.iii. 8-((S)-1-tert-butoxycarbonyl-4-methylene-pyrrolidin-2-ylmethoxy)-1-ethyl-6,7-difluoro-4-oxo-1,4-dihydro-quinoline-3-carboxylic acid ethyl ester:

This compound was obtained as a beige solid in 52.4% yield, starting from intermediate 19.ii (1.0 g), (2*S*)-2-(hydroxymethyl)-4-methylene-1-pyrrolidinecarboxylic acid *tert*-butyl ester (1.98 g), PPh₃ (3.30 g) and DIAD (2.66 ml) and using the procedure of Example 1, step 1.B.v.

¹H NMR (DMSO_{d6}; δ ppm): 1.29 (3H, t, J = 7); 1.33 (3H, t, J = 7); 1.42 (9H, s); 2.65-2.75 (1H, m); 2.85-2.99 (1H, m); 3.75-3.93 (1H, m); 3.95-4.35 (4H, m); 4.23 (2H, q, J = 7); 4.43-4.55 (2H, m); 5.06 (2H, s); 7.93 (1H, dd, J = 9 and J = 10); 8.62 (1H, s); (contaminated by 40% PPh₃O).

MS (ESI): 492.8.

### 19.iv. 8-((S)-1-tert-butoxycarbonyl-4-methylene-pyrrolidin-2-ylmethoxy)-1-ethyl-6,7-difluoro-4-oxo-1,4-dihydro-quinoline-3-carboxylic acid:

This compound was obtained as a colourless solid in 59.2% yield, starting from intermediate 19.iii (3.0 g) and LiOH (803 mg) and using the procedure of Example 1, step 1.B.vi. The crude reaction product was stirred in a mixture of dioxane/EA (1:1; 80 ml) and filtered prior to acidification.

¹H NMR (DMSO_{d6}; δ ppm): 1.38 (3H, t, J = 7); 1.42 (9H, s); 2.65-2.75 (1H, m); 2.87-3.02 (1H, m); 3.75-3.93 (1H, m); 3.95-4.38 (4H, m); 4.60-4.75 (2H, m); 5.06 (2H, s); 8.10 (1H, dd, J = 9 and J =10); 9.00 (1H, s); 14.71 (1H, s).

MS (ESI): 465.0.

### 19.v. 8-((S)-1-tert-butoxycarbonyl-4-methylene-pyrrolidin-2-ylmethoxy)-1-ethyl-6,7-difluoro-4-oxo-1,4-dihydro-quinoline-3-carboxylic acid benzyl ester:

This compound was obtained as an orange oil in 100% yield, starting from intermediate 19.iv (747 mg) and BnBr (0.5 ml) and using the procedure of Example 1, step 1.B.vii.

¹H NMR (DMSO_{d6}; δ ppm): 1.32 (3H, t, J = 7); 1.42 (9H, s); 2.65-2.75 (1H, m); 2.82-3.00 (1H, m); 3.75-3.93 (1H, m); 3.95-4.35 (4H, m); 4.45-4.58 (2H, m); 5.06 (2H, s); 5.30 (2H, s); 7.30-7.44 (3H, m); 7.47-7.52 (2H, m); 7.96 (1H, dd, J = 9 and J = 10); 8.68 (1H, s).

MS (ESI): 554.9.

### 19.vi. 8-((2S,4S)-1-tert-butoxycarbonyl-4-hydroxy-4-hydroxymethyl-pyrrolidin-2-ylmethoxy)-1-ethyl-6,7-difluoro-4-oxo-1,4-dihydro-quinoline-3-carboxylic acid benzyl ester:

This compound was obtained as a beige solid in 69% yield (dr 93:7), starting from intermediate 19.v (2.27 g) and AD-mix α and using the procedure of Example 1, step 1.B.viii.

¹H NMR (DMSO_{d6}; δ ppm): 1.30-1.45 (12H, m); 1.85-1.98 (1H, m); 2.15-2.33 (1H, m); 3.07-3.18 (1H, m); 3.30-3.37 (2H, m); 3.43-3.50 (1H, m); 4.12-4.37 (2H, m); 4.46 (1H, t, J = 9); 4.50-4.65 (2H, m); 4.82-4.90 (1H, m); 4.93 (1H, t, J = 6); 5.31 (2H, s); 7.31-7.43 (3H, m); 7.48-7.52 (2H, m); 7.95 (1H, dd, J = 9 and J = 10); 8.69 (1H, s). MS (ESI): 588.8.

### 19.vii. 1-ethyl-6,7-difluoro-8-((2S,4S)-4-hydroxy-4-hydroxymethyl-pyrrolidin-2-ylmethoxy)-4-oxo-1,4-dihydro-quinoline-3-carboxylic acid benzyl ester hydrochloride:

This compound was obtained as a colourless solid in 81% yield, starting from intermediate 19.vi (1.66 g) and 6*M* HCl in dioxane (2.3 ml) and using the procedure of Example 1, step 1.B.ix.

¹H NMR (DMSO_{d6}; δ ppm): 1.35 (3H, t, J = 7); 1.64-1.73 (1H, m); 2.35-2.43 (1H, m); 3.08-3.51 (4H, m); 4.15 (1H, m); 4.43-4.84 (5H, m); 5.31 (2H, s); 5.44 (1H, m); 7.29-7.46 (3H, m); 7.49-7.53 (2H, m); 7.97 (1H, dd, J = 9 and J = 11); 8.70 (1H, s), 9.52 (1H, broad); 10.03 (1H, broad).

MS (ESI): 489.0 (M+H-HCl)⁺

### 19.viii. (13S,16S)-1-ethyl-7-fluoro-16-hydroxy-16-hydroxymethyl-4-oxo-1,4,13,15,16,17-hexahydro-12H-11-oxa-1,14-diaza-cyclopenta[a]phenanthrene-3-carboxylic acid benzyl ester:

This compound was obtained as a colourless solid in 64.5% yield, starting from intermediate 19.vii (1.19 g), NaHCO₃ (383 mg) and DIPEA (0.78 ml) and using the procedure of Example 1, step 1.B.x. The crude product was purified by stirring in EtOH (10 ml).

¹H NMR (DMSO_{d6}; δ ppm): 1.32 (3H, t, J = 7); 1.65 (1H, dd, J = 3 and J = 13); 2.34 (1H, dd, J = 9 and J = 13); 3.30-3.40 (1H, m); 3.42 (2H, d, J = 6); 3.53-3.65 (1H, m); 3.79 (1H, t, J = 10); 3.93 (1H, dd, J = 4 and J = 11); 4.45-4.65 (3H, m); 4.87 (1H, s); 4.97 (1H, t, J = 6); 5.28 (2H, s); 7.28-7.42 (3H, m); 7.45-7.55 (3H, m); 8.49 (1H, s).

MS (ESI): 468.8.

### 19.ix. (13S,16S)-1-ethyl-7-fluoro-16-hydroxy-16-methanesulfonyloxymethyl-4-oxo-1,4,13,15,16,17-hexahydro-12H-11-oxa-1,14-diaza-cyclopenta[a]phenanthrene-3-carboxylic acid benzyl ester:

This compound was obtained as a yellowish solid in 60.5% yield, starting from intermediate 19.viii (265 mg), TEA (0.16 ml) and methanesulfonic anhydride (118 mg) and using the procedure of Example 5, step 5.4.

¹H NMR (DMSO_{d6}; δ ppm): 1.32 (3H, t, J = 7); 1.82 (1H, dd, J = 4 and J = 14); 2.34 (1H, dd, J = 9 and J = 14); 3.24 (3H, s); 3.47 (1H, dd, J = 4 and J = 11); 3.59-3.66 (1H, m); 3.77 (1H, t, J = 10); 3.93 (1H, dd, J = 3 and J = 11); 4.29 (2H, s); 4.50-4.65 (3H, m); 5.28 (2H, s); 5.51 (1H, s); 7.26-7.42 (3H, m); 7.47-7.58 (3H, m); 8.50 (1H, s).

MS (ESI): 546.7.

### 19.x. (13S,16S)-1-ethyl-7-fluoro-16-[2-fluoro-4-((R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid benzyl ester:

This compound was obtained as a beige solid in 89.5% yield, starting from intermediates 19.ix (145 mg) and 1.A.ii (74 mg) and using the procedure of Example 1, step 1.A.viii.

¹H NMR (DMSO_{d6}; δ ppm): 1.33 (3H, t, J = 7); 1.86 (1H, dd, J = 4 and J = 14); 2.44 (1H, dd, J = 9 and J = 14); 3.53 (1H, dd, J = 5 and J = 11); 3.60-3.72 (1H, m); 3.73-3.91 (2H, m); 4.04 (1H, dd, J=3 and J= 11); 4.12 (2H, s); 4.22 (1H, t, J=9); 4.49-4.64 (3H, m); 4.83 (2H, d, J = 5); 5.08-5.15 (1H, m); 5.28 (2H, s); 5.37 (1H, s); 7.11-7.18 (1H, m); 7.20-7.45 (4H, m); 7.46-7.60 (4H, m); 7.77 (1H, d, J= 1); 8.17 (1H, d, J = 1); 8.51 (1H, s).

MS (ESI): 728.8.

### 19.xi. (13S,16S)-1-ethyl-7-fluoro-16-[2-fluoro-4-((R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diaza-cyclopenta[a]phenanthrene-3-carboxylic acid:

The compound was obtained as a colourless solid in 54% yield, starting from intermediate 19.x (70 mg) and using the procedure of Example 1, step 1.B.xiii.

¹H NMR (DMSO_{d6}; δ ppm): 1.38 (3H, t, J = 7); 1.89 (1H, dd, J = 4 and J = 14); 2.46 (1H, dd, J = 8 and J = 14); 3.63 (1H, dd, J = 5 and J = 11); 3.67-3.78 (1H, m); 3.79-3.92 (2H, m); 4.08 (1H, dd, J = 3 and J =11); 4.13 (2H, s); 4.22 (1H, t, J = 9); 4.65 (1H, dd, J = 3 and 10); 4.75 (2H, quint., J = 7); 4.84 (2H, d, J = 5); 5.08-5.17 (1H, m); 5.41 (1H, s); 7.11-7.19 (1H, m); 7.26 (1H, t, J = 9); 7.50 (1H, dd, J = 3 and J = 14); 7.66 (1H, d, J= 13); 7.77 (1H, d, J = 1); 8.18 (1H, d, J= 1); 8.78 (1H, s); 15.37 (1H, broad).

MS (ESI): 638.8.

### Example 20: (13S,16S)-1-cyclopropyl-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diazacyclopenta[a]phenanthrene-3-carboxylic acid:

### 20.i. 1-cyclopropyl-7-fluoro-8-methoxy-4-oxo-1,4-dihydro-quinoline-3-carboxylic acid:

A solution of 1-cyclopropyl-7-fluoro-1,4-dihydro-8-methoxy-4-oxo-3-quinolinecarboxylic acid ethyl ester (13 g; prepared according to WO 2004/013103) in AcOH/water/conc. H₂SO₄ (8/6/1; 170 ml) was refluxed overnight. The reaction mixture was cooled to 0°C and the crystals were collected by filtration, washed with water (70 ml) and dried affording 8.63 g (73% yield) of a colourless material.

¹H NMR (DMSO_{d6}; δ ppm): 1.21-1.27 (4H, m); 4.07-4.08 (3H, s); 4.26-4.31 (1H, s); 7.67-7.73 (1H, dd, J = 10.5 and J = 9); 8.19-8.24 (1H, dd, J = 9 and J = 6); 8.82 (1H, s); 14.84 (1H, s).

MS (ESI): 278.4.

### 20.ii. 1-cyclopropyl-7-fluoro-8-hydroxy-4-oxo-1,4-dihydro-quinoline-3-carboxylic acid:

A solution of intermediate 20.i (7.87g) in HBr (33% in AcOH; 100 ml) was stirred at 90°C for 60 h. The solvent was removed *in vacuo* and the residue was crystallized form MeCN affording 7.5 g (100% yield) of a colourless material.

¹H NMR (DMSO_{d6}; δ ppm): 1.02-1.20 (4H, m); 4.29-4.37 (1H, m); 7.51-7.57 (1H, dd, J = 10 and J = 9); 7.84-7.89 (1H, dd, J = 9 and J = 6); 8.71 (1H, s): 10.72-11.02 (2H, broad).

MS (ESI): 264.2.

### 20.iii. 1-cyclopropyl-7-fluoro-8-hydroxy-4-oxo-1,4-dihydro-quinoline-3-carboxylic acid ethyl ester:

A solution of intermediate 20.ii (6.5 g) in 9*N* HCl in EtOH (50 ml) was stirred at 60°C for 3 days. The reaction mixture was treated with triethyl orthoformate (20.5 ml) and the reaction mixture was further stirred at 60°C for 24 h. The solvents were evaporated *in vacuo* and the residue was purified by chromatography over SiO₂ using DCM/MeOH 95:5 as eluent, affording 3.90 g (54.3% yield) of a white solid.

¹H NMR (DMSO_{d6}; δ ppm): 0.99-1.15 (4H, m); 1.24-1.29 (3H, t, J = 7); 4.12-4.24 (3H, m); 7.30-7.36 (1H, dd, J = 10 and J = 9); 7.69-7.74 (1H, dd, J=9 and J = 6); 8.48 (1H, s); 10.4 (1H, s).

### 20.iv. 8-((2S)-1-tert-butoxycarbonyl-4-methylene-pyrrolidin-2-ylmethoxy)-1-cyclopropyl-7-fluoro-4-oxo-1,4-dihydro-quinoline-3-carboxylic acid ethyl ester:

This compound was obtained as a colourless material in 83.5% yield, starting from intermediate 20.iii (1.0 g), (2*S*)-2-(hydroxymethyl)-4-methylene-1-pyrrolidinecarboxylic acid *tert*-butyl ester (732 mg), PPh₃ (1.35 g) and DIAD (1.041 g) and using the procedure of Example 1, step 1.B.v.

¹H NMR (DMSO_{d6}; δ ppm): 1.035-1.23 (4H, m); 1.25-1.3 (3H, t, J = 7); 1.38 (9H, s); 2.66-2.72 (1H, d, J = 16); 2.84-2.91 (1H, m); 3.85-4.07 (5H, m); 4.18-4.26 (3H, m); 5.05 (2H, s); 7.39-7.45 (1H, dd, J = 10 and J = 9); 7.97-8.02 (1H, dd, J = 9 and J = 6); 8.50 (1H, s).

MS (ESI): 487.5.

### 20.v. (2S)-8-(1-tert-butoxycarbonyl-4-methylene-pyrrolidin-2-ylmethoxy)-1-cyclopropyl-7-fluoro-4-oxo-1,4-dihydro-quinoline-3-carboxylic acid:

This compound was obtained as a colourless material in 75.8% yield, starting from intermediate 20.iv (500 mg) and LiOH (108 mg) and using the procedure of Example 1, step 1.B.vi.

¹H NMR (DMSO_{d6}; δ ppm): 1.11-1.23 (4H, m); 1.39 (9H, s); 2.67-2.72 (1H, d, J = 16); 2.85-2.91 (1H, m); 3.78-3.83 (1H, m); 3.93-4.07 (3H, m); 4.16 (1H, m); 4.28 (1H, m); 5.06 (2H, s); 7.60-7.66 (1H, dd, J = 10 and J = 9); 8.14-8.19 (1H, dd, J = 9 and J = 6); 8.76 (1H, s); 14.73 (1H,s).

MS (ESI): 458.8.

### 20.vi. (2S)-8-(1-tert-butoxycarbonyl-4-ntethylene-pyrrolidin-2-ylmethoxy)-1-cyclopropyl-7-fluoro-4-oxo-1,4-dihydro-quinoline-3-carboxylic acid benzyl ester:

This compound was obtained as a colourless material in 68.5% yield, starting from intermediate 20.v (111 mg) and BnBr (46 mg) and using the procedure of Example 1, step 1.B.vii.

¹H NMR (DMSO_{d6}; δ ppm): 1.03-1.15 (4H, m); 1.38 (9H, s); 2.66-2.71 (1H, d, J= 15); 2.86-2.89 (1H, m); 3.77-3.83 (1H, broad); 3.91-4.02 (3H, m); 4.25 (1H, broad); 5.05 (2H, s); 5.28 (2H, s); 7.30-7.50 (6H, m); 7.99-8.04 (1H, dd, J = 9 and J = 6); 8.56 (1H, s).

MS (ESI): 548.6.

### 20.vii. (2S,4S)-8-(1-tert-butoxycarbonyl-4-hydroxy-4-hydroxymethyl-pyrrolidin-2-ylmethoxy)-1-cyclopropyl-7-fluoro-4-oxo-1,4-dihydro-quinoline-3-carboxylic acid benzyl ester:

The compound was obtained as a colourless material in 80.5% yield, starting from intermediate 20.vi (289 mg) and AD-mix α and using the procedure of Example 1, step 1.B.viii.

¹H NMR (DMSO_{d6}; δ ppm): 0.83-0.95 (4H, m); 1.11-1.26 (9H); 1.91-1.99 (1H, m); 2.18-2.20 (1H, m); 3.09-3.13 (1H, m); 3.41-3.45 (1H, d, J = 11); 4.06-4.21 (3H, m); 4.31-4.37 (1H, t, J = 9); 4.81 (1H, s); 4.88-4.92 (1H, t, J = 5); 5.29 (2H, s); 7.30-7.50 (6H, m); 7.98-8.03 (1H, dd, J = 9 and J = 6); 8.57 (1H, s).

MS (ESI): 583.0.

### 20.viii. (2S,4S)-2-(3-benzyloxycarbonyl-1-cyclopropyl-7-fluoro-4-oxo-1,4-dihydro-quinolin-8-yloxymethyl)-4-hydroxy-4-hydroxymethyl-pyrrolidinium hydrochloride:

This compound was obtained as a colourless material in 55.3% yield, starting from intermediate 20.vii (289 mg) and 5*M* HCl in dioxane and using the procedure of Example 1, step 1.B.ix.

MS (ESI): 482.9.

### 20.ix. (13S,16S)-1-cyclopropyl-16-hydroxy-16-hydroxymethyl-4-oxo-1,4,13,15,16,17-hexahydro-12H-11-oxa-1,14-diaza-cyclopenta[a]phenanthrene-3-carboxylic acid benzyl ester:

This compound was obtained as a colourless material in 99.5% yield, starting from intermediate 20.viii (91 mg) and DIPEA (68 mg) and using the procedure of Example 1, step 1.B.x.

MS (ESI): 463.0.

### 20.x. (13S,16S)-1-cyclopropyl-16-hydroxy-16-methanesulfonyloxymethyl-4-oxo-1,4,13,15,16,17-hexahydro-12H-11-oxa-1,14-diaza-cyclopenta[a]phenanthrene-3-carboxylic acid benzyl ester:

This compound was obtained in the form of colourless crystals in 32% yield, starting from intermediate 20.ix. (81 mg), mesyl chloride (22 mg) and pyridine (0.4 ml) and using the procedure of Example 1, step 1.B.xi.

¹H NMR (DMSO_{d6}; δ ppm): 0.84-1.07 (4H, m); 1.74-1.81 (1H, dd, J = 8 and J = 13); 2.28-2.35 (1H, dd, J = 7 and J=13); 3.25 (3H, s); 3.37-3.41 (1H, d, J = 10); 3.47-3.51 (1H, m); 3.65-3.77 (2H, m); 4.02-4.10 (1H, m); 4.31 (2H, s); 4.55-4.59 (1H, dd, J = 3 and J = 10); 5.25 (2H, s); 5.63 (1H, s); 6.78-6.81 (1H, d, J = 9); 7.29-7.50 (5H, m); 7.71-7.74 (1H, d, J = 9); 8.43 (1H, s).

MS (ESI): 540.8.

### 20.xi. (13S,16S)-1-cyclopropyl-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diazacyclopenta[a]phenanthrene-3-carboxylic acid benzyl ester:

This compound (17 mg) was obtained as a yellow solid in 41.7% yield, starting from intermediate 20.x (30 mg) and intermediate 1.A.ii (17 mg) and using the procedure of Example 1, step 1.A.viii.

MS (ESI): 722.8.

### 20.xii. (13S,16S)-1-cyclopropyl-16-[2-fluoro-4-((5R)-2-oxo-5-[1,2,3]triazol-1-ylmethyl-oxazolidin-3-yl)-phenoxymethyl]-16-hydroxy-4-oxo-1,4,13,15,16,17-hexahydro-12H-11-oxa-1,14-diazacyclopenta[a]phenanthrene-3-carboxylic acid:

The title compound (13 mg) was obtained as a yellow solid in 98.6% yield, starting from intermediate 20.xi (15 mg) and using the procedure of Example 5, step 5.ii.

¹H NMR (DMSO_{d6}; δ ppm): 0.71-1.55 (4H, m); 1.81-1.87 (1H, dd, J = 8 and J = 12); 3.51-3.56 (2H, m); 3.79-3.89 (2H, m); 4.16-4.24 (4H, m); 4.65-4.67 (1H, dd, J= 8 and J = 4); 4.75-4.84 (2H, d, J = 5); 5.11-5.13 (1H, m); 5.51 (1H, s); 6.99-7.01 (1H, d, J = 9); 7.16 (1H, m); 7.25-7.29 (1H, t, J = 8); 7.47-7.51 (1H, dd, J= 13 and J = 2); 7.76 (1H, s); 7.84-7.86 (1H, d, J = 9); 8.16 (1H, s); 8.58 (1H, s); 15.56 (1H, s).

MS (ESI): 632.7.

### Example 21: (13S,16S)-1-cyaopropyl-7-fluoro-16-[2-fluoro-4-((5R)-2-oxo-5-[1,2,3]triazol-1-ylmethyl-oxazohdin-3-yl)-phenoxymethyl]-4-oxo-1,4,13,15,16,17-hexahydro-12H-11-oxa-1,14-diazacyclopenta[a]phenanthrene-3-carboxylic acid:

### 21.i. (S)-2-(2,2-dimethyl-propionyloxymethyl)-4-oxo-pyrrolidine-1-carboxylic acid tert-butyl ester:

A colourless solution of (*2S*,*4R*)-4-hydroxy-2-hydroxymethyl-pyrrolidine-1-carboxylic acid *tert*-butyl ester (660 g) in DCM (6.61) was cooled down to 0°C and treated with TEA (510 ml) and, dropwise, with pivaloyl chloride (378 ml). The reaction mixture was stirred for 24 h at rt. The reaction mixture was cooled to -8 °C and treated with DIPEA (1506 ml). A solution of pyridine sulfur trioxide complex (1036 g) in DMSO (4 1) was added dropwise over 90 min. The reaction mixture was quenched with the addition of 41 of H₂O. The org. layer was concentrated *in vacuo* and the aqueous layer was extracted with Et₂O/Hex (1:1; 2 x 11). The combined org. layers were concentrated in *vacuo* and the residue was dissolved in Et₂O/Hex (1:1; 41). After washing with water (3 x 11) and brine (11), drying over MgSO₄, filtering and concentrating, a beige solid was obtained which was crystallized from Hex (873 g), affording 674.8 g of a colourless powder (74.2% yield).

¹H NMR (DMSO_{d6}; δ ppm): 1.08 (9H, s); 1.42 (9H, s); 2.28-2.34 (1H, d, J = 18); 2.89-3.10 (1H, m); 3.52 (1H, m); 3.82-3.89 (1H, d, J = 18); 4.03-4.07 (1H, m); 4.22-4.25 (1H, m); 4.47 (1H, m).

MS (ESI): 300.5.

### 21.ii. (S)-2-(2,2-dimethyl-propionyloxymethyl)-4-methylene-pyrrolidine-1-carboxylic acid tert-butyl ester:

*t*-BuOK (56.2 g) was added in one portion to a white suspension of methyl triphenylphosphonium bromide (178.9 g) in THF (600 ml) at rt under nitrogen. The resulting yellow suspension was stirred at rt for 1 h. A solution of intermediate 21.i (60 g) in THF (150 ml) was added dropwise at such a rate that the temperature stayed below 25°C. The reaction mixture was stirred at rt for 1 h and quenched by the addition of water (20 ml). The reaction mixture was concentrated to a volume of 50 ml and diluted with Et₂O (100 ml) and Hept (250 ml). The mixture was stirred at 0°C for 2 h, filtered. The filtrate was washed with MeOH/water (2:1; 3 x 200 ml) and brine, dried over MgSO₄ and filtered. The filtrate was concentrated *in vacuo* and purified by filtration over SiO₂ using Hept and Hept/EA (97:3 to 95:5) to give a yellow liquid (47.75 g, 80% yield).

¹H NMR (DMSO_{d6}; δ ppm): 1.12 (9H, s); 1.40 (9H,s); 2.32-2.43 (1H, t, J = 15); 2.72-2.84 (1H, m); 3.75-3.84 (1H, m); 3.95-4.03 (4H, m); 4.99 (2H, s).

MS (ESI): 298.2.

### 21.iii. (2S,4RS)-2-(2,2-dimethyl-propionyloxymethyl)-4-hydroxymethyl-pyrrolidine-1-carboxylic acid tert-butyl ester:

A 0.5M solution of 9-BBN in THF (16 ml) was added to a solution of intermediate 21.ii (598 mg) in THE (4 ml) at rt under nitrogen. The orange mixture was stirred at rt for 2 h. MeOH (5 ml) and pH 7.2 phosphate buffer (5 ml) were added dropwise at rt, followed by aq. H₂O₂ (35%; 11.7*M*; 1.5 ml). The reaction mixture was stirred at rt for 16 h. Sat. aq. Na₂S₂O₃ and EA were added and the mixture was vigorously stirred for 15 min. The org. layer was separated and washed with sat. aq. NH₄Cl, sat. aq. NaHCO₃, water, brine and dried over MgSO₄. The filtrate was filtered, concentrated *in vacuo* to give a colourless liquid (987 mg) and purified by chromatography over SiO₂ (Hex/EA 8:2 to 6:4). A colourless liquid (503 mg) was obtained.

¹H NMR (DMSO_{d6}; δ ppm): 1.12-1.16 (9H, m); 1.39 (9H, s); 1.35-1.90 (1H, m); 2.00-2.50 (2H, m); 2.75-3.15 (1H, m); 3.22-3.50 (2H, m); 3.55-3.70 (1H, m); 3.80-4.30 (3H, m); 4.60-4.70 (1H, m).

MS (ESI): 338.1 (M+Na)⁺.

### 21.iv. (2S,4RS)-4-(tert-butyl-dimethyl-silanyloxymethyl)-2-(2,2-dimethyl-propionyloxymethyl)-pyrrolidine-1-carboxylic acid tert-butyl ester:

Imidazole (162 mg) was added to a colourless solution of intermediate 21.iii (506 mg) in DCM (5 ml) at 0 °C. A solution of TBDMSCl (300 mg) in DCM (1 ml) was added and the mixture stirred at rt for 16 h. The reaction mixture was diluted with DCM,

washed with water and brine, dried over MgSO₄, filtered and concentrated *in vacuo* to give a yellow oil. After chromatography over SiO₂ (Hex/EA 95:5), a yellowish oil (567 mg, 83% yield) was obtained.

¹H NMR (DMSO_{d6}; δ ppm): 0.02-0.06 (6H, m); 0.84-0.88 (9H, m); 1.12-1.16 (9H, m); 1.39 (9H, s); 1.45-1.90 (1H, m); 2.00-2.50 (2H, m); 2.75-3.20 (1H, m); 3.45-3.65 (3H, m); 3.85-4.30 (3H, m).

MS (ESI): 429.1.

### 21.v. (2S,4RS)4-(tert-butyl-dimethyl-silanyloxymethyl)-2-hydroxymethyl-pyrrolidine-1-carboxylic acid tert-butyl ester:

A solution of intermediate 21.iv (550 mg) in MeOH (5 ml) was treated at rt with NaOMe (70 mg). The reaction mixture was stirred at rt for 60 h, then quenched with sat. aq. NH₄Cl. The mixture was diluted with EA and the aq. layer was back-extracted with EA. The combined org. layers were dried over MgSO₄, filtered and concentrated *in vacuo* to give a yellow oil (470 mg). After chromatography over SiO₂ (Hex/EA 8:2), a yellow oil (405 mg, 91.6% yield) was obtained.

¹H NMR (DMSO_{d6}; δ ppm): 0.02-0.06 (6H, m); 0.84-0.88 (9H, m); 1.39 (9H, s); 1.40-1.62 (1H, m); 1.80-2.05 (1H, m); 2.05-2.40 (1H, m); 2.65-3.05 (1H, m); 3.15-3.70 (6H, m); 4.55-4.70 (1H, m).

### 21.vi. 8-[(2S,4RS)-1-tert-butoxycarbonyl-4-(tert-butyl-dimethyl-silanyloxymethyl)-pyrrolidin-2-ylmethoxyl-1-cyclopropyl-6,7-difluoro-4-oxo-1,4-dihydro-quinoline-3-carboxylic acid ethyl ester:

A solution of intermediate 21.v (380 mg) and intermediate 1.B.iv (310 mg) in dry THF (4 ml) was treated with PPh₃ (393 mg). The white suspension was treated dropwise over 2 h with DIAD (0.32 ml). The clear orange solution was further stirred at rt for 16 h and the reaction mixture was then concentrated *in vacuo.* Purification of the residue by chromatography over SiO₂ (Hex/EA 8:2 to 6:4) gave a white foam (365 mg, 57% yield).

¹H NMR (DMSO_{d6}; δ ppm): 0.02-0.06 (6H, m); 0.84-0.88 (9H, m); 0.92-1.10 (4H, m); 1.27 (3H, t, J = 7); 1.37 (9H, s); 1.75-1.95 (1H, m); 2.02-2.20 (2H, m); 2.75-3.20 (1H, m); 3.45-3.65 (3H, m); 3.90-4.25 (4H, m); 4.22 (2H, q, J = 7); 7.78-7.92 (1H, m); 8.52 (1H, s).

MS (ESI): 636.8.

### 21.vii. 8-[(2S,4RS)-1-tert-butoxycarbonyl-4-(tert-butyl-dimethyl-silanyloxymethyl)-pyrrolidin-2-ylmethoxy]-1-cyclopropyl-6,7-difluoro-4-oxo-1,4-dihydro-quinoline-3-carboxylic acid:

A solution of intermediate 21.vi (360 mg) in dioxane/water (9:1; 4 ml) was treated at rt with LiOH (75 mg) for 16 h. The reaction mixture was concentrated *in vacuo.* The residue was suspended in water (5 ml) and treated at 0°C with 1*M* HCl (5 ml). The suspension was stirred for 4 h at 0°C and filtered. The white solid was dried to give a white powder (272 mg; 79% yield).

¹H NMR (DMSO_{d6}; δ ppm): 0.02-0.05 (6H, m); 0.84-0.88 (9H, m); 1.00-1.20 (4H, m); 1.37 (9H, s); 1.78-1.97 (1H, m); 2.02-2.20 (2H, m); 2.80-3.25 (1H, m); 3.48-3.65 (3H, m); 4.02-4.38 (4H, m); 8.03 (1H, t, J = 9); 8.78 (1H, s); 14.52 (1H, s).

MS (ESI): 609.0.

### 21.viii. 8-[(2S,4RS)-1-tert-butoxycarbonyl-4-(tert-butyl-dimethyl-silanyloxymethyl)-pyrrolidin-2-ylmethoxy]-1-cyclopropyl-6,7-difluoro-4-oxo-1,4-dihydro-quinoline-3-carboxylic acid benzyl ester:

K₂CO₃ (90 mg) and BnBr (0.06 ml) were added to a solution of intermediate 21.vii (265 mg) in DMF (2 ml). The suspension was stirred at 60 °C for 90 min. The reaction mixture was concentrated *in vacuo* and the residue was diluted in DCM. The org. layer was washed with water and brine, dried over MgSO₄, filtered and concentrated in *vacuo.* The resulting oil was purified by chromatography over SiO₂ (Hex/EA 75:25 to 65:35) to give a white foam (241 mg, 79% yield).

¹H NMR (DMSO_{d6}; δ ppm): 0.02-0.05 (6H, m); 0.84-0.88 (9H, m); 0.98-1.14 (4H, m); 1.37 (9H, s); 1.77-1.95 (1H, m); 2.02-2.35 (2H, m); 2.75-3.20 (1H, m); 3.45-3.70 (3H, m); 3.90-4.30 (4H, m); 5.28 (2H,s); 7.26-7.40 (3H, m); 7.41-7.50 (2H, m); 7.80-7.95 (1H, m); 8.56 (1H, s).

MS (ESI): 698.6.

### 21.ix. 8-[(2S,4RS)-1-tert-butoxycarbonyl-4-hydroxymethyl-pyrrolidin-2-ylmethoxy)-1-cyclopropyl-6,7-difluoro-4-oxo-1,4-dihydro-quinoline-3-carboxylic acid benzyl ester:

A solution of intermediate 21.viii (235 mg) in dioxane (3 ml) was treated at rt with 1*M* HCl in dioxane (0.7 ml). The reaction mixture was stirred at rt for 4 h, diluted with Hex (3 ml) and further stirred at rt for 20 min. The solid was filtered and dried to give a white solid (105 mg; 49% yield).

¹H NMR (DMSO_{d6}; δ ppm): 1.00-1.22 (4H, m); 1.39 (9H, s); 1.78-1.95 (1H, m); 2.02-2.38 (2H, m); 2.85-3.15 (1H, m); 3.30-3.75 (3H, m); 3.98-4.40 (4H, m); 4.73 (1H, broad); 5.33 (2H,s); 7.32-7.48 (3H, m); 7.49-7.58 (2H, m); 7.85-7.98 (1H, m); 8.61 (1H, s).

MS (ESI): 584.8.

### 21.x. 1-cyclopropyl-6,7-difluoro-8-((2S,4RS)-4-hydroxymethyl-pyrrolidin-2-ylmethoxy)-4-oxo-1,4-dihydro-quinoline-3-carboxylic acid benzyl ester hydrochloride:

A solution of intermediate 21.ix (100 mg) in dioxane (1 ml) was treated at rt with 1*M* HCl in dioxane (0.4 ml) and stirred at rt for 32 h. The reaction mixture was concentrated *in vacuo* and the residue taken up in EA (5 ml). The heterogeneous mixture was stirred at rt for 1 h and the precipitate was filtered and dried to give a white solid (77 mg; 86.4% yield).

¹H NMR (DMSO_{d6}; δ ppm; as 1:1 mixture benzyl ester/acid): 0.90-1.18 (4H, m); 1.20-1.82 (1H, m); 1.85-2.32 (1H, m); 2.95-3.15 (1H, m); 3.18-3.60 (3H, m); 3.95-4.23 (2H, m); 4.25-4.63 (3H, m); 4.85-5.08 (1H, broad); 5.29 (2H,s); 7.25-7.55 (5H, m); 7.81-7.95 (1H, m); 8.58 (1H, s); 9.20-9.50 (1H, broad); 9.60-10.00 (1H, broad).

MS (ESI): 485.1 (M+H-HCl)⁺

### 21.xi. (13S,16RS)-1-cyclopropyl-7-fluoro-16-hydroxymethyl-4-oxo-1,4,13,15,16,17-hexahydro-12H-11-oxa-1,14-diaza-cyclopenta[a]phenanthrene-3-carboxylic acid benzyl ester:

A suspension of intermediate 21.x. (in mixture with its corresponding free acid; 70 mg) in MeCN (1.5 ml) was treated with NaHCO₃ (23 mg) and DIPEA (0.05 ml) and stirred at 80 °C for 2 h. The reaction mixture was concentrated *in vacuo* and the residue was diluted in DCM, washed with water, dried over MgSO₄, filtered and concentrated in *vacuo.* The residue was purified by chromatography over SiO₂ (DCM/MeOH 98:2 to 90:10) to give a yellow solid (35 mg, 56% yield).

¹H NMR (DMSO_{d6}; δ ppm): 0.95-1.30 (4H, m); 1.55-2.45 (2H, m); 3.00-3.75 (6H, m); 3.95-4.10 (2H, m); 4.45-4.60 (1H, m); 4.65-4.80 (1H, m); 5.25 (2H,s); 7.25-7.50 (6H, m); 8.43 (1H, 2 s).

MS (ESI): 464.7.

### 21.xii. (13S,16RS)-1-cyclopropyl-7-fluoro-16-[2-fluoro-4-((5R)-2-oxo-5-[1,2,3]triazol-1-ylmethyl-oxazolidin-3-yl)-phenoxymethyl]-4-oxo-1,4,13,15,16,17-hexahydro-12H-11-oxa-1,14-diaza-cyclopenta[a]phenanthrene-3-carboxylic acid benzyl ester.

A suspension of intermediate 21.xi (15.7 mg) and intermediate 1.A.ii (11.3 mg) in dry dioxane (0.6 ml) was treated at rt under nitrogen with PPh₃ (28 mg) and DIAD (20 ml). The suspension was stirred at rt for 12 h at 50°C. The reaction mixture was filtered and the precipitate washed with dioxane (2 ml). The solid was purified by repeated chromatography over SiO₂ (DCM/MeOH, 97:3 to 90:10), affording a solid (17.4 mg; 28% yield).

MS (ESI): 725.48.

### 21.xiii. (13S,16RS)-1-cyclopropyl-7-fluoro-16-[2-fluoro-4-((5R)-2-oxo-5-[1,2,3]triazol-1-ylmethyl-oxazolidin-3-yl)-phenoxymethyl]4-oxo-1,4,13,15,16,17-hexahydro-12H-11-oxa-1,14-diaza-cyclopenta[a]phenanthrene-3-carboxylic acid:

A solution of intermediate 21.xii (17 mg) in DMA (400 ml) was hydrogenated over 10% Pd/C (10 mg) for 12 h. The catalyst was removed by filtration and the filtrate evaporated under reduced pressure. The residue was crystallized twice from MeOH (0.4 ml) affording a brown solid, which was dried under HV (1.6 mg).

MS (ESI): 635.5.

### Example 22: (13S,16S)-1-cyclopropyl-7-fluoro-16-[2-fluoro-4-((5R)-2-oxo-5-[1,2,3]triazol-1-ylmethyl-oxazolidin-3-yl)-phenoxymethyl]-4-oxo-16-phosphonooxy-1,4,13,15,16,17-hexahydro-12H-11-oxa-1,14-diaza-cyclopenta[a]phenanthrene-3-carboxylic acid:

### 22.i. 16-(bis-benzyloxy-phosphoryloxy)-1-cyclopropyl-7-fluoro-16-[2-fluoro-4-(2-oxo-5-[1,2,3]triazol-1-ylmethyl-oxazolidin-3-yl)-phenoxymethyl]4-oxo-1,4,13,15,16,17-hexahydro-12H-11-oxa-1,14-diaza-cyclopenta[a]phenanthrene-3-carboxylic acid:

A suspension of intermediate 1.A.ix (24 g) in DCM (300 ml) was sequentially treated with a solution of tetrazole in MeCN (0.45M; 164 ml) and dibenzyl *N*,*N*-diisopropylphosphoramidite (18 ml). The reaction was stirred at rt for 1 h. The solution was treated with *tert*-butyl hydroperoxide (70% in water; 16 ml) and the reaction was stirred for 1 h. The org. solvents were evaporated under reduced pressure and the residue was diluted in DCM (300 ml). The org layer was washed with water and brine, dried over MgSO₄, filtered and the filtrate evaporated *in vacuo* affording 33.5 g (99.5% yield) of yellow material.

¹H NMR (DMSO_{d6}; δ ppm): 1.0-1.1 (4H, m); 2.35 (1H, dd, J = 5 and J = 14.3); 2.65-2.73 (1H, m); 3.59 (1H, t, J = 10); 3.76-3.83 (1H, m); 3.87 (1H, dd, J = 6 and J= 9.5); 4.11-4.26 (1H, m); 4.48-4.62 (2H, m); 4.81-4.97 (2H, m); 4.98 (1H, d, J = 8); 5.0-5.16 (1H, m); 5.76 (1H, s); 7.0-4.75 (10H, m); 7.46 (1H, dd, J = 3 and J = 13.5); 7.60 (1H, d, J = 12.5); 7.77 (1H, d, J = 1); 8.16 (1H, d, J = 1); 8.61 (1H, s); 15.25 (1H,s).

MS (ESI): 910.9.

### 22.ii. (13S,16S)-1-cyclopropyl-7-fluoro-16-[2-fluoro-4-((5R)-2-oxo-5-[1,2,3]triazol-1-ylmethyl-oxazolidin-3-yl)-phenoxymethyl]-4-oxo-16-phosphonooxy-1,4,13,15,16,17-hexahydro-12H-11-oxa-1,14-diaza-cyclopenta[a]phenanthrene-3-carboxylic acid:

### 22.ii.a. Acidic deprotection:

A solution of intermediate 22.i. (34 g) in AcOH (150 mL) was treated with HBr (33% in AcOH; 62 ml) and stirred at rt for 1 h. The solvent was removed under reduced pressure and the residue was stirred in water. The solid was collected by filtration and washed with water (300 ml). The solid was diluted with DCM/MeOH (500 ml; 7/3) and the DCM was evaporated. The resulting slurry was diluted with MeOH (100 ml), and stirred at rt for 15 min. The solid was collected by filtration and dried to afford 23.5 g (89.3% yield) of a yellow solid.

¹H NMR (DMSO_{d6}; δ ppm): 1.01-1.16 (4H, m); 2.35 (1H, dd, J = 3.5 and J = 13.5); 2.62 (1H, dd, J = 6 and J = 14); 3.76 (2H, d, J = 5.5); 3.88 (1H, dd, J = 6 and J = 9.5); 4.18-4.26 (4H, m); 4.43 (2H, s); 4.65 (1H, d, J = 6.5); 4.84 (2H, d, J = 5); 5.09-5.20 (1H, m); 7.16 (1H, dd, J = 1.5 and J = 9); 7.24 (1H, t, J = 9); 7.5 (1H, dd, J = 2.5 and J = 13.5); 7.59 (1H, d, J = 12.5); 7.77 (1H, d, J = 1); 8.17 (1H, d, J = 1); 8.61 (1H, s); 15.25 (1H,s).

MS (ESI): 730.5.

### 22.ii.b. Deprotection by hydrogenation:

A suspension of intermediate 22.i. (441 mg) in THF/MeOH (1:1; 10 ml) was was treated with a solution of sodium acetate (164 mg) in water (5 ml) and hydrogenated over 10% Pd/C (10 mg) for 3 h. The catalyst was filtered off and the org. solvents were removed under reduced pressure. The aq. residue was treated with 4N HCl to reach pH 3. The solid was collected by filtration and washed with water (20 ml). The solid was stirred in EtOH (20 ml) for 30 min. The resulting solid was collected by filtration affording 656 mg (80% yield) of yellow material.

¹H NMR and MS data identical to those for a sample obtained according to 22.ii.a.

### Example 23: (13R,16S)-1-cyclopropyl-7-fluoro-16-[2-fluoro-4-((5R)-2-oxo-5-[1,2,3]triazol-1-ylmethyl-oxazolidin-3-yl)-phenoxymethyl]-4-oxo-16-phosphonooxy-1,4,13,15,16,17-hexahydro-12H-11-oxa-1,14-diaza-cyclopenta[a]phenanthrene-3-carboxylic acid:

### 23.i. (13R,16S)-16-(bis-benzyloxy-phosphoryloxy)-1-cyclopropyl-7-fluoro-16-[2fluoro-4-((5R)-2-oxo-5-[1,2,3]triazol-1-ylmethyl-oxazolidin-3-yl)-phenoxymethyl]-4-oxo-1,4,13,15,16,17-hexahydro-12H-11-oxa-1,14-diaza-cyclopenta[a]phenanthrene-3-carboxylic acid:

This compound was obtained as a yellow solid in 63.1% yield, starting from intermediate 6.ix (100 mg), dibenzyl *N*,*N*-diisopropylphosphoramidite (77.7 mg) and *tert*-butyl hydroperoxide (0.067 ml) and using the procedure of Example 22, step 22.i.

MS (ESI): 911.72.

### 23.ii. (13R,16S)-1-cyclopropyl-7-fluoro-16-[2-fluoro-4-((5R)-2-oxo-5-[1,2,3]triazol-1-ylmethyl-oxazolidin-3-yl)-phenoxymethyl]-4-oxo-16-phosphonooxy-1,4,13,15,16,17-hexahydro-12H-11-oxa-1,14-diaza-cyclopenta[a]phenanthrene-3-carboxylic acid:

This compound was obtained as a yellow solid in 66.3% yield, starting from intermediate 23.i (88 mg) and HBr in AcOH (0.5 ml) and using the procedure of Example 22, step 22.ii.a.

¹H NMR (DMSO_{d6}; δ ppm): 0.98-1.20 (4H, m); 2.38 (1H, dd, J = 3.7 and 13.5); 2.59 (1H, dd, J = 7 and J = 13.5); 3.76 (2H, m); 3.85 (1H, dd, J = 6 and J = 9.5); 4.00-4.20 (4H, m); 4.47 (2H, s); 4.68 (1H, d, J = 8.5); 4.85 (2H, d, J = 5); 5.10-5.30 (1H, m); 7.15-7.34 (2H, m); 7.48 (1H, dd, J = 5 and J = 13.5); 7.60 (1H, d, J = 12.6); 7.77 (1H, s); 8.17 (1H, s); 8.63 (1H, s); 15.20 (1H, s broad).

MS (ESI): 730.7.

### Example 24: (13S,16R)-1-cyclopropyl-7-fluoro-16-[2-fluoro-4-((5R)-2-oxo-5-[1,2,3]triazol-1-ylmethyl-oxazolidin-3-yl)-phenoxymethyl]-4-oxo-16-phosphonooxy-1,4,13,15,16,17-hexahydro-12H-11-oxa-1,14-diaza-cyclopenta[a]phenanthrene-3-carboxylic acid:

### 24.i. (13S,16R)-16-(bis-benzyloxy-phosphoryloxy)-1-cyclopropyl-7-fluoro-16-(2-fluoro-4-((5R)-2-oxo-5-[1,2,3]triazol-1-ylmethyl-oxazolidin-3-yl)-phenoxymethyl)-4-oxo-1,4,13,15,16,17-hexahydro-12H-11-oxa-1,14-diaza-cyclopenta[a]phenanthrene-3-carboxylic acid:

This compound was obtained as a yellow solid in 65.2% yield, starting from intermediate 5.ii (100 mg), dibenzyl *N*,*N*-diisopropylphosphoramidite (77.7 mg) and *tert*-butyl hydroperoxide (0.067 ml) and using the procedure of Example 22, step 22.i.

MS (ESI): 910.8.

### 24.ii. (13S,16R)-1-cyclopropyl-7-fluoro-16-[2-fluoro-4-((5R)-2-oxo-5-[1,2,3]triazol-1-ylmethyl-oxazolidin-3-yl)-phenoxymethyl]-4-oxo-16-phosphonooxy-1,4,13,15,16,17-hexahydro-12H-11-oxa-1,14-diaza-cyclopenta[a]phenanthrene-3-carboxylic acid:

This compound was obtained as a yellow solid in 75.6% yield, starting from intermediate 24.i (440 mg) and using the procedure of Example 22, step 22.ii.b.

¹H NMR (DMSO_{d6}; δ ppm): 0.99-1.15 (4H, m); 1.78 (1H, t broad, J = 10.5); 2.13 (1H, dd, J = 6.5 and J = 13); 3.50 (1H, t, J = 10); 3.78 (1H, dd, J = 3.5 and J = 9.5); 3.87 (1H, dd, J = 6 and J = 9.5); 3.92-4.26 (6H, m); 4.76 (1H, dd; J = 3.5 and J = 10.5); 4.83 (2H, d, J = 5); 5.08-5.17 (1H, m); 5.53 (1H, s); 7.13 (1H, ddd, J = 1, J = 2.5 and J = 10); 7.25 (1H, t, J = 9); 7.50 (1H, dd, J = 2.5 and J = 13.5); 7.58 (1H, d, J = 13.5); 7.7 0 (1H, d, J= 1); 8.17 (1H, d, J= 1); 8.60 (1H, d, J= 1), 15.25 (1H, s).

MS (ESI): 650.8.

### Example 25: (13R,16S)-1-cyclopropyl-7-fluoro-16-[2-fluoro-4-((5S)-2-oxo-5-[1,2,3]triazol-1-ylmethyl-oxazolidin-3-yl)-phenoxymethyl]-4-oxo-16-phosphonooxy-1,4,13,15,16,17-hexahydro-12H-11-oxa-1,14-diaza-cyclopenta[a]phenanthrene-3-carboxylic acid:

### 25.i. (13R,16S)-16-(bis-benzyloxy-phosphoryloxy)-1-cyclopropyl-7-fluoro-16-[2-fluoro-4-((S)-2-oxo-5-[1,2,3]triazol-1-ylmethyl-oxazolidin-3-yl)phenoxymethyl]-4-oxo-1,4,13,15,16,17-hexahydro-12H-11-oxa-1,14-diaza-cyclopenta[a]phenanthrene-3-carboxylic acid:

This compound was obtained as a yellow solid in 35.7% yield, starting from intermediate 7.ii (200 mg), dibenzyl *N*,*N*-diisopropylphosphoramidite (169 mg) and *tert*-butyl hydroperoxide (0.134 ml) and using the procedure of Example 22, step 22.i.

MS (ESI): 910.6.

### 25.ii. (13R,16S)-1-cyclopropyl-7-fluoro-16-[2-fluoro-4-((5S)-2-oxo-5-[1,2,3]triazol-1-ylmethyl-oxazolidin-3-yl)-phenoxymethyl]-4-oxo-16-phosphonooxy-1,4,13,15,16,17-hexahydro-12H-11-oxa-1,14-diaza-cyclopenta[a]phenanthrene-3-carboxylic acid:

This compound was obtained as a yellow solid in 58% yield, starting from intermediate 25.i (87 mg) and HBr in AcOH (1 ml) and using the procedure of Example 22, step 22.ii.a.

¹H NMR (DMSO_{d6}; δ ppm): 0.98-1.20 (4H,m); 2.38 (1H, dd, J = 3 and J = 13.5); 2.61 (1H, dd, J = 6 and J = 13.5); 3.76 (2H, m); 3.90 (1H, dd, J = 6 and J = 9.5); 4.00-4.20 (4H, m); 4.33 (2H, s); 4.62 (1H, d, J = 8.5); 4.83 (2H, d, J = 5); 5.10-5.20 (1H, m); 7.15 (1H, dd, J = 2 and J = 9); 7.24 (1H, t, J = 9); 7.50 (1H, dd, J = 2.5 and J = 13.5); 7.60 (1H, d, J = 12.6); 7.77 (1H, s); 8.17 (1H, s); 8.66 (1H, s); 15.2 (1H, s broad).

MS (ESI): 730.7.

### Example 26: (13S,16S)-1-cyclopropyl-7-fluoro-16-[2-fluoro-4-((5S)-2-oxo-5-[1,2,3]triazol-1-ylmethyl-oxazolidin-3-yl)-phenoxymethyl]-4-oxo-16-phosphonooxy-1,4,13,15,16,17-hexahydro-12H-11-oxa-1,14-diaza-cyclopenta[a]phenanthrene-3-carboxylic acid:

### 26.i. (13S,16S)-16-(bis-benzyloxy-phosphoryloxy)-1-cyclopropyl-7-fluoro-16-[2-fluoro-4-((S)-2-oxo-5-[1,2,3]triazol-1-ylmethyl-oxazolidin-3-yl)-phenoxymethyl]-4-oxo-1,4,13,15,16,17-hexahydro-12H-11-oxa-1,14-diaza-cyclopenta[a]phenanthrene-3-carboxylic acid:

This compound was obtained as a yellow solid in 95.4% yield, starting from intermediate 8.ii (347 mg), dibenzyl *N*,*N*-diisopropylphosphoranmidite (269 mg) and *tert*-butyl hydroperoxide (0.23 ml) and using the procedure of Example 22, step 22.i.

MS (ESI): 910.7.

### 26.ii. (13S,16S)-1-cyclopropyl-7-fluoro-16-[2-fluoro-4-((5S)-2-oxo-5-[1,2,3]triazol-1-ylmethyl-oxazolidin-3-yl)-phenoxymethyl]-4-oxo-16-phosphonooxy-1,4,13,15,16,17-hexahydro-12H-11-oxa-1,14-diaza-cyclopenta[a]phenanthrene-3-carboxylic acid:

This compound was obtained as a yellow solid (250 mg, 75% yield), starting from intermediate 26.i (420 mg) and HBr in AcOH (33%; 1 ml) and using the procedure of Example 22, step 22.ii.a.

¹H NMR (DMSO_{d6}; δ ppm): 0.98-1.20 (4H, m); 2.38 (1H, dd, J = 3 and J = 13.5); 2.59 (1H, dd, J = 6.5 and J = 13.5); 3.76 (2H, m); 3.85 (1H, dd, J = 6 and J = 9.5); 4.10-4.30 (4H, m); 4.43 (2H, s); 4.68 (1H, d, J = 7); 4.85 (2H, d, J = 5); 5.10-5.3 (1H, m); 7.15 (1H, dd, J = 2 and J = 9); 7.24 (1H, t, J = 9); 7.48 (1H, dd, J = 2.6 and J = 13.5); 7.60 (1H, d, J = 12.6); 7.77 (1H, s); 8.17 (1H, s); 8.63 (1H, s); 15.2 (1H, s broad).

MS (ESI): 730.6.

### Example 27: (13S,16R)-1-cyclopropyl-7-fluoro-16-[2-fluoro-4-((5S)-2-oxo-5-[1,2,3]triazol-1-ylmethyl-oxazolidin-3-yl)-phenoxymethyl]-4-oxo-16-phosphonooxy-1,4,13,15,16,17-hexahydro-12H-11-oxa-1,14-diaza-cyclopenta[a]phenanthrene-3-carboxylic acid:

### 27.i. (13S,16R)-16-(bis-benzyloxy-phosphoryloxy)-1-cyclopropyl-7-fluoro-16-[2-fluoro4-((5S)-2-oxo-5-[1,2,3]triazol-1-ylmethyl-oxazolidin-3-yl)-phenoxymethyl]-4-oxo-1,4,13,15,16,17-hexahydro-12H-11-oxa-1,14-diaza-cyclopenta[a]phenanthrene-3-carboxylic acid:

This compound was obtained as a yellow solid in 58.3% yield, starting from intermediate 9.ii (300 mg), dibenzyl *N*,*N*-diisopropylphosphoramidite (253 mg) and *tert*-butyl hydroperoxide (0.20 ml) and using the procedure of Example 22, step 22.i.

MS (ESI): 910.6.

### 27.ii. (13S,16R)-1-cyclopropyl-7-fluoro-16-[2-fluoro-4-((5S)-2-oxo-5-[1,2,3]triazol-1-ylmethyl-oxazolidin-3-yl)-phenoxymethyl]-4-oxo-16-phosphonooxy-1,4,13,15,16,17-hexahydro-12H-11-oxa-1,14-diaza-cyclopenta[a]phenanthrene-3-carboxylic acid:

This compound was obtained as a yellow solid in 68% yield, starting from intermediate 27.i (220 mg) and HBr in AcOH (33%; 1 ml) and using the procedure of Example 22, step 22.ii.a.

¹H NMR (DMSO_{d6}; δ ppm): 0.98-1.15 (4H, m); 1.91 (1H, t, J= 13); 2.77 (1H, dd, J = 6.5 and J = 13.5); 3.56 (1H, t; J = 12.6); 3.72-3.90 (2H, m); 3.96-4.04 (1H, m); 4.23 (2H, d, J=9), 4.39 (1H, d, J = 10); 4.48 (1H, m); 4.55 (1H, d, J = 10); 4.75-4.90 (3H, m); 5.08-5.44 (1H, m); 7.15 (1H, dd, J = 1.5 and J = 9); 7.27 (1H, t, J=9); 7.52 (1H, dd, J=2.5 and J = 13.5); 7.60 (1H; d, J = 13); 7.77 (1H, s); 8.17 (1H, s); 8.66 (1H,s), 15.2 (1H, s broad).

MS (ESI): 730.6.

### BIOLOGICAL ASSAYS

### In vitro assay

### Experimental method:

These assays have been performed following the description given in "Methods for dilution Antimicrobial Susceptibility Tests for Bacteria that Grow Aerobically, 4th ed.; Approved standard: NCCLS Document M7-A4; National Committee for Clinical Laboratory Standards: Villanova, PA, USA, 1997". Minimal inhibitory concentrations (MICs; mg/l) were determined in cation-adjusted Mueller-Hinton Broth (BBL) by a microdilution method following NCCLS guidelines (National Committee for Clinical Laboratory Standards. Methods for Dilution Antimicrobial Susceptibility). The pH of the test medium was 7.2-7.3.

### Results:

All the above Examples were tested against several Gram positive and Gram negative bacteria. Typical antibacterial spectra are given in the table below (MIC in mg/l)

| Example No. | *S. aureus* A798 | *S Pneunoniae* 49619 | *M catarrhalis* A894 |
|---|---|---|---|
| 1 | ≤ 0.063 | ≤ 0.063 | ≤ 0.063 |
| 2 | ≤ 0.063 | ≤ 0.063 | ≤ 0.063 |
| 4 | ≤ 0.063 | ≤ 0.063 | 0.25 |

## Claims

1. A compound selected from the group consisting of a compound of the formula I 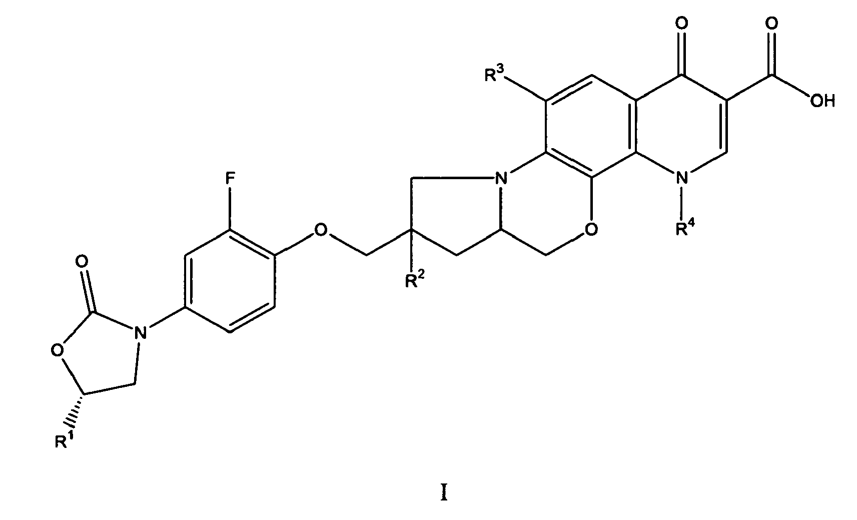 wherein
R¹ is heteroarylmethyl, heteroaryloxymethyl or heteroarylaminomethyl;
R² is H, OH, OSO₃H, OPO₃H₂, OCH₂OPO₃H₂, OCOCH₂CH₂COOH or OCOR⁶;
R³ is H or halogen;
R⁴ is (C₁-C₃) alkyl, (C₁-C₃) haloalkyl or cycloalkyl; and
R⁶ is the residue of a naturally occurring amino acid or of dimethylaminoglycine;
and a prodrug, a tautomer, an optically pure enantiomer, a mixture of enantiomers, a racemate, an optically pure diastereoisomer, a mixture of diastereoisomer, a diastereoisomeric racemate, mixtures of diastereoisomeric racemates, a meso-form, a morphological form, a salt or a solvent complex of such a compound.

2. A compound according to claim 1, wherein R¹ is heteroarylmethyl.

3. A compound according to claim 2, wherein R¹ is 1,2,3-triazol-1-ylmethyl, 1,2,4-triazol-1-ylmethyl, pyrazol-1-ylmethyl, imidazol-1-ylmethyl or tetrazol-1-ylmethyl.

4. A compound according to claim 2, wherein R¹ is 1,2,3-triazol-1-ylmethyl.

5. A compound according to claim 1, wherein R² is OH, OPO₃H₂ or OCOR⁶.

6. A compound according to claim 5, wherein R² is OH.

7. A compound according to claim 1, wherein R³ is halogen.

8. A compound according to claim 1, wherein R⁴ is (C₁-C₃)alkyl or cycloalkyl.

9. A compound according to claim 1, which is selected from the following:
- (*16S*,*13S*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid;
- (*16S*,*13R*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-1-oxa-1,14-diaza-cyclopenta[*a*]phenanthrene-3-carboxylic acid;
- (*13S*,*16R*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-1-oxa-1,14-diazacyclopenta[*a*]phenanthrene-3-carboxylic acid;
- (*13R*,*16R*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-11-oxa-1,14-diazacyclopenta[*a*]phenanthrene-3-carboxylic acid;
- (*13R*,*16R*)-1-cyclopropyl-7-fluoro-16-[2-fluoro-4-((*5S*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid;
- (*13S*,*16S*)-1-cyclopropyl-7-fluoro-16-[2-fluoro-4-((*5S*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid;
- (*13S*,*16R*)-1-cyclopropyl-7-fluoro-16-[2-fluoro-4-((*5S*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-11-oxa-1,14-diazacyclopenta[*a*]phenanthrene-3-carboxylic acid;
- (*13R*,*16S*)-1-cyclopropyl-7-fluoro-16-[2-fluoro-4-((*5S*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-11-oxa-1,14-diazacyclopenta[*a*]phenanthrene-3-carboxylic acid;
- (*13S*,*16S*)-1-cyclopropyl-7-fluoro-16-[2-fluoro-4-((*5R*)-2-oxo-5-pyrazol-1-ylmethyl-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid;
- (*13S*,*16S*)-1-cyclopropyl-7-fluoro-16-[2-fluoro-4-((*5S*)-5-imidazol-1-ylmethyl-2-oxo-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid;
- (*13S*,*16S*)-1-cyclopropyl-7-fluoro-16-{2-fluoro-4-[(*5R*)-5-(4-methyl-[1,2,3]triazol-1-ylmethyl)-2-oxo-oxazolidin-3-yl]-phenoxymethyl}-16-hydroxy-4oxo-1,4,13,15,16,17-hexahydro-12*H*-11-oxa-1,14-diaza-cyclopenta[*a*]phenanthrene-3-carboxylic acid;
- (*13S*,*16S*)-1-cyclopropyl-7-fluoro-16-[2-fluoro-4-((*5R*)-2-oxo-5-tetrazol-1-ylmethyl-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid;
- (*13S*,*16S*)-1-cyclopropyl-7-fluoro-16-[2-fluoro-4-((*5R*)-2-oxo-5-[1,2,4]triazol-1-ylmethyl-oxazolidin-3-yl)-phenoxymethyl]-16-hydroxy-4-oxo-1,4,13,15,16,17-hexahydro-12*H*-11-oxa-1,14-diazacyclopenta[*a*]phenanthrene-3-carboxylic acid;
- (*13S*,*16S*)-1-cyclopropyl-7-fluoro-16-{2-fluoro-4-[(*5R*)-5-(isoxazol-3 yloxymethyl)-2-oxo-oxazolidin-3-yl]-phenoxymethyl}-16-hydroxy-4-oxo-1,4,13,15,16,17-hexahydro-12*H*-11-oxa-1,14-diaza-cyclopenta[*a*]phenanthrene-3-carboxylic acid;
- (*13S*,*16S*)-1-ethyl-7-fluoro-16-[2-fluoro-4-((*5R*)-2-oxo-5[1,2,3] triazol-1-ylmethyl-oxazolidin-3-yl)-phenoxymethyl]-16-hydroxy-4-oxo-1,4,13,15,16,17 hexahydro-12*H*-11-oxa-1,14-diaza-cyclopenta[*a*]phenanthrene-3-carboxylic acid;
- (*13S*,*16S*)-1-cyclopropyl-16-[2-fluoro-4-((*5R*)-2-oxo-5-[1,2,3]triazol-1-ylmethyl-oxazolidin-3-yl)-phenoxymethyl]-16-hydroxy-4-oxo-1,4,13,15,16,17 hexahydro-12*H*-11-oxa-1,14-diazacyclopenta[*a*]phenanthrene-3-carboxylic acid;
- (*13S*,*16S*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-4-oxo-1,4,13,15,16,17-hexahydro-12*H*-11-oxa-1,14-diazacyclopenta[*a*]phenanthrene-3-carboxylic acid;
- (*13S*,*16S*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-4-oxo-16-phosphonooxy-1,4,13,15,16,17-hexahydro-12*H*-11-oxa-1,14-diaza-cyclopenta[*a*]phenanthrene-3-carboxylic acid;
- (*13R, 16S*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-4-oxo-16-phosphonooxy-1,4,13,15,16,17-hexahydro-12*H*-11-oxa-1,14-diaza-cyclopenta[*a*]phenanthrene-3-carboxylic acid;
- (*13S*,*16R*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-4-oxo-16-phosphonooxy-1,4,13,15,16,17-hexahydro-12*H*-11-oxa-1,14-diaza-cyclopenta[*a*]phenanthrene-3-carboxylic acid;
- (*13R*,*16S*)-1-cyclopropyl-7-fluoro-16-[2-fluoro-4-((*5S*)-2-oxo-5-[1,2,3]triazol-1-ylmethyl-oxazolidin-3-yl)-phenoxymethyl]-4-oxo-16-phosphonooxy-1,4,13,15,16,17-hexahydro-12*H*-11-oxa-1,14-diaza-cyclopenta[*a*]phenanthrene-3-carboxylic acid;
- (*13S, 16S*)-1-cyclopropyl-7-fluoro-16-[2-fluoro-4-((*5S*)-2-oxo-5-[1,2,3]triazol-1-ylmethyl-oxazolidin-3-yl)-phenoxymethyl]-4-oxo-16-phosphonooxy-1,4,13,15,16,17-hexahydro-12*H*-11-oxa-1,14-diaza-cyclopenta[*a*]phenanthrene-3-carboxylic acid;
- (*13S*,*16R*)-1-cyclopropyl-7-fluoro-16-[2-fluoro-4-((*5S*)-2-oxo-5-[1,2,3]triazol-1-ylmethyl-oxazolidin-3-yl)-phenoxymethyl]-4-oxo-16-phosphonooxy-1,4,13,15,16,17-hexahydro-12*H*-11-oxa-1,14-diaza-cyclopenta[*a*]phenanthrene-3-carboxylic acid;
or a pharmaceutically acceptable salt of such a compound.

10. A compound according to claim 9, which is (*16S,13S*)-1-cyclopropyl-7-fluoro-16-[2-fluoro-4-((*5R*)-2-oxo-5-[1,2,3]triazol-1-ylmethyl-oxazolidin-3-yl)-phenoxymethyl]-16-hydroxy-4-oxo-1,4,13,15,16,17-hexahydro-12*H*-11-oxa-1,14-diaza-cyclopenta[*a*]phenanthrene-3-carboxylic acid, or a pharmaceutically acceptable salt thereof.

11. As a medicament, a compound of formula I as defined in claim 1 or a pharmaceutically acceptable salt thereof.

12. A pharmaceutical composition containing, as active principle, a compound of formula I as defined in claim 1 or a pharmaceutically acceptable salt thereof, and at least one therapeutically inert excipient.

13. Use of a compound of formula I as defined in claim 1, or of a pharmaceutically acceptable salt thereof, for the manufacture of a medicament intended for the prevention or treatment of infections.

14. A compound of formula I as defined in claim 1, or a pharmaceutically acceptable salt thereof, for the prophylaxis and chemotherapy of local and systemic infections caused by bacteria and bacteria-like organisms.
